# EUROPEAN PATENT SPECIFICATION

(11) **EP 3 180 433 B1**
(45) Date of publication and mention of the grant of the patent: **18.04.2018**
(21) Application number: 16753630.9
(22) Date of filing: 10.08.2016
(51) Int. Cl.: C12N 15/10, A61K 35/17, G01N 33/50

(54) **METHOD FOR PROVIDING TUMOUR-SPECIFIC T CELLS**
VERFAHREN ZUR BEREITSTELLUNG TUMORSPEZIFISCHER T-ZELLEN
PROCÉDÉ POUR PRODUIRE DES LYMPHOCYTES T SPÉCIFIQUES À LA TUMEUR

(30) Priority: 10.08.2015 EP 15180383; 23.12.2015 EP 15202419
(43) Date of publication of application: 21.06.2017
(73) Proprietor: HS Diagnomics GmbH, 12165 Berlin (DE)
(72) Inventor: HAMMER, Rudolf, 55270 Zornheim (DE); HENNIG, Steffen, 10961 Berlin (DE)
(74) Representative: Schulz Junghans Patentanwälte PartGmbB
(86) International application number: PCT/EP2016/069041
(87) International publication number: WO 2017/025564

(56) References cited:
- EP-A1- 2 653 556
- CARSTEN LINNEMANN ET AL: "High-throughput identification of antigen-specific TCRs by TCR gene capture", NATURE MEDICINE, vol. 19, no. 11, 13 October 2013 (2013-10-13), pages 1534-1541, XP055190765, ISSN: 1078-8956, DOI: 10.1038/nm.3359
- WU R ET AL: "Adoptive T-cell therapy using autologous tumor-infiltrating lymphocytes for metastatic melanoma: Current status and future outlook", CANCER JOURNAL, JONES AND BARTLETT PUBLISHERS, US, vol. 18, no. 2, 1 March 2012 (2012-03-01), pages 160-175, XP009170931, ISSN: 1528-9117

## Description

The present invention relates to a method for providing a tumour-specific, and particularly tumour-reactive, T cell preparation and use thereof, particularly for adoptive transfer and cancer treatment.

Cancer is one of the most frequent causes of death in countries of the developed world. Despite intensive research in the field of cancer treatment, there is yet an immense need of therapies for cancer treatment. Recently, efforts have been made to treat cancer by autologous transfer of immune cells of the patient to fight the disease, wherein T cells obtained from a patient's tumour were expanded and adoptively transferred.

EP 2653556 A1 discloses that autologous T cells or B cells can be obtained for personalized immunotherapy, for instance of cancer.

Linnemann et al. ("High-throughput identfication of antgen-specific TCRs by TCR gene capture, Nature Medicine, vol. 19, no. 11, October 13, 2013, pages 15341541) discloses a profiling of intratumoral tumour-reactive TCR repertoires by sequencing. The use of the TCRs in autologous immunotherapy of cancer, by re-introducing the genes into autologous is discussed.

Wu et al. ("Adoptive T-cell therapy using autologous tumor-infiltrating lymphocytes fro metastatic melanoma; Current status and future outlook", Cancer Journal, Jones and Bartlett Publishers, US, vol. 18, no. 2, March 1, 2012, pages 160-175) discusses the use of autologous tumour-infiltrating lymphocytes in the treatment of metastatic melanoma by adoptive transfer.

However, known transferred cells generally lack in high tumour-specificity and reactivity since they merely resemble a broad collection of many types of T cells and therefore induce only a moderate and improvable immune response. Consequently, the provision of autologous, highly tumour-specific and tumour-reactive T cells would be highly desirable.

Thus, it is the objective of the present invention to provide such cells, particularly for use in the adoptive transfer and treatment of cancer. This objective is attained by the subject matter of the independent claims.

### Terms and definitions

The term "nucleic acid" in the context of the present specification refers to an oligomer or polymer of nucleotides. The oligomer or the polymer may include natural nucleosides (i.e., adenosine, thymidine, guanosine, cytidine, uridine, deoxyadenosine, deoxythymidine, deoxyguanosine, and deoxycytidine), nucleoside analogs (e.g. 2-aminopurine, 2-aminoadenosine, 2-thiothymidine, inosine, pyrrolo-pyrimidine, 3-methyl adenosine, 5-methylcytidine, C5-bromouridine, C5-fluorouridine, C5-iodouridine, C5-propynyl-uridine, C5-propynyl-cytidine, C5-methylcytidine, 7-deazaadenosine, 7-deazaguanosine, 8-oxoadenosine, 8-oxoguanosine, O(6)-methylguanine, and 2-thiocytidine), chemically modified bases, biologically modified bases (e.g., methylated bases), intercalated bases, abasic sites, ribose sugars (RNA), 2'-deoxyribose sugars (DNA), terminal 3'-deoxyribose or 2',3'-dideoxyribose sugars, modified sugars (e.g., 2'-fluororibose, arabinose, and hexose), or modified phosphate groups (e.g., phosphorothioates and 5'-N-phosphoramidite linkages). Furthermore, the backbone may include modified locked (LNA) unlocked (UNA) sugars, mirror-form sugars (spiegelmer) or a peptide backbone (PNA) or a mixture thereof.

The term "probe" in the context of the present specification refers to a nucleic acid which is able to hybridise to a complementary nucleic acid. A probe may be modified to include labels for detection or identification such as fluorescent dyes or radioactive isotopes, haptens for capture, detection, or immobilisation such as biotin or digoxigenin, or reactive groups such as thiol, alkyne, azide, or EDC, for immobilisation, ligation or derivatisation.

The term "tumour sample" in the context of the present specification refers to a sample or a pool of samples obtained from a tumour of a patient. The tumour may also include metastases or a collection of metastases.

The term "non-tumour sample" in the context of the present specification refers to a sample or a pool of samples obtained from tissue in close proximity to the tumour of a patient.

The term "blood sample" or "sample from blood" in the context of the present specification refers to a sample from blood or a pool of samples obtained from blood of a patient.

The term "cell-free sample" in the context of the present specification refers to a sample or a pool of samples obtained from a patient which is preferably T cell-free or more preferably completely free of any cells comprising nucleic acids. Cell-free samples are preferably obtained from blood, typically as serum or plasma samples. Other examples for cell free samples are samples obtained from other bodily fluids such as cerebrospinal fluid, peritoneal fluid, synovial liquid, saliva, urine or feces.

The term "clonotype" in the context of the present specification refers to a group of T cells that comprise T cell receptor nucleic acid sequences that exhibit a virtually identical nucleic acid sequence with respect to the variable region of the TCR or that comprise T cell receptor amino acid sequences that exhibit a virtual identical amino acid sequence with respect to the variable region of the TCR. Clonotypes exhibiting a virtual identical amino acid sequence with respect to the variable region of the TCR may also referred to as clustertype.

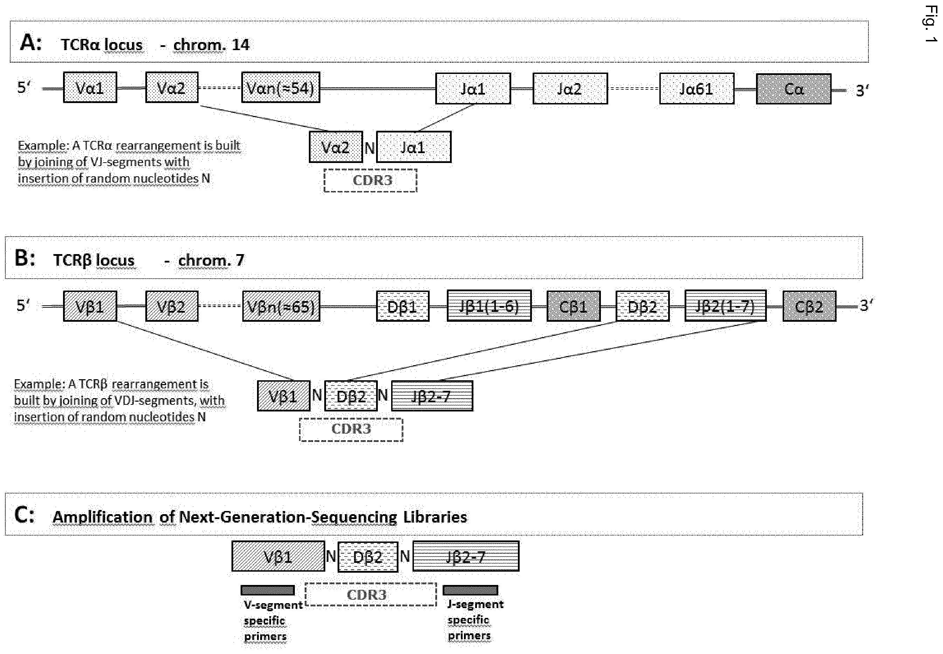

The term "clustertype" in the context of the present specification refers to a group of T cells that comprise T cell receptor nucleic acid sequences that exhibit a virtually identical amino acid sequence with respect to the variable region of the TCR.The term "variable region" in the context of the present specification refers to the region newly generated by the TCR rearrangement comprising a V- and J-segment as well as the CDR3 region (see fig.1).

The term "CDR3" in the context of the present specification refers to the hypervariable complementarity determining region 3. The size of CDR3 is particularly characterized by the total number of amino acids (AA) and respective nucleotides from the conserved cysteine in the Vβ, or Vα or Vγ or Vδ segment to the position of the conserved phenylalanine in the Jβ or Jα, Jγ or Jδ segment.

The term "tumour-specific" in the context of the present specification particularly refers to T cells occurring in a particular tumour and particularly exhibiting a preferential distribution in the particular tumour.

The term "tumour-reactive" in the context of the present specification particularly refers to T cells that are able to indirectly or directly modulate the growth, viability or proliferation of tumour cell of a particular tumour. Such tumour reactive T cells are particularly characterized by an increased expression of cytokines or surface activation markers when co-cultured with autologous tumour cells of the particular tumour.

The term "TIL" in the context of the present specification refers to tumour infiltrating lymphocytes.

The term "CD45RA" in the context of the present specification refers to the human naive T lymphocyte marker (PTPRC; Uniprot ID P07585; isoform A).

The term "CCR7" in the context of the present specification refers to the human chemokine receptor 7 (Uniprot ID P32248).

The term "CD62L" in the context of the present invention refers to a cell adhesion molecule on the surface on lymphocytes (Uniprot ID P14151). CD62L is also referred to as L-selectin.

The term "CD25" in the context of the present specification refers to the alpha chain of the human interleukin-2 receptor (Uniprot ID P01589).

The term "Foxp3" in the context of the present specification refers to a specific marker for natural T regulatory T cells and induced T regulatory T cells (Uniprot ID B7ZIG1). Foxp3 is also referred to as scurfin.

The term "LAG3" (Lymphocyte activation gene 3) in the context of the present specification refers to a marker for activated T cells (UniProtKB: P18627).

The term "CD69" in the context of the present specification refers to a marker for activated T cells (Uniprot Q7108).

The term "CD137" in the context of the present specification refers to a marker for activated T cells (Uniprot Q07011).

The term "CD154" in the context of the present specification refers to a marker for activated T cells (Uniprot P29965).

The term "PD-1" in the context of the present specification refers to a cell surface receptor expressed by T cells (Uniprot Q15116). PD-1 is also referred to as CD279.

The term "B7-H4" in the context of the present specification refers a marker for activated T cells (Uniprot Q7Z7D3). B7-H4 is also referred to as VTCN1.

The term "OX40" in the context of the present specification refers to the tumour necrosis factor receptor superfamily, member 4 (Uniprot P43489). OX40 is also referred to as TNFFSF4 or CD134.

The term "CD107a" in the context of the present specification refers to the lysosomal-associated membrane protein 1 (Uniprot P11279), CD107a is also referred to as LAMP-1.

The term "VISTA" in the context of the present specification refers to the V-domain Ig Suppressor of T cell Activation. VISTA is also referred to as PD-1 homolog (PD-1H).

The term "Butyrophilin" in the context of the present specification refers to a family of proteins constituting a subgroup of the IG superfamiliy that are expressed on activated T cells.

The term "Butyrophilin-like protein" in the context of the present specification refers to a marker of activated T cells.

The term "TNFalpha" in the context of the present specification refers to cytokine that is secreted by activated T cells (Uniprot P01375).

The term "interferon gamma" or "IFN gamma" in the context of the present specification refers to cytokine that is secreted by activated T cells (Uniprot P01579).

If any cell population is designated "positive" with respect to a certain marker protein, this designation shall mean that said cell population can be stained by a common fluorescent-dye-labelled antibody against the marker protein and will give a fluorescence signal of at least one log higher intensity compared to unlabeled cells or cells labelled with the same antibody but commonly known as not expressing said marker protein. Alternatively, the cell population may be stained by a labelled nucleic acid probe being able to specifically hybridizing to an mRNA encoding the aforementioned marker protein or a correlating regulatory entity. The marker protein correlating entity may represent a marker protein-regulating transcription factor mRNA, a non-coding RNA, or any other RNA which is specifically co-expressed in a cell population expressing said marker protein.

The term "T cell activation marker" in the context of the present specification refers to a molecule on the surface of an activated T cell.

High affinity in the context of the present specification refers to the dissociation constant of the binding of the ligand to the target molecule, wherein the dissociation constant is, 10⁻⁷ mol/L, 10⁻⁸ mol/L or 10⁻⁹ mol/l or less, and wherein the ligand does not bind to control molecules, for example proteins, with unrelated structural features. Control molecules are, by way of non-limiting example, plasma proteins such as albumins, globulins, lipoproteins, fibrinogens, prothrombin, acute phase proteins, and tumour markers such as CEA, CA19-9 orAFP and transferrin.

High specificity in the context of the present specification refers to the ratio of properly detected targets or analytes and the sum of all detected compounds or substances, wherein the ratio is 80%, 85%, 90%, 95%, 99% or 99.9%.

The term "optimal annealing temperature" in the context of the present specification refers to the temperature, at which the probe of the invention exhibits the highest probability of binding to the tumour-specific T cell receptor nucleic acid sequence within the cell.

The term "nanogold" in the context of the present specification refers to a submicrometre-size gold particle.

Uniprot ID numbers refer to entries in the UniProt Knowledgebase.

DSMZ numbers refer to entries or deposits at the Leibniz-Institut DSMZ- German Collection of Microorganisms and Cell Cultures GmbH, Braunschweig, Germany.

A transfection reagent in the context of the present invention refers to a compound that enables or supports the process of deliberately introducing nucleic acids into cells, particularly into human immune cells.

### Summary of the invention

According to a first aspect of the invention, a method for providing a tumour-specific T cell preparation is provided. The method comprises the steps of:
a. selecting tumour-specific T cell clones by:
   - providing a tumour sample obtained from a patient, wherein the tumour sample comprises T cells that infiltrated the tumour;
   - isolating a nucleic acid preparation from the tumour sample in a nucleic acid isolation step;
   - obtaining a plurality of T cell receptor nucleic acid sequences from the nucleic acid preparation or a plurality of T cell receptor amino acid sequences encoded by the plurality of T cell receptor nucleic acid sequences;
   - selecting a tumour-specific T cell receptor nucleic acid sequence from the plurality of T cell receptor nucleic acid sequences or a tumour-specific T cell receptor amino acid sequence from the plurality of T cell receptor amino acid sequences in a sequence selection step;
b. sorting tumour-specific T cells clones by:
   - providing a lymphocyte preparation obtained from the patient;
   - isolating cells that comprise the selected tumour-specific T cell receptor nucleic acid sequence or the selected tumour-specific T cell receptor amino acid sequence from the lymphocyte preparation in an isolation step.

In certain embodiments, the isolation step comprises the steps of:
- contacting the lymphocyte preparation with a specifically reactive ligand being able to bind an amino acid sequence comprised within the V segment of the T cell receptor that corresponds to the selected tumour-specific T cell receptor nucleic acid sequence or tumour-specific T cell receptor amino acid sequence, wherein the ligand is attached to a detectable label, and
- isolating T cells carrying the detectable label from the lymphocyte preparation.

Particularly, the V segment is encoded by a nucleic acid molecule that is uniquely related to the selected tumour-specific T cell receptor nucleic acid sequence.

In certain embodiments, the ligand binds the amino acid sequence with a dissociation constant of 10⁻⁷, 10⁻⁸ or 10⁻⁹ mol/l or less.

In certain embodiments, the selected tumour-specific T cell receptor nucleic acid sequence is uniquely related to a nucleic acid sequence encoding the V-segment of the beta chain of the human T cell receptor.

In certain embodiments, the specifically reactive ligand is an anti-V_{β} antibody, which is directed to the V segment of the beta chain of a T cell receptor.

Such anti-V_{β} antibodies are known, see for example http://www.imgt.org/IMGTrepertoire/ Regulation/antibodies/human/TRB/TRBV/Hu TRBVMab.html, and can be obtained for example at Pierce Endogen, Serotec or Coulter.

In certain embodiments, the isolation step comprises the steps of:
- contacting the lymphocyte preparation with a nucleic acid probe specifically binding to the selected tumour-specific T cell receptor nucleic acid sequence, wherein the nucleic acid probe is attached to a detectable label;
- isolating T cells carrying the detectable label from the lymphocyte preparation.

Particularly, specifically binding of the nucleic acid probe to the selected tumour-specific T cell receptor nucleic acid sequence particularly refers to a hybridization of the probe to the selected sequence, particularly under high stringency conditions.

In certain embodiments, the isolation step comprises the steps of:
- contacting the lymphocyte preparation with specifically reactive ligand being able to bind an amino acid sequence comprised within the V region of the T cell receptor that corresponds to the selected tumour-specific T cell receptor nucleic acid sequence or tumour-specific T cell receptor amino acid sequence, wherein the ligand is attached to a detectable label,
- isolating T cells carrying the detectable label from the lymphocyte preparation,
- contacting the isolated cells with a nucleic acid probe specifically binding to the selected tumour-specific T cell receptor nucleic acid sequence, wherein said nucleic acid probe is attached to another detectable label, and
- isolating T cells carrying the other detectable label from the previously isolated cells.

In certain embodiments, the isolation step comprises
- a separating step, wherein the lymphocyte preparation is separated into a plurality of fractions,
- an expanding step, wherein cells comprised within said plurality of fractions are expanded under conditions of cell culture, and
- a selecting step, wherein at least one fraction of said plurality of fraction that comprises the selected tumour-specific T cell receptor nucleic acid sequence is selected.

Particularly, the lymphocyte preparation is separated into the plurality of fractions such that not all fraction of the plurality, preferably less than half of the plurality, more preferable less than 10 percent of the plurality, even more preferable less than 5 percent, most preferable less than 1 percent, comprises the selected tumour-specific T cell receptor nucleic acid sequence. Such separation may be achieved by limiting the number of cells per fraction of the plurality.

In certain embodiments, each of the fractions of the plurality comprises not more than 10⁵cells, preferably not more 10⁴ cells, more preferable not more than 10³ cells, even more preferable not more than 10² cells.

In certain embodiments, the lymphocyte preparation is separated into at least 96 fraction, preferable into 96, wherein particularly each of the fractions comprises not more than 10⁵ cells.

In certain embodiments, the lymphocyte preparation is separated into 96 fractions to 384 fractions.

In certain embodiments, the selecting step comprises obtaining T cell receptor nucleic acid sequences from the plurality of fraction and identifying fractions comprising the selected tumour-specific T cell receptor nucleic acid sequence, wherein particularly the T cell receptor nucleic acid sequences are obtained by amplification, particularly by PCR,

In certain embodiments, fractions comprising the selected tumour-specific T cell receptor nucleic acid sequence are identified by an amplification reaction with primers that specifically anneal to at least a part of the selected tumour-specific T cell receptor nucleic acid, wherein particularly fractions not comprising the selected tumour-specific T cell receptor nucleic acid sequence do not exhibit an amplification product.

In certain embodiments, the T cell receptor nucleic acid sequences are obtained from an aliquot of cells comprised within the respective fraction or from the supernatant of the respective fraction.

Advantageously, no expensive probes with long delay in synthesis and validation are necessary by such selecting. Furthermore, the above-mentioned embodiment is applicable to very rare clonotypes due to PCR sensitivity vs. probe background. Additionally, the expanding step yields rapidly dividing cells that can be directly applied to in vitro expansion for potential autologuous cell treatment.

In certain embodiments, the selecting step comprises contacting the fractions of the plurality with a nucleic acid probe specifically binding to the selected tumour-specific T cell receptor nucleic acid sequence, wherein the nucleic acid probe is attached to a detectable label, and selecting at least one fraction of the plurality that comprises T cells carrying the detectable label.

In certain embodiments, the separating step is preceded by the steps of
- contacting the lymphocyte preparation with a specifically reactive ligand being able to bind an amino acid sequence comprised within the V segment of the T cell receptor that corresponds to the selected tumour-specific T cell receptor nucleic acid sequence or to the selected tumour-specific T cell receptor amino acid sequence, wherein the ligand is attached to a detectable label, and
- isolating T cells carrying the detectable label from the lymphocyte preparation, wherein afterwards the isolated T cells are subjected to the separating step as described above.

In certain embodiments, the isolation step further comprises
- a second separation step, wherein the selected fraction is separated into a second plurality of fraction,
- a second expanding step, wherein cells comprised with the second plurality of fraction are expanded under conditions of cell culture, and
- a second selecting step, wherein at least one fraction of the second plurality of fraction that comprises the selected tumour-specific T cell receptor nucleic acid sequence is selected.

Particularly, the separation step, the expanding step and the selecting step may be repeated with each newly selected fraction that comprises the selected tumour-specific T cell receptor nucleic acid sequence. Preferably, the separation step, the expanding step and the selecting step are repeated one to four times.

In certain embodiments, the plurality of T cell receptor nucleic acid sequences is obtained by sequencing the nucleic acids of the nucleic acid sequences. In certain embodiments, the nucleic acids of the nucleic acid preparation are sequenced in parallel. A suitable method for parallel sequencing is disclosed in WO 2014/096394 A1.

In certain embodiments, the nucleic acid preparation comprises genomic DNA of the cells of the tumour sample, particularly of the T cells that infiltrated the tumour.

In certain embodiments, the nucleic acid preparation comprises at least 10 ng DNAfrom mature or activated T cells of the tumour sample, which particularly corresponds to the amount of DNA of around 1,500 mature T cells. The quantification of the amount of mature T cell DNA may be determined by method known to the skilled person such as for example quantitative PCR or, digital droplet PCR. The sequencing of the nucleic acid preparation may include the sequencing of a reference sample with known amount of DNA. Additionally, the amount of mature T cells in the tumour sample may be measured by immunohistochemical staining and microscopy or cell-sorting by FACS.

In certain embodiments, the lymphocyte preparation is provided by the tumour sample obtained from the patient, by a blood sample obtained from the patient or a whole-tumour sample obtained from the patient.

Advantageously, the method of the invention is independent of viable and/or proliferating tumour-specific T cells that are obtained from the tumour sample for identifying tumour-specific clonotype and for preparing those. Once one or more tumour-specific clonotypes are identified by means of a tumour-specific T cell receptor nucleic acid sequence or a tumour-specific T cell receptor amino acid sequence, they may be prepared from other sources such as another tumour sample or the blood of the patient.

In certain embodiments, the sequence selecting step comprises the steps of:
- aligning the plurality of T cell receptor nucleic acid sequences obtained from the tumour sample;
- grouping T cell receptor nucleic acid sequences comprised in the plurality of T cell receptor nucleic acid sequences into a plurality of tumour sample clonotypes, wherein
   a) T cell receptors nucleic acid sequences comprised within a particular clonotype exhibit a virtually identical nucleic acid sequence with respect to the variable region of the TCR, and/or
   b) T cell receptor amino acid sequences encoded by the T cell receptor nucleic acid sequences comprised within a particular clonotype exhibit an identical amino acid sequence with respect to the variable region of the TCR;
- determining the number of T cell receptor nucleic acid sequences within the plurality of T cell receptor nucleic acid sequences associated with each clonotype, thereby yielding a clonotype frequency for each of said clonotypes,
- selecting a tumour-specific clonotype from the plurality of tumour sample clonotypes, wherein the tumour specific clonotype is one of the 100 most frequent clonotypes of the plurality of tumour sample clonotypes or is another clonotype of the plurality of tumour sample clonotypes that comprises a T cell receptor amino acid sequence being identical or virtually identical to a T cell receptor amino acid sequence encoded by a T cell receptor nucleic acid sequence of the plurality of T cell receptor nucleic acid sequences comprised within the one tumour-specific clonotype of the 100 most frequent clonotypes of the plurality of tumour sample clonotypes, and
- selecting a T cell receptor nucleic acid sequence of the plurality of T cell receptors nucleic acid sequences comprised within the selected tumour-specific clonotype as the tumour-specific receptor nucleic acid sequence.

Particularly, after sequencing, pairs joining all identical and related nucleic acid sequence reads that deviate up to one base pair mismatch are clustered and designated as clonotypes.

In certain embodiments, a tumour-specific clonotype is selected from the plurality of tumour sample clonotypes that is one of the 50 most frequent clonotypes of the plurality of tumour sample clonotypes. In certain embodiments, a tumour-specific clonotype is selected from the plurality of tumour sample clonotypes that is one of the 20 most frequent clonotypes of the plurality of tumour sample clonotypes.

Particularly, a first T cell receptor nucleic acid sequence is virtually identical to a second T cell receptor nucleic acid sequence, if both sequences differ in not more than one position.

In certain embodiments, a first T cell receptor nucleic acid sequence is virtually identical to a second T cell receptor nucleic acid sequence, if both sequences differ in not more than one position, and the first T cell receptor nucleic acid sequence exhibits in the respective sample, particularly in the tumour sample, an at least twentyfold frequency compared to the second T cell receptor nucleic acid sequence. Consequently, both first and second T cell receptor nucleic acid sequences are assigned to the same clonotype.

Likewise, a first T cell receptor amino acid sequences is virtually identical to a second T cell receptor amino acid sequence if both amino acid sequences differ in not more than at one or two position from each other. The above mentioned T cell receptor amino acid sequences may be comprised within the alpha or beta chain of the TCRα/β or within the gamma or delta chain of the TCRγ/δ.

Particularly, the clonotype frequency is a measure of the relative or absolute frequency of the T cell identified by the TCR nucleic acid sequence within the tumour sample.

Particularly, the clonotype frequency in a given sample is a measure of the relative or absolute frequency of the T cell identified by the TCR nucleic acid sequence within said sample.

In certain embodiments, the T cell receptor nucleic acid sequences of the above-mentioned plurality are comprised within nucleic acid sequences encoding one of the polypeptide chains that form a human T cell receptor, particularly TCRα/β or TCRγ/δ. In certain embodiments, the T cell receptor nucleic acid sequences of the above-mentioned plurality do not comprise non-coding nucleic acid sequences. Non-coding sequences refer to clonotypes with stop-codons or frame shifts that lead to non-functional TCR protein sequences.

In certain embodiments, the tumour-specific T cell receptor nucleic acid sequence is characterized by a length of 30 nucleotides to 110 nucleotides.

In certain embodiments, the tumour-specific T cell receptor nucleic acid sequence encodes a unique amino acid sequence comprised within any one of the polypeptide chains (alpha, beta, gamma and delta) that form a human T cell receptor, wherein the unique amino acid sequence exclusively occurs in a particular clonotype or clustertype and not any other clonotype or clustertype.

Accordingly, the selection step additionally or alternatively comprises the steps of:
- aligning the plurality of T cell receptor amino acid sequences obtained from the tumour sample;
- grouping T cell receptor nucleic acid sequences comprised in the plurality of T cell receptor amino acid sequences into a plurality of tumour sample clonotypes, wherein T cell receptors amino acid sequences comprised within a particular clonotype exhibit a virtually identical or identical amino acid sequence with respect to the variable region of the TCR,
- determining the number of T cell receptor amino acid sequences within the plurality of T cell receptor amino acid sequences associated with each clonotype, thereby yielding a clonotype frequency for each of said clonotypes,
- selecting a tumour-specific clonotype from the plurality of tumour sample clonotypes, wherein the tumour specific clonotype is one of the 100 most frequent clonotypes of the plurality of tumour sample clonotypes or is another clonotype of the plurality of tumour sample clonotypes that comprises a T cell receptor amino acid sequence being virtually identical or identical to a T cell receptor amino acid sequence of the plurality of T cell receptor amino acid sequences comprised within the one tumour-specific clonotype of the 100 most frequent clonotypes of the plurality of tumour sample clonotypes, and
- selecting a T cell receptor amino acid sequence of the plurality of T cell receptors nucleic acid sequences comprised within the selected tumour-specific clonotype as the tumour-specific receptor amino acid sequence.

In certain embodiments, one of the 100 most frequent clonotypes of the plurality of tumour sample clonotypes, particularly the most frequent clonotype, and one or more additional clonotypes of the plurality of tumour sample clonotypes that comprise a T cell receptor amino acid sequence being identical to a T cell receptor amino acid sequence encoded by a T cell receptor nucleic acid sequence of the plurality of T cell receptor nucleic acid sequences comprised within the one tumour-specific clonotype of the 100 most frequent clonotypes of the plurality of tumour sample clonotypes are selected as tumour-specific clonotypes, and isolated from the lymphocytes preparation, particularly by contacting the lymphocyte with nucleic acid probes specifically binding to the tumour-specific T cell receptor nucleic acid sequences comprised within the selected clonotypes, wherein said nucleic acid probes are attached to a detectable label, and cell carrying the label are isolated from the lymphocyte preparation.

In certain embodiments, the 5 most frequent clonotypes, the 10 most frequent clonotypes, the 15 most frequent clonotypes or the 20 most frequent clonotypes of the plurality of tumour sample clonotypes, and/or one or more additional clonotypes of the plurality of tumour sample clonotypes that comprise a T cell receptor amino acid sequence being identical to a T cell receptor amino acid sequence encoded by a T cell receptor nucleic acid sequence of the plurality of T cell receptor nucleic acid sequences comprised within the 5 most frequent clonotypes, the 10 most frequent clonotypes, the 15 most frequent clonotypes or the 20 most frequent clonotypes of the plurality of tumour sample clonotypes are selected as tumour-specific clonotypes, and isolated from the lymphocytes preparation, particularly by contacting the lymphocyte with a specifically reactive ligand being able to bind an amino acid sequence comprised within the V segment of the T cell receptor that corresponds to the selected tumour-specific T cell receptor nucleic acid sequence or to the selected tumour-specific T cell receptor amino acid sequence comprised within the selected clonotypes, wherein said ligand is attached to a detectable label, and cells carrying the label are isolated from the lymphocyte preparation.

In certain embodiments, the tumour-specific receptor nucleic acid sequence encodes the CDR3 region of the T cell receptor. In certain embodiments, the tumour-specific receptor amino acid sequence comprises or is comprised within the CDR3 region of the T cell receptor.

In certain embodiments, the method of the invention further comprises
- providing a non-tumour sample obtained from the patient;
- isolating a nucleic acid preparation from the non-tumour sample in a nucleic acid isolation step;
- obtaining a plurality of T cell receptor nucleic acid sequences from the nucleic acid preparation, yielding a plurality of non-tumour-specific T cell receptor nucleic acid sequences;
- aligning the plurality of non-tumour-specific T cell receptor nucleic acid sequences obtained from the non-tumour sample;
- grouping T cell receptor nucleic acid sequences comprised in the plurality of non-tumour-specific T cell receptor nucleic acid sequences into a plurality of non-tumour-specific clonotypes, wherein
   a) T cell receptor nucleic acid sequences comprised within a particular clonotype exhibit a virtually identical sequence with respect to the variable region of the TCR, particularly the CDR3 region and/or
   b) T cell receptor amino acid sequences encoded by the T cell receptor nucleic acid sequences comprised within a particular clonotype exhibit an identical sequence with respect to the variable region of the TCR, particularly the CDR3 region;
- selecting a tumour specific clonotype from the plurality of tumour sample clonotypes, wherein
   - the tumour specific clonotype is one of the 100 most frequent clonotypes of the plurality of tumour sample clonotypes or is another clonotype of the plurality of tumour sample clonotypes that comprises a T cell receptor amino acid sequence being identical or virtually identical to a T cell receptor amino acid sequence encoded by a T cell receptor nucleic acid sequence of the plurality of T cell receptor nucleic acid sequences comprised within the one tumour-specific clonotype of the 100 most frequent clonotypes of the plurality of tumour sample clonotypes, and
   - the one tumour-specific clonotype of the 100 most frequent clonotypes of the plurality of tumour sample clonotypes is absent in the non-tumour sample or can be assigned to a non-tumour-specific clonotype that shows a frequency (within the non-tumour sample) of not more than 20 %, 15%, 10 % or 5 % of the frequency of the tumour-specific clonotype.

Particularly, the non-tumour-specific T cell receptor nucleic acid sequences are grouped into the plurality of non-tumour-specific clonotypes in the same manner as the T cell receptor nucleic acid sequences obtained from the tumour into the plurality of tumour sample clonotypes, particularly to allow an assignment of a tumour sample clonotype to a non-tumour clonotype.

Particularly, a tumour-specific clonotype can be assigned to a non-tumour-specific clonotype, if
a) any one of the T cell receptor nucleic acid sequences of the plurality of T cell receptor nucleic acid sequences comprised within this clonotype is virtually identical to a T cell receptor nucleic acid sequence comprised within the non-tumour clonotype and/or
b) a T cell receptor amino acid sequence encoded by a T cell receptor nucleic acid sequence of the plurality of T cell receptor nucleic acid sequences comprised within this clonotype is identical to a T cell receptor amino acid sequence encoded by a T cell receptor nucleic acid sequence comprised within the non-tumour sample clonotype.

Likewise, a tumour-specific clonotype is absent in the non-tumour sample if this clonotype cannot be assigned to any of the clonotypes of the non-tumour sample.

In certain embodiments, the non-tumour sample is a sample of non-tumour tissue adjacent to the tumour. Such non-tumour tissue can be identified by common techniques such as ultra sound examination, radiography, CT or immunostaining.

In certain embodiments, the method of the invention further comprises
- providing a non-tumour sample obtained from the patient;
- isolating a nucleic acid preparation from the non-tumour sample in a nucleic acid isolation step;
- obtaining a plurality of T cell receptor nucleic acid sequences from the nucleic acid preparation and a plurality of T cell amino acid sequences encoded by the plurality of T cell receptor nucleic acid sequences, yielding a plurality of non-tumour-specific T cell receptor amino acid sequences;
- aligning the plurality of non-tumour-specific T cell receptor amino acid sequences obtained from the non-tumour sample;
- grouping T cell receptor amino acid sequences comprised in the plurality of non-tumour-specific T cell receptor amino acid sequences into a plurality of non-tumour-specific clonotypes, wherein T cell receptor amino acid sequences comprised within a particular clonotype exhibit a virtually identical or an identical sequence with respect to the variable region of the TCR, particularly the CDR3 region,
- selecting a tumour specific clonotype from the plurality of tumour sample clonotypes, wherein
   - the tumour specific clonotype is one of the 100 most frequent clonotypes of the plurality of tumour sample clonotypes or is another clonotype of the plurality of tumour sample clonotypes that comprises a T cell receptor amino acid sequence being virtually identical or identical to a T cell receptor amino acid sequence of the plurality of T cell receptor amino acid sequences comprised within the one tumour-specific clonotype of the 100 most frequent clonotypes of the plurality of tumour sample clonotypes, and
   - the one tumour-specific clonotype of the 100 most frequent clonotypes of the plurality of tumour sample clonotypes is absent in the non-tumour sample or can be assigned to a non-tumour-specific clonotype that shows a frequency (within the non-tumour sample) of not more than 20 %, 15%, 10 % or 5 % of the frequency of the tumour-specific clonotype.

Particularly, the non-tumour-specific T cell receptor amino acid sequences are grouped into the plurality of non-tumour-specific clonotypes in the same manner as the T cell receptor amino acid sequences obtained from the tumour into the plurality of tumour sample clonotypes, particularly to allow an assignment of a tumour sample clonotype to a non-tumour clonotype.

Particularly, a tumour-specific clonotype can be assigned to a non-tumour-specific clonotype, if any one of the T cell receptor amino acid sequences of the plurality of T cell receptor amino acid sequences comprised within this clonotype is virtually identical or identical to a T cell receptor amino acid sequence comprised within the non-tumour clonotype.

In certain embodiments, the method of the invention further comprises:
- providing a blood sample obtained from the patient;
- isolating a nucleic acid preparation from the blood sample in a nucleic acid isolation step;
- obtaining a plurality of T cell receptor nucleic acid sequences from the nucleic acid preparation,
- aligning the plurality of T cell receptor nucleic acid sequences;
- grouping T cell receptor nucleic acid sequences comprised in the plurality of T cell receptor sequences into a plurality of blood sample clonotypes, wherein
   a) T cell receptor nucleic acid sequences comprised within a particular clonotype exhibit a virtually identical sequence with respect to the variable region of the TCR, particularly the CDR3 region, and/or
   b) T cell receptor amino acid sequences encoded by the T cell receptor sequences comprised within a particular clonotype exhibit an identical sequence with respect to the variable region of the TCR, particularly the CDR3 region;
- selecting a tumour specific clonotype from the plurality of tumour sample clonotypes, wherein
   - the tumour specific clonotype is one of the 100 most frequent clonotypes of the plurality of tumour sample clonotypes or is another clonotype of the plurality of tumour sample clonotypes that comprises a T cell receptor amino acid sequence being identical or virtually identical to a T cell receptor amino acid sequence encoded by a T cell receptor nucleic acid sequence of the plurality of T cell receptor nucleic acid sequences comprised within the one tumour-specific clonotype of the 100 most frequent clonotypes of the plurality of tumour sample clonotypes, and
   - the one tumour-specific clonotype of the 100 most frequent clonotypes of the plurality of tumour sample clonotypes can be assigned to a blood sample clonotype that shows a frequency below the frequency of the one tumour-specific clonotype.

Particularly, the T cell receptor nucleic acid sequences obtained from the blood sample are grouped into the plurality of blood sample clonotypes in the same manner as the T cell receptor nucleic acid sequences obtained from the tumour into the plurality of tumour sample clonotypes, particularly to allow an assignment of a tumour sample clonotype to a blood sample clonotype.

Particularly, a tumour-specific clonotype can be assigned to a blood sample clonotype, if
a) any one of the T cell receptor nucleic acid sequences of the plurality of T cell receptor nucleic acid sequences comprised within this clonotype exhibits a virtually identical sequence to a T cell receptor nucleic acid sequence comprised within the blood sample clonotype, and/or
b) a T cell receptor amino acid sequence encoded by a T cell receptor nucleic acid sequence of the plurality of T cell receptor nucleic acid sequences comprised with this clonotype is identical to a T cell receptor amino acid sequence encoded by a T cell receptor sequence comprised within the blood sample clonotype.

In certain embodiments, the method of the invention further comprises:
- providing a blood sample obtained from the patient;
- isolating a nucleic acid preparation from the blood sample in a nucleic acid isolation step;
- obtaining a plurality of T cell receptor nucleic acid sequences from the nucleic acid preparation and a plurality of T cell amino acid sequences encoded by the plurality of T cell receptor nucleic acid sequences,
- aligning the plurality of T cell receptor amino acid sequences;
- grouping T cell receptor amino acid sequences comprised in the plurality of T cell receptor sequences into a plurality of blood sample clonotypes, wherein T cell receptor amino acid sequences comprised within a particular clonotype exhibit a virtually identical sequence with respect to the variable region of the TCR
- selecting a tumour specific clonotype from the plurality of tumour sample clonotypes, wherein
   - the tumour specific clonotype is one of the 100 most frequent clonotypes of the plurality of tumour sample clonotypes or is another clonotype of the plurality of tumour sample clonotypes that comprises a T cell receptor amino acid sequence being virtually identical or identical to a T cell receptor amino acid sequence of the plurality of T cell receptor amino acid sequences comprised within the one tumour-specific clonotype of the 100 most frequent clonotypes of the plurality of tumour sample clonotypes, and
   - the one tumour-specific clonotype of the 100 most frequent clonotypes of the plurality of tumour sample clonotypes can be assigned to a blood sample clonotype that shows a frequency below the frequency of the one tumour-specific clonotype.

Particularly, the T cell receptor amino acid sequences obtained from the blood sample are grouped into the plurality of blood sample clonotypes in the same manner as the T cell receptor amino acid sequences obtained from the tumour into the plurality of tumour sample clonotypes, particularly to allow an assignment of a tumour sample clonotype to a blood sample clonotype.

Particularly, a tumour-specific clonotype can be assigned to a blood sample clonotype, if any one of the T cell receptor amino acid sequences of the plurality of T cell receptor amino acid sequences comprised within this clonotype exhibits a virtually identical or identical sequence to a T cell receptor amino acid sequence comprised within the blood sample clonotype.

In certain embodiments, the method of the invention further comprises
- providing a cell-free sample obtained from the patient;
- isolating a nucleic acid preparation from the cell-free sample in a nucleic acid isolation step;
- obtaining a plurality of T cell receptor nucleic acid sequences from the nucleic acid preparation,
- aligning the plurality of T cell receptor nucleic acid sequences;
- grouping T cell receptor nucleic acid sequences comprised in the plurality of T cell receptor nucleic acid sequences into a plurality of cell-free sample clonotypes, wherein
   a) T cell receptor nucleic acid sequences comprised within a particular clonotype exhibit a virtually identical sequence with respect to the variable region of the TCR, particularly the CDR3 region, and or
   b) T cell receptor amino acid sequences encoded by a T cell receptor nucleic acid sequence comprised within a particular clonotype exhibit an identical sequence with respect to the variable region of the TCR, particularly the CDR3 region;
- selecting a tumour specific clonotype from the plurality of tumour sample clonotypes, wherein
   - the tumour specific clonotype is one of the 100 most frequent clonotypes of the plurality of tumour sample clonotypes or is another clonotype of the plurality of tumour sample clonotypes that comprises a T cell receptor amino acid sequence being identical or virtually identical to a T cell receptor amino acid sequence encoded by a T cell receptor nucleic acid sequence of the plurality of T cell receptor nucleic acid sequences comprised within the one tumour-specific clonotype of the 100 most frequent clonotypes of the plurality of tumour sample clonotypes, and
   - the one tumour-specific clonotype of the 100 most frequent clonotypes of the plurality of tumour sample clonotypes can be assigned to a cell-free sample clonotype, particularly to a cell-free clonotype that shows a frequency above 0.001 % of all frequencies in the plurality of cell-free sample clonotypes.

Particularly, the T cell receptor nucleic acid sequences obtained from the cell-free sample are grouped into the plurality of cell-free sample clonotypes in the same manner as the T cell receptor nucleic acid sequences obtained from the tumour into the plurality of tumour sample clonotypes, particularly to allow an assignment of a tumour sample clonotype to a cell-free sample clonotype.

Particularly, a tumour-specific clonotype can be assigned to a cell-free sample clonotype, if
a) any one of the T cell receptor nucleic acid sequences of the plurality of T cell receptor nucleic acid sequences comprised within this clonotype is virtually identical to a T cell receptor nucleic acid sequence comprised within the cell-free sample clonotype, and/or
b) a T cell receptor amino acid sequence encoded by a T cell receptor nucleic acid sequence of the plurality of T cell receptor nucleic acid sequences comprised with this clonotype is identical to a T cell receptor amino acid sequence encoded by a T cell receptor nucleic acid sequence comprised within the cell-free sample clonotype.

In certain embodiments, the method of the invention further comprises
- providing a cell-free sample obtained from the patient;
- isolating a nucleic acid preparation from the cell-free sample in a nucleic acid isolation step;
- obtaining a plurality of T cell receptor nucleic acid sequences from the nucleic acid preparation and a plurality of T cell receptor amino acid sequences encoded by the plurality of T cell receptor nucleic acid sequences,
- aligning the plurality of T cell receptor amino acid sequences;
- grouping T cell receptor amino acid sequences comprised in the plurality of T cell receptor amino acid sequences into a plurality of cell-free sample clonotypes, wherein T cell receptor nucleic acid sequences comprised within a particular clonotype exhibit a virtually identical or identical sequence with respect to the variable region of the TCR, particularly the CDR3 region;
- selecting a tumour specific clonotype from the plurality of tumour sample clonotypes, wherein
   - the tumour specific clonotype is one of the 100 most frequent clonotypes of the plurality of tumour sample clonotypes or is another clonotype of the plurality of tumour sample clonotypes that comprises a T cell receptor amino acid sequence being virtually identical or identical to a T cell receptor amino acid sequence of the plurality of T cell receptor amino acid sequences comprised within the one tumour-specific clonotype of the 100 most frequent clonotypes of the plurality of tumour sample clonotypes, and
   - the one tumour-specific clonotype of the 100 most frequent clonotypes of the plurality of tumour sample clonotypes can be assigned to a cell-free sample clonotype, particularly to a cell-free clonotype that shows a frequency above 0.001 % of all frequencies in the plurality of cell-free sample clonotypes.

Particularly, the T cell receptor amino acid sequences obtained from the cell-free sample are grouped into the plurality of cell-free sample clonotypes in the same manner as the T cell receptor amino acid sequences obtained from the tumour into the plurality of tumour sample clonotypes, particularly to allow an assignment of a tumour sample clonotype to a cell-free sample clonotype.

Particularly, a tumour-specific clonotype can be assigned to a cell-free sample clonotype, if any one of the T cell receptor amino acid sequences of the plurality of T cell receptor amino acid sequences comprised within this clonotype is virtually identical or identical to a T cell receptor amino acid sequence comprised within the cell-free sample clonotype.

In certain embodiments, the selected tumour-specific clonotype cannot be assigned to a known clonotype being reactive to the human cytomegalovirus or the Epstein-Barr-virus.

Such assignment may be performed by bioinformatics methods, wherein particularly a tumour-specific T cell receptor nucleic acid sequence comprised within the selected tumour-specific clonotype is compared to nucleic acid sequences of known clonotypes being reactive to the human cytomegalovirus or the Epstein-Barr-virus.

Particularly, the selected tumour-specific clonotype cannot be assigned to a known clonotype being reactive to the human cytomegalovirus or the Epstein-Barr-virus, if
a) none of the T cell receptor nucleic acid sequences of the plurality of T cell nucleic acid sequences comprised within this clonotype is virtually identical to a T cell receptor nucleic acid sequence comprised within the known clonotype,
b) none of the T cell receptor amino acid sequences encoded by a T cell receptor nucleic acid sequence of the plurality of T cell receptor nucleic acid sequences comprised with this clonotype is identical to a T cell receptor amino acid sequence encoded by a T cell receptor nucleic acid sequence comprised within the known clonotype, or
c) none of the T cell receptor amino acid sequences of the plurality of T cell amino acid sequences comprised within this clonotype is virtually identical or identical to a T cell receptor amino acid sequence comprised within the known clonotype.

In certain embodiments, the method of the invention further comprised the steps of:
- selecting a tumour specific clonotype from the plurality of tumour sample clonotypes, wherein
   - the tumour specific clonotype is one of the 100 most frequent clonotypes of the plurality of tumour sample clonotypes or is another clonotype of the plurality of tumour sample clonotypes that comprises a T cell receptor amino acid sequence being identical or virtually identical to a T cell receptor amino acid sequence encoded by a T cell receptor nucleic acid sequence of the plurality of T cell receptor nucleic acid sequences comprised within the one tumour-specific clonotype of the 100 most frequent clonotypes of said plurality of tumour sample clonotypes, and
   - the one tumour-specific clonotype of the 100 most frequent clonotypes of the plurality of tumour sample clonotypes can be assigned to another clonotype of the plurality of tumour sample clonotypes that comprises a T cell amino acid sequence being identical or virtually identical to a T cell receptor amino acid encoded by a T cell receptor nucleic acid sequence of the plurality of T cell receptor nucleic acid sequences comprised within the one of the 100 most frequent tumour-specific clonotype.

In certain embodiments, the most frequent clonotype of the tumour sample clonotypes or another clonotype from the plurality of tumour sample clonotypes that comprises a T cell receptor amino acid sequence being virtually identical to a T cell receptor amino acid sequence encoded by a T cell receptor nucleic acid sequence of the plurality of T cell receptor nucleic acid sequences comprised within the most frequent clonotype is selected as tumour-specific clonotype, wherein particularly the most frequent clonotype is absent in the non-tumour sample or can be assigned to a non-tumour clonotype that shows a frequency (within the non-tumour sample) of not more than 20 %, 15%, 10 % or 5 % of the frequency of the most frequent clonotype, and/or can be assigned to a blood sample clonotype that shows a frequency below the frequency of most frequent clonotype, and/or can be assigned to a cell-free clonotype, particularly to a cell-free clonotype that shows a frequency above 0.001% of all frequencies of the plurality of serum sample clonotypes, and/or can be assigned to another clonotype of the plurality of tumour sample clonotypes that comprises a T cell amino acid sequence being identical or virtually identical to a T cell receptor amino acid encoded by a T cell receptor nucleic acid sequence of the plurality of T cell receptor nucleic acid sequences comprised within the most frequent tumour-specific clonotypes.

In certain embodiments, the method of the invention further comprises:
- selecting 5, 10, 15 or 20 tumour-specific clonotypes from the tumour sample, wherein
   - the tumour-specific clonotypes are 5, 10, 15 or 20 of the 100 most frequent of the plurality of tumour sample clonotypes or are another clonotypes from the plurality of tumour sample clonotypes that comprise a T cell receptor amino acid sequence being identical or virtually identical to a T cell receptor amino acids sequence encoded by a T cell receptor nucleic acid sequence of the plurality of T cell receptor nucleic acid sequences comprised within the 5, 10 or 20 tumour-specific clonotypes of the 100 most frequent clonotypes of the plurality of tumour sample clonotypes, and optionally
   - the 5, 10, 15 or 20 tumour-specific clonotypes of the 100 most frequent clonotypes of the plurality of tumour sample clonotypes are absent in the non-tumour sample or can be assigned to a non-tumour-specific clonotype that exhibits a frequency (within the non-tumour-sample) of not more than 20 %, 15%, 10 % or 5 % of the frequency of the tumour-specific clonotypes of the 100 most frequent clonotype of the plurality of tumour sample clonotypes, and/or
   - the 5, 10, 15 or 20 tumour-specific clonotypes of the 100 most frequent clonotypes of the plurality of tumour sample clonotypes can be assigned to a blood sample clonotype that shows a frequency below the frequency of said tumour-specific clonotypes, and/or
   - the 5, 10, 15 or 20 tumour-specific clonotypes of the 100 most frequent clonotypes of the plurality of tumour sample clonotypes can be assigned to a cell-free sample clonotype, particularly to a cell-free clonotype that shows a frequency above 0.001% of all frequencies in the plurality of cell-free sample clonotypes, and/or
   - the 5, 10, 15 or 20 tumour-specific clonotypes of the 100 most frequent clonotypes of the plurality of tumour sample clonotypes can be assigned to another clonotype of the plurality of tumour sample clonotypes that comprises a T cell amino acid sequence being identical or virtually identical to a T cell receptor amino acid encoded by a T cell receptor nucleic acid sequence of the plurality of T cell receptor nucleic acid sequences comprised within the tumour-specific clonotypes of the 100 most frequent clonotype of the plurality of tumour sample clonotypes.

Particularly, each of the 5, 10, 15 or 20 tumour-specific clonotypes is individually compared, and particularly assigned, to the clonotypes of the above-mentioned non-tumour sample, blood sample and/or cell-free sample.

In certain embodiments,
- any one of the one, 5, 10, 15 or 20 tumour-specific clonotypes of the 100 most frequent of the plurality of tumour sample clonotypes is assigned to a non-tumour-specific clonotype, if a T cell receptor amino acid sequence encoded by a T cell receptor sequence of the plurality of T cell receptor nucleic acid sequences comprised within the tumour-specific clonotype is identical to a T cell receptor amino acid sequence encoded by a T cell receptor sequence comprised within the non-tumour sample clonotype, or if a T cell amino acid sequence of said plurality of T cell receptor amino acid sequences comprised with said tumour-specific clonotype is identical to a T cell receptor amino acid sequence comprised within said non-tumour sample clonotype, and/or
- any one of the one, 5, 10, 15 or 20 tumour-specific clonotypes of 100 most frequent of the plurality of tumour sample clonotypes is assigned to a blood sample clonotype, if a T cell receptor amino acid sequence encoded by a T cell receptor sequence of the plurality of T cell receptor nucleic acid sequences comprised within the tumour-specific clonotype is identical to a T cell receptor amino acid sequence encoded by a T cell receptor sequence comprised within the blood sample clonotype, or if a T cell amino acid sequence comprised with said tumour-specific clonotype is identical to a T cell receptor amino acid sequence comprised within said blood sample clonotype, and/or
- any one of the one, 5, 10, 15 or 20 tumour-specific clonotypes of 100 most frequent of the plurality of tumour sample clonotypes is assigned to a cell-free sample clonotype, if a T cell receptor amino acid sequence encoded by a T cell receptor nucleic acid sequence of the plurality of T cell receptor nucleic acid sequences comprised within the tumour-specific clonotype or the tumour-specific clonotypes is identical to a T cell receptor amino acid sequence encoded by a T cell receptor nucleic acid sequence comprised within the cell-free sample clonotype, or if a T cell amino acid sequence of said plurality of T cell amino acid sequences comprised with said tumour-specific clonotype is identical to a T cell receptor amino acid sequence comprised with said cell-free sample clonotype.

In certain embodiments, the method of the invention further comprises:
- selecting 5, 10, 15 or 20 tumour-specific clonotypes from the tumour sample, wherein
   - the tumour-specific clonotypes are the 5 most frequent clonotypes, the 10 most frequent clonotypes, the 15 most frequent clonotypes or the 20 most frequent clonotypes of the plurality of tumour sample clonotypes or are another clonotypes from the plurality of tumour sample clonotypes that comprise a T cell receptor amino acid sequence being identical or virtually identical to a T cell receptor amino acids encoded by a T cell receptor nucleic acid sequence of the plurality of T cell receptor nucleic acid sequences comprised within the selected 5, 10, 15 or 20 tumour-specific clonotypes of the plurality of tumour sample clonotypes, and optionally
   - the selected 5, 10, 15 or 20 tumour-specific clonotypes are absent in the non-tumour sample or can be assigned to a non-tumour-specific clonotype that exhibits a frequency (within the non-tumour sample) of not more than 20 %, 15%, 10 % or 5 % of the frequency of the selected 5, 10, 15 or 20 tumour-specific clonotypes within the plurality of the tumour sample clonotypes, and/or
   - the selected 5, 10, 15 or 20 tumour-specific clonotypes of the plurality of tumour sample clonotypes can be assigned to a blood sample clonotype that shows a frequency below the frequency of selected 5, 10, 15 or 20 tumour-specific clonotypes within the plurality of the tumour sample clonotypes, and/or
   - the selected 5, 10, 15 or 20 tumour-specific clonotypes of the plurality of tumour sample clonotypes can be assigned to a cell-free sample clonotype, particularly to a cell-free clonotype that shows a frequency above 0.001% of all frequencies in the plurality of cell-free sample clonotypes, and/or
   - the selected 5, 10, 15 or 20 tumour-specific clonotypes of the plurality of tumour sample clonotypes can be assigned to another clonotype of the plurality of tumour sample clonotypes that comprises a T cell amino acid sequence being virtually identical to a T cell receptor amino acid sequence of the plurality of T cell receptor amino acid sequences comprised within the tumour-specific clonotypes of the 100 most frequent clonotype of the plurality of tumour sample clonotypes.

Particularly, each of the selected 5, 10 or 20 tumour-specific clonotypes is individually compared, and particularly assigned, to the clonotypes of the above-mentioned non-tumour sample, blood sample and/or cell-free sample.

In certain embodiments,
- any one of the selected 5, 10, 15 or 20 tumour-specific clonotypes of the plurality of tumour sample clonotypes is assigned to a non-tumour-specific clonotype, if a T cell receptor amino acid sequence encoded by a T cell receptor nucleic acid sequence of the plurality of T cell receptor nucleic acid sequences comprised within the tumour-specific clonotype is identical to a T cell receptor amino acid sequence encoded by a T cell receptor nucleic acid sequence comprised within the non-tumour sample clonotype, or if a T cell amino acid sequence of the plurality of T cell receptor amino acid sequences comprised within the tumour-specific clonotype is identical to a T cell receptor amino acid sequence comprised within the non-tumour sample clonotype, and/or
- any one of the selected 5, 10, 15 or 20 tumour-specific clonotypes of the plurality of tumour sample clonotypes is assigned to a blood sample clonotype, if a T cell receptor amino acid sequence encoded by a T cell receptor nucleic acid sequence of the plurality of T cell receptor nucleic acid sequences comprised within the tumour-specific clonotype is identical to a T cell receptor amino acid sequence encoded by a T cell receptor nucleic acid comprised within the blood sample clonotype, or if a T cell amino acid sequence of the plurality of T cell receptor amino acid sequences comprised within the tumour-specific clonotype is identical to a T cell receptor amino acid sequence comprised within the blood sample clonotype, and/or
- any one of the selected 5, 10, 15 or 20 tumour-specific clonotypes of the plurality of tumour sample clonotypes is assigned to a cell-free sample clonotype, if a T cell receptor amino acid sequence encoded by a T cell receptor nucleic acid sequence of the plurality of T cell receptor nucleic acid sequences comprised within the tumour-specific clonotype is identical to a T cell receptor amino acid sequence encoded by a T cell receptor nucleic acid sequence comprised within the cell-free sample clonotype, or if a T cell amino acid sequence of the plurality of T cell receptor amino acid sequences comprised within the tumour-specific clonotype is identical to a T cell receptor amino acid sequence comprised with the cell-free sample clonotype.

In certain embodiments, the nucleic acid probe specifically binding to the selected tumour-specific T cell receptor nucleic acid sequence is a double stranded oligonucleotide, wherein a first strand of the oligonucleotide is complementary to the selected tumour-specific nucleic acid sequence and connected to a nanogold particle, and wherein a second strand is complementary to the first strand and bears a luminescent label, wherein the luminescence of the label is quenched by the nanogold particle if the second strand is bound to the first strand. A non-limiting example for such a probe is SmartFlare probe, obtainable from Merck Millipore (Merck KGaA, Darmstadt, Germany). In certain embodiments, the above-mentioned double stranded oligonucleotide is characterized by a length of less than 35 bases, particularly by a length of 18 to 30 bases.

In certain embodiments, the nucleic acid probe specifically binding to the selected tumour-specific T cell receptor nucleic acid sequence is a peptide nucleic acid probe, wherein a nucleobase is replaced by a dye which luminescence (fluoresce or phosphoresce) upon probe binding or hybridisation to the selected tumour-specific T cell receptor sequence. Such dye are known as intercalating dye, wherein non-limiting examples encompasses dye such as thiazole orange dye or an oxazole yellow dye. Such probes are also known as forced intercalation probes. Examples for such probes are disclosed in WO 2006/072368 A2. In certain embodiments, the above mentioned peptide nucleic acid probe is characterized by a length of less than 20 bases, particularly by a length of 18 nucleotides.

In certain embodiments, the nucleic acid probe specifically binding to the selected tumour-specific T cell receptor nucleic acid sequence is a peptide acid probe, wherein a nucleobase or a peptide acid monomer is replaced by a dye which luminesces upon probe binding or hybridization to selected tumour-specific T cell receptor nucleic acid sequence. In certain embodiments, the nucleic acid probe specifically binding to the selected tumour-specific T cell receptor nucleic acid sequence is a nucleic acid probe, wherein a nucleobase is replaced a thiazole orange dye or an oxazole yellow dye.

In certain embodiments, the nucleic acid probe specifically binding to the selected tumour-specific T cell receptor nucleic acid sequence is an oligmer comprising nucleic acid monomers and peptide acid monomers, wherein at least one of the monomers is replaced by a dye which luminesces upon probe binding or hybridization, particularly by a thiazole orange dye or an oxazole yellow dye.

In certain embodiments, the nucleic acid isolation step comprises the steps of:
a. isolating T cells from the tumour sample and isolating nucleic acids from the T cells, and/or
b. conducting a nucleic acid amplification reaction that specifically amplifies T cell receptor nucleic acid sequences.

In certain embodiments, a cells suspension is prepared from the tumour-sample, wherein the cell suspension comprises tumour cells of the tumour sample and particularly T cells that infiltrated the tumour. Such cell suspension may be prepared from the tumour sample by, for example, the gentleMACS system from Miltenyi Biotech, Bergisch Gladbach, Germany

In certain embodiments, CD3+ T cells are isolated from the tumour sample or the cell suspension, and optionally from the non-tumour sample, and/or the blood sample, wherein particularly frequencies of clonotypes are assessed or compared among the isolated cells between the tumour sample or the cell suspension and the non-tumour sample and/or the blood sample.

In certain embodiments, CD4+ T cells are isolated from the tumour sample or the cell suspension, and optionally from the non-tumour sample, and/or the blood sample, wherein particularly frequencies of clonotypes are assessed or compared among the isolated cells between the tumour sample or the cell suspension and the non-tumour sample and/or the blood sample.

In certain embodiments, CD8+ T cells are isolated from the tumour sample or the cell suspension, and optionally from the non-tumour sample, and/or the blood sample, wherein particularly frequencies of clonotypes are assessed or compared among the isolated cells between the tumour sample or the cell suspension and the non-tumour sample and/or the blood sample.

In certain embodiments, T cells comprising a T cell activation marker or secreting interferon gamma or TNF alpha are isolated from the tumour sample or the cell suspension, and optionally from the non-tumour sample, and/or the blood sample, wherein particularly frequencies of clonotypes are assessed or compared among the isolated cells between the tumour sample or the cell suspension and the non-tumour sample and/or the blood sample. and wherein particularly the T cells are stained with a specifically reactive ligand being able to bind to a T cell activation marker, interferon gamma or TNF alpha with a dissociation constant of 10⁻⁷, 10⁻⁸ or 10⁻⁹ mol/l or less, or with a nucleic acid probe being able to specifically hybridizing to an mRNA encoding the activation marker, interferon gamma or TNF alpha, and the stained T cells are isolated.

In certain embodiments, T cells isolated from the tumour sample are stained with a specific ligand binding to a T cell activation marker.

In certain embodiments, T cells isolated from the tumour sample are subjected to an expansion step, wherein the T cells are expanded under conditions of cell culture.

In certain embodiments, the T cells are stained before isolating with a specifically reactive ligand being able to bind to a T cell activation marker with a dissociation constant of 10⁻⁷, 10⁻⁸ or 10⁻⁹ mol/l or less, and the stained T cells are isolated.

A ligand according to the invention may be any molecule that binds to a target molecule or analyte with high affinity and specificity. Such a ligand may be an antibody, an antibody fragment, an antibody-like molecule or a nucleic acid aptamer molecule of 10 to 75 nucleotides in length, any of which binds to the target molecule.

An antibody fragment may be a Fab fragment, which is the antigen-binding fragment of an antibody, or a single-chain variable fragment, which is a fusion protein of the variable regions of the heavy and the light chain of an antibody connected by a peptide linker. An antibody-like molecule may be a repeat protein, such as a designed ankyrin repeat protein (Molecular Partners, Zürich).

Suitable ligands according to the above aspect of the invention may also be developed by methods such as phage display, ribosome display or SELEX, wherein polypeptide or oligonucleotides are selected due to their binding affinity to a target of interest. Additionally, the binding affinity of an identified ligand may be improved by cycles of evolution of the amino acid sequence or nucleotide sequence, and selection of the evolved inhibitors may be effected based on the required affinity.

In certain embodiments, the T cell activation marker is selected from LAG3, OX40, CD107a, CD154, PD-1, B7-H, VISTA, a member of Butyrophilin, a Butyrophilin-like protein, CD69 and CD137. In certain embodiments, the T cell activation marker is the secretion of interferon gamma or TNF alpha. Particularly, T cells secreting interferon gamma and/or TNFalpha may be isolated with, for example, the IFN-γ Secretion Assay or the IFN gamma and TNF alpha Intracellular Cytokine Staining Assay by Miltenyi Biotech, Bergisch Gladbach, Germany.

In certain embodiments, the above-mentioned isolated T cells are depleted from CD25+ regulatory T cells and/or regulatory Foxp3+ T cells before the isolation step, wherein the nucleic acid preparation is isolated from the isolated cells, or before conducting the above mentioned nucleic acid amplification. Particularly, CD25+ regulatory T cells and/or regulatory Foxp3+ T cells are depleted by staining the aforementioned cells with an anti-CD25 antibody or with a nucleic acid probe capable of hybridizing to a nucleic acid at least partly encoding CD25 or Foxp3 and sorting the stained cells, by for example a flow cytometric method.

In certain embodiments, a cell suspension is prepared from the tumour-sample, wherein the cell suspension comprises tumour cells of the tumour sample and T cells that infiltrated the tumour, CD154+ T cells are isolated from the cell suspension, nucleic acids are isolated from the isolated CD154+ T cells, and a plurality of T cell receptor nucleic acid sequences is obtained from the isolated nucleic acids. Preferably, the CD154+ T cell are labelled with an anti-CD154 antibody that is attached to an optical label such a fluorophor or to a magnetic particle or bead, and the labelled cells are isolated by means of flow cytometry or magnetic separation. Advantageously, due to the presence of tumour antigens in the cell suspension, no further stimulation is needed, for example in form of antigens, antigen fragments or antigen presenting cells. Additionally, the use of an anti-CD40-antibody to prevent a down-regulation of CD154+ T cells is also not necessary. The remaining fraction of the cell suspension may be further processed as described above.

In certain embodiments, the isolation step, wherein tumour-specific T cells are isolated from the lymphocyte preparation, is followed by an expansion step, wherein the isolated T cells are expanded under conditions of cell culture.

Particularly, the isolated tumour-specific T cells may comprise or consists of one to twenty different clonotypes. In certain embodiments, the isolated tumour-specific T cells comprise or consist of five, ten, fifteen or twenty different clonotypes. Advantageously, the isolated tumour-specific T cell are characterized by a high affinity and reactivity to the tumour or tumour cells of the patient.

T cells comprising or exposing a T cell activation marker and/or secreting interferon gamma or TNF alpha may be isolated from the tumour-specific T cell preparation isolated from the lymphocyte preparation or from the expanded T cell preparation, wherein particularly the T cells are stained for a T cell activation marker and/or for the secretion of interferon gamma or TNF alpha, and the stained cells are isolated, yielding an activated tumour-specific T cell preparation.

The tumour-specific T cell preparation of the invention or the expanded T cell preparation may also be co-cultured with a cell suspension of the autologous tumour of the patient, and T cells comprising or exposing a T cell activation marker and/or secreting interferon gamma or TNF alpha may be isolated from the above mentioned T cell preparations yielding the activated tumour-specific T cell preparation.

Such activated T cell preparation is particularly characterized by T cells with high tumour reactivity.

In certain embodiments, CD4+ T cells are isolated from the tumour-specific T cell preparation of the invention, the expanded T cell preparation or from the activated tumour specific T cell preparation.

In certain embodiments, CD8+ T cells are isolated from the tumour-specific T cell preparation of the invention, the expanded T cell preparation or from the activated tumour specific T cell preparation.

In certain embodiments, CCR7+ CD62L+ central memory T cells, particularly CCR7+CD62L+ CD45RO+ T cells, more particularly CCR7+CD62L+CD45RO+CD45RA- T cells, are isolated from the tumour-specific T cell preparation of the invention, the expanded T cell preparation or from the activated tumour specific T cell preparation yielding a tumour-specific central memory T cell preparation.

In certain embodiments, CD4+CCR7+ CD62L+ central memory T cells, particularly CD4+CCR7+CD62L+ CD45RO+ T cells, more particular CD4+ CCR7+CD62L+CD45RO+CD45RA- T cells, are isolated from the tumour-specific T cell preparation of the invention, the expanded T cell preparation or from the activated tumour specific T cell preparation yielding a tumour-specific central memory CD4+ T cell preparation.

In certain embodiments, CD8+CCR7+ CD62L+ central memory T cells, particularly CD8+CCR7+CD62L+ CD45RO+ T cells, more particular CD8+CCR7+CD62L+CD45RO+ CD45RA- T cells, are isolated from the tumour-specific T cell preparation of the invention, the expanded T cell preparation or from the activated tumour specific T cell preparation yielding a tumour-specific central memory CD8+ T cell preparation.

In certain embodiments, CCR7- CD62- effector memory T cells, particularly CCR7-CD62L-CD45RO+ T cells more particular CCR7-CD62L-CD45RP+CD45RA- T cells, are isolated from the tumour-specific T cell preparation of the invention, the expanded T cell preparation or from the activated tumour specific T cell preparation yielding a tumour-specific effector memory T cell preparation.

In certain embodiments, CD4+CCR7- CD62- effector memory T cells, particularly CD4+CCR7-CD62L- CD45RO+ T cells more particular CD4+CCR7-CD62L-CD45RP+CD45RA- T cells, are isolated from the tumour-specific T cell preparation of the invention, the expanded T cell preparation or from the activated tumour specific T cell preparation yielding a tumour-specific effector memory CD4+ T cell preparation.

In certain embodiments, CD8+CCR7- CD62- effector memory T cells, particularly CD8+CCR7-CD62L- CD45RO+ T cells more particular CD8+CCR7-CD62L-CD45RP+CD45RA- T cells, are isolated from the tumour-specific T cell preparation of the invention, the expanded T cell preparation or from the activated tumour specific T cell preparation yielding a tumour-specific effector memory CD8+ T cell preparation.

In certain embodiments, CD25+ and/or Foxp3+ regulatory T cells are isolated from the tumour-specific T cell preparation of the invention, the expanded T cell preparation or from the activated tumour specific T cell preparation yielding a tumour-specific regulatory T cell preparation.

In certain embodiments, the tumour-specific T cell preparation of the invention, the expanded T cell preparation or the activated tumour specific T cell preparation is depleted from CD25+ regulatory T cells and/or regulatory Foxp3+ T cells, particularly before the expansion step. Advantageously, the resulting tumour-specific T cells preparation is characterized by an increased tumour-reactivity and higher proliferation ability due to the absence of tumour-specific suppressive regulatory T cells.

Particularly, the tumour reactivity of the tumour-specific T cell preparation of the invention may be confirmed by :
- co-culturing at least an aliquot of any one of the above mentioned tumour specific T cell preparations of the invention, particularly an aliquot of the tumour-specific T cells that are isolated from the lymphocyte preparation in the isolation steps, with a cell suspension of the autologous tumour of the patient or a lysate of the autologous tumour together with autologous antigen presenting cells of the patient, and
- determining the amount of interferon gamma or TNF alpha produced by the T cell preparation in presence of the cell suspension, and/or determining the level of a T cell activation marker in the T cell preparation in presence of the cell suspension, particularly determining the level of OX40, CD107a, CD137, CD154, LAG3, PD-1, B7-H4, PD-1, a member of Butyrophilin, a Butyrophilin-like protein and/or CD69.

Particularly, a tumour-specific T cell preparation characterized by the secretion of interferon gamma or TNF alpha and/or the expression or increased expression of a T cell activation marker is regarded as activated tumour-specific T cell preparation.

In certain embodiments, the tumour-specific T cell receptor nucleic sequence is comprised within a nucleic acid sequence encoding the CDR3 region of a chain of the human T cell receptor, particularly the alpha chain or the beta chain of the human T cell receptor.

In certain embodiments, the tumour-specific T cell receptor amino sequence is comprised within or is the CDR3 region of a chain of the human T cell receptor, particularly the alpha chain or the beta chain of the human T cell receptor.

In certain embodiments, the tumour-specific nucleic acid sequence is comprised within an RNA. In certain embodiments, the mRNA encodes an amino acid sequence comprised within the CDR3 region of the alpha chain or the beta chain of the human T cell receptor.

In certain embodiments, the lymphocyte preparation is treated with an agent that increases the transcript level of TCR mRNA before contacting with the nucleic acid probe specifically binding to the selected tumour-specific T cell receptor nucleic acid sequence.
Advantageously, increasing the level of TCR mRNA improves the signal to noise ratio for the specific detection of the desired tumour-specific clonotypes.

In certain embodiments, the nucleic acid probe specifically binding to the selected tumour-specific T cell receptor nucleic acid sequence is characterized by an optimal target annealing temperature of not more than 45°C under the physiological conditions of the annealing medium such as the T cell cytoplasm. Advantageously, the optimal annealing temperature lies within the optimal cultivation temperature for the lymphocyte preparation. In certain embodiments, the optimal annealing temperature is between 20°C and 37°C.

According to another aspect of the invention, a method for determining the immunosuppressive effect of an anti-cancer drug is provided. The method comprises the steps of:
- providing a tumour specific T cell preparation by the method of the invention,
- contacting the tumour specific T cell preparation with the anti-cancer drug, and
- determining the functionality and/or viability of the tumour-specific T cell preparation after contacting.

In certain embodiments, determining the functionality of the tumour-specific T cell preparation comprises the steps of:
- co-culturing the tumour specific T cell preparation with a cell suspension of the autologous tumour of the patient or or a lysate of the autologous tumour together with autologous antigen presenting cells of the patient, and
- determining the amount of interferon gamma or TNF alpha produced by the T cell preparation in presence of the cell suspension, and/or determining the level of a T cell activation marker in the T cell preparation in presence of the cell suspension, particularly determining the level of OX40, CD107a,CD137, CD154, LAG3, PD-1, B7-H4, PD-1, a member of Butyrophilin, a Butyrophilin-like protein and/or CD69.

The method of the invention may be performed with a kit of parts for isolating tumour-specific T cells. The kit comprises a transfection reagent and a nucleic acid probe specifically binding to an mRNA specific for mature T cells.

The nucleic acid probe may serve as a positive control in order to identify mature T cells in a mixed population of cells and confirm the transfection efficiency.

Preferably, the transfection reagent provided in the kit is designed for the delivery of intracellular probes such as the above mentioned nucleic acid probe specifically binding to an mRNA specific for mature T cells or a custom designed T cell clone-specific probe, which are to be monitored individually at separate wavelengths.

Preferably, the transfection reagent is selected from the group comprised of streptolysin-O, nanogold, lipofectamine and polyethyleneimine.

Preferably, the mRNA specific for mature T cells is an mRNA encoding one subunit of the mature T cell receptor. Preferably, the nucleic acid probe specifically binds to a region of the mRNA encoding the constant portion of the mature T cell receptor. More preferable, the mRNA encodes the beta subunit of the mature T cell receptor.

Preferably, the transfection reagent is connected to the nucleic acid probe providing both a luminescence quenching function and effecting cellular uptake of the probe. The T cell specific mRNA probe may hybridise specifically to a T cell receptor mRNA such as TCR alpha or TCR beta mRNA, the TCR gamma or TCR delta mRNA or another mRNA or RNA, which is unique to T cells. Preferably, the probe binds to a constant region of the TCR beta mRNA comprising the conserved region coding for Cbeta1 or Cbeta2 domains and the transmembrane domain (nucleotides 181 to 709 of the Jurkat TCR beta mRNA, SEQ ID NR 101), and more preferably to a region which is highly conserved between different individuals and shares least homology with other RNA transcripts present in the cell. It is yet more preferred that the probe binds to a highly conserved region with little structural complexity in order to result in a highly efficient and specific probe hybridisation for detection such as the regions comprising nucleotides 356 to 437 and 618 to 660 of the Jurkat TCR beta mRNA (SEQ ID NR 101). Most preferably, the T cell specific RNA probe hybridises specifically to a region comprising the nucleotides 370 to 419 of the Jurkat TCR beta mRNA. This probe can serve as a positive control in order to identify T cells in a mixed population of cells and confirm the transfection or uptake efficiency. Optionally, the kit may also comprise a general uptake or transfection positive control probe which does not bind to any target RNA and is luminescent when transferred into a cell. The kit may additionally comprise a scrambled negative control probe to determine the signal background level of the probes. Preferably, the scrambled negative control probe comprises a sequence that is not present in any complementary sequence of cellular RNA.

Preferably, enrichment of sequence-specific T cell clones by cell sorting is carried out on the basis that signals from both probes (nucleic acid probe specifically binding to an mRNA specific for mature T cells and the above mentioned custom designed T cell clone-specific probe) have to be present. Cells bearing only one of either probe signals, or none at all, are discarded.

The kit may further comprise an agent that increases the transcript level of TCR mRNA. Preferably, the agent is interleukin 2 or cycloheximide.

The kit further may comprise a control T cell line and a control nucleic acid probe that specifically binds to an mRNA that encodes a unique amino acid sequence comprised within the control T cell line and not in another T cell or T cell line. Preferably, the unique amino acid sequence is comprised within the CDR3 region of the beta subunit of the T cell receptor of the control T cell line. Preferably, the control T cell line is the Jurkat cell line DSMZ no. ACC 282. Preferably, the control nucleic acid probe that specifically binds to an mRNA that encodes a unique amino acid sequence comprised within the control T cell line and not in another T cell or T cell line consists of or comprised a nucleic acid sequence characterized by SEQ ID NR 101 (Human T-cell receptor active beta-chain mRNA from Jurkat cell line (clone JUR-beta-1)).

The kit further may comprise means for isolating T cells from blood. Preferably, the means is a magnetic bead comprising an antibody against CD3, CD8 or CD4. More preferable, the means is an antibody against a T cell specific marker such as for example CD3, CD4 or CD8, wherein the antibody suitable for fluorescence based flow cytometry.

The kit may further comprise means for isolating T cells from blood. Preferably, the means is a probe specific for the mRNA detection of CD3, CD8 or CD4, a particularly a labelled nucleic acid probe being able to specifically hybridizing to an mRNA encoding CD3, CD8 or CD4. The means may also be a probe specific for the mRNA of a T cell specific marker, particularly a nucleic acid probe being able to specifically hybridizing to an mRNA encoding a T cell specific marker, such as for example CD3, CD4 or CD8, wherein the probe is suitable for luminescence or fluorescence based flow cytometry, particularly by means of a luminescent or fluorescent label attached to the probe. The means may also be a probe specific for the mRNA of a T cell specific marker such as for example CD3, CD4 or CD8, wherein the probe is suitable for detection by PCR, wherein particularly the probe is a primer or a primer pair being able to specifically annealing to a nucleic acid encoding the T cell specific marker, particularly such that an only in cells comprising the nucleic acid encoding the T cell specific marker an amplification product of the PCR is obtainable.

The tumour-specific T cell preparation obtainable by the method of the invention may be used in a method for manufacturing an artificial tumour-specific T cell receptor. The method comprises the steps of:
- providing any one of the tumour specific T cell preparations of the invention by the method of the invention, particularly providing an activated tumour-specific T cell preparation,
- isolating an individual tumour-specific T cell from the tumour-specific T cell preparation;
- determining the CDR3 regions of both subunits of the T cell receptor of the isolated individual tumour-specific T cell;
- preparing an artificial T cell receptor comprising the determined CDR3 regions of both subunits.

Preferably, the artificial T cell receptor comprises a moiety, by which the receptor can be isolated. Preferably, the artificial receptor comprises the CDR3 regions of the alpha chain and the beta chain. Preferably, the artificial receptor comprises the CDR3 regions of the gamma chain and the delta chain. Preferably, the artificial T cell receptor is comprised within a tetramer of T cell receptors, wherein at least one or all monomers comprise the determined CDR3 regions.

Preferably, the artificial T cell receptor is recombinantly prepared, wherein a nucleic acid encoding the artificial T cell receptor is introduced into a host cell and expressed yielding the artificial T cell receptor. Preferably, the nucleic acid is under control of a promoter operable in the host cells. Preferably, the host cell is a human CD3+ cell. Such CD3+ cell comprising the artificial T cell receptor may be used for adoptive transfer, particularly for treating cancer.
Preferably, the artificial T cell receptor is functionally exposed on the surface of the host cell.

The tumour-specific T cell preparation obtainable by the method of the invention may also be used in a method for isolating cells bearing a tumour-specific antigen. The method comprises the steps of:
- providing a tumour specific T cell preparation by the method of the invention,
- isolating an individual tumour-specific T cell from the tumour-specific T cell preparation;
- determining the CDR3 regions of both subunits of the T cell receptor of the isolated individual tumour-specific T cell;
- preparing an artificial T cell receptor comprising the determined CDR3 regions of both subunits, wherein the artificial T cell receptor comprises a moiety, by which the artificial T cell receptor selectively can be isolated,
- contacting the artificial T cell receptor with cells bearing antigens,
- isolating cells that bind to the artificial receptor.

Preferably, the T cell receptor of the isolated individual tumour-specific T cell comprises an alpha-chain and a beta chain, wherein the CDR3 region of both chains are determined. Alternatively, the T cell receptor of the isolated individual tumour-specific T cell comprises a gamma-chain and a delta-chain, wherein the CDR3 region of both chains are determined.

Preferably, the moiety is a biotin or a magnetic bead. Preferably, the cells to be isolated are obtained from the blood of a subject. Preferably, the antigen being recognized by the artificial T cell receptor from at least one of the isolated cells is identified, particularly by mass spectroscopy.

The invention is further illustrated by the following examples and figures, from which further embodiments and advantages can be drawn. These examples are meant to illustrate the invention but not to limit its scope.

### Description of the figures

- Fig. 1: shows in A: scheme of the CDR3 region of the alpha-chain of the human T cell receptor. B: Same as A, but for the beta chain of the human T cell receptor. C: Principle of amplification of the genomic region containing CDR3. PCR primers are specific for the repertoires of V/J - segments and amplify small regions of the V- and J-segments with the CDR3 region between them.

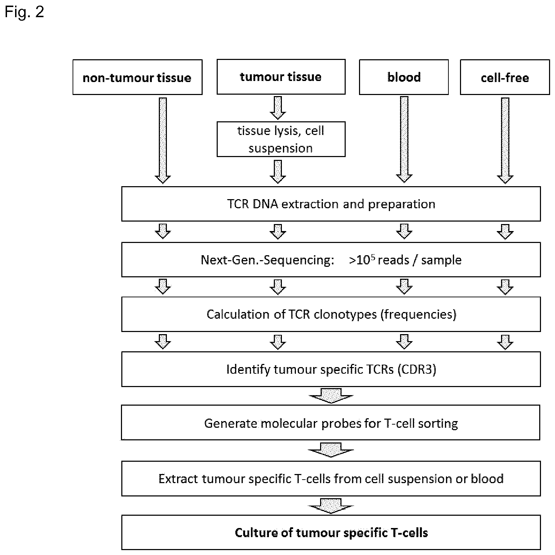

- Fig.2: shows a flow diagram depicting the principles of the method of the invention.

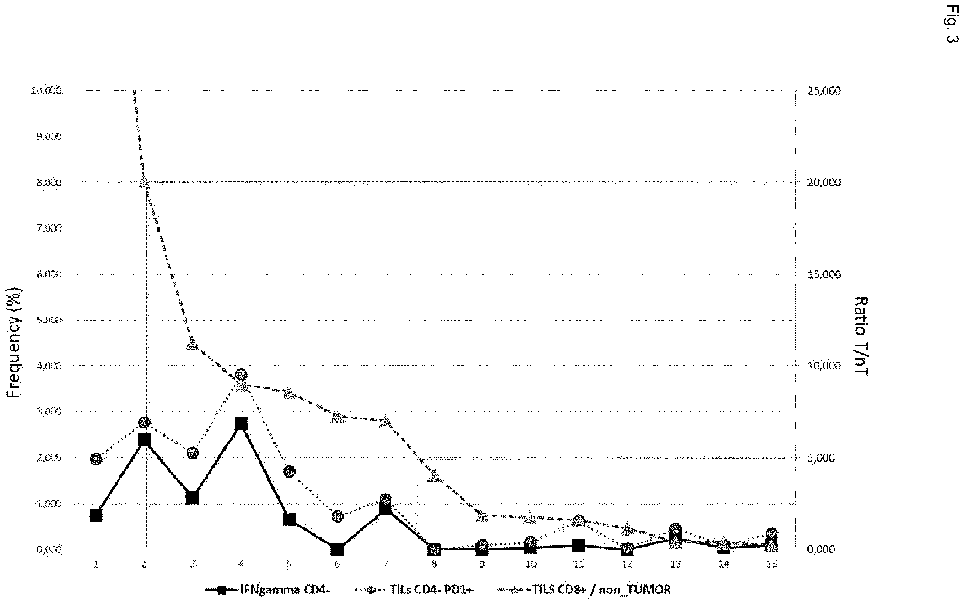

- Fig. 3: shows how the ratio of TILs and non-tumour T cells separates TILs into highly tumour-reactive and minor tumour-reactive T cell clonotypes. The dashed line depicts the tumour vs non-tumour ratios (T/nT), the dotted and solid lines show the frequencies of T cell clones carrying/expressing specific activation markers (PD-1, IFNgamma). For a threshold ratio T/nT > 5 almost all tumour-reactive clones are correctly predicted, for T/nT > 20 2 clones are selected and correctly predicted as tumour-reactive.

### Examples

### Example 1: Identification of tumour-specific T cells and tumour-specific sequences by comparative sequence analysis

Available Next-Generation-Sequencing (NGS) technology was used to sequence many thousand TCR beta CDR3 regions (one TCR corresponds to one T cell) per sample in high-throughput, whereby sequencing libraries for the CDR3-region of human TCR beta were generated. The resulting sequences were analysed by bioinformatics tools and the final result per sample is a table listing the respective clonotypes (types of T cells with the same TCR beta).

The CDR3 region of the T cell receptor is determined by the constant V- and J-segments (see Fig. 1) and the highly variable regions between them. Due to this structure one and the same CDR3 amino acid sequence can be encoded by multiple nucleotide sequences, which may be even composed of distinct V/J-segments. The occurrence of multiple ( >1 ) nucleotide CDR3 sequences per one amino acid sequence among the set of tumour-specific T cells and potential tumour-reactive T cells (TRTC) is a strong hint that the T cells with the respective CDR3 amino acid sequence is reactive with respect to the tumour cells.

CDR3 sequences, with this property are always added to the final selection of CDR3 sequences, if their score (see table 1 below) is greater or equal to 1000.

### Scoring schema for identification of tumour specific T cells (TSTCs) by sequence profiling and bioinformatics analysis.

The method is based on a scoring system given below (Table. 1), where one or several samples are taken and analysed in parallel, and the best scores are gained for clonotypes with respective ratios of frequencies per sample type. Generally, tumour infiltrating lymphocytes were identified by the following series of analysis steps:
a.) Next-Generation-Sequencing (NGS) is performed starting from tumour samples. Tumour samples are either defined as one sample or a set of replicate samples taken from tumour tissue. In practice, the material to analyse the TCRs is obtained by either
   i.) Selecting distinct biopsies or different areas of one biopsy. This may be assisted by immunohistochemical staining, wherein particularly tumour reactive T cells (TRTCs) are immunohistologically stained with preferably T cell activation markers such as LAG3, OX40, CD107a or CD137 and stained regions are selected for DNA extraction;.
   ii.) Lysis of tumour tissue e.g. by bead-based technologies for preparation of single cell suspensions as starting point for TCR analysis. Single cell suspensions may be separated in different T cell subsets, e.g. CD4+ and CD8+ subsets.
      In addition, tumour samples may be stored under cell-preserving conditions as resource for cell materials.
**b)** Non-tumour samples from the same patient are selected from tissue/regions adjacent to tumour sample, if possible in replicates, where possible from distinct tissue spots and α- and/or β-TCR / CDR3 NGS sequence analysis was performed.
**c)** Blood samples (cellular components) are taken from the same patient: By standard hematological fractionation cellular components were isolated from full blood, α and/or β TCR / CDR3 NGS sequence analysis was performed and TCR-profiles were calculated.
**d)** Serum, plasma or other cell-free biological fluids/tissues are taken from the same patient, optionally by additional removal of cellular components by standard hematological fractionation. The presence of TCR-specific DNA in cell-free samples can be a strong hint for apoptotic processes against T cells. If a significant amount of clonotypes (see below, Table.1) is found in cell-free sample and tumour, the score contributes to the scoring table (Table. 1).
**e)** Optionally, 2 or more time points in the course of the patients treatment/diagnosis are used for screening - i.e. samples are taken at distinct time points from blood, etc. (see 2.a-d.). This will enable e.g. diagnosis of relapse or detection of new TSTCs directed against metastases etc.

### Principles of Clonotype (sequence cluster) calculation from NGS data

a. CDR3 regions of the TCRα- and TCRβ-chain are sequenced with NGS technology. A 2-step PCR method (as disclosed in WO 2014/096394 A1) was used with TCRα or TCRβ primers binding specifically to the V- and J-segments adjacent to the CDR3 region. DNA was used as starting material for the NGS process.
b. Per sample a large (>10⁵) number of reads (nucleotide sequences) is commonly produced by NGS, the reads are merged into clusters of virtually identical nucleotide sequences, the number of reads per cluster determines the frequency of that cluster, where frequency of a cluster is measured in percentage of reads of this sample falling into this cluster.
c. Clustering is very conservative and works in two rounds: In a first step all reads with 100% nucleotide sequence identity are counted as 1 cluster with the cluster sequence being identical to the read sequence. In the second step clusters are compared among each other and those with
   i. not more than 1 bp mismatch and
   ii. where one cluster (cluster A) has at least 20x more reads than the other cluster (cluster B)
   are merged and regarded as identical to cluster A. The nucleotide sequence clusters are regarded as equivalent to clonotypes.
d. the nucleotide sequence clusters are translated to amino acid sequences (peptides) and tabulated. Each cluster is regarded as one clonotype with a frequency as defined in (1.b). The frequency is a direct measure of the frequency of the respective T cell in the sample.
e. Clusters (Clonotypes) sharing a virtually identical amino acid sequence are merged into clustertypes, the frequency of a clustertype is identical to the sum of frequencies of nucleotide sequence clusters being elements of said clustertype.

The ranking of TSTC (tumour-specific T cell) score is given in 4 digit numbers 1011,1010,1001,1000 (from best to lowest), all other cases are excluded.

Within the columns the scoring is defined as follows

**Table 1. The scoring table for selection of best TSTC-clonotypes.**

| T cell CDR3 nucleotide sequence | TSTC score | A: Tumour tissue | B: non-tumour tissue | C: blood cellular | D: cell-free DNA |
|---|---|---|---|---|---|
| Seq1 | 1011 | 1 | 0 | 1 | 1 |
| Seq2 | 1110 | 1 | 1 | 1 | 0 |
| Seq3 | 1001 | 1 | 0 | 0 | 1 |

Within each column (1 column per tissue type) simple binary scores are given per CDR3 nucleotide sequence (Seq1,2,3,..): '1' means, that the respective CDR3 DNA sequence occurs, '0' means it is either absent or found in low levels. The precise definition is given below. The binary scores are combined to a 4-digit TSTC score as shown in Table 2. The ranking of accepted TSTC scores is given by their natural order: 1011, 1010, 1001, 1000 (from best to worst), all other scores are excluded. The TSTC scoring schema also includes cases, where e.g. no blood sample exists, i.e. columns C and D would be filled with '0', or where there are only tumour samples, i.e columns B, C and D would be filled with '0'. The binary scores per column (=tissue type) is defined as follows:
A: score=1: The sequence (seq1,2,..) is among top 100 clonotypes (sorted by their frequency from highest to lowest) and shows an intact open reading frame, i.e. no stop codons or frame shifts are found, otherwise score=0
B: score=0: The sequence (Seq1,2,3,..) is either absent in non-tumour sample or found identical in non-tumour sample, but with a ratio R = pepB/pepA less or equal to 0.2, 0.15, 0.1 or 0.05, if pepB is the frequency in non-tumour sample and pepA is the frequency in tumour sample. In all other cases score=1.
C: score=1: The frequency of sequence Seq1,2,3,..is lower than the frequency of the respective sequence in tumour tissue (A), otherwise score=0
D: score=1: The frequency of the sequence Seq1,2,3,.. is higher than 0.001% of all sequences derived from cell-free DNA, otherwise score=0
E: For CDR3 sequences Seq1,2,3,.. already selected by their TSTC score (see A-D above), optionally the following additional filter can be applied: if identical CDR3 amino acid sequences from A (tumour sample) are encoded by different CDR3 nucleotide sequences Seq1,2,3,.. this is indicative of convergent recombination and highly immunogenic tumour antigens. Clonotypes with this property are given the highest TSTC score = 1011.
**F:** For CDR3 sequences Seq1,2,3,.. selected by their TSTC score (see A-D above), optionally the following additional filter is applied: CDR3 sequences translated into amino acid sequences from A (tumour sample) may be compared among each other by protein alignment (blast) using amino acid substitution matrices like BLOSUM80 or BLOSUM62.
Amino acid sequences being highly similar with maximal 1 mismatch are grouped into similarity clusters and each member (Seq1,2,3..) of the similarity cluster is given the same TSTC-score as the best scoring CDR3 sequence in that similarity cluster.

Within each score group 1011, 1010, 1001, 1000 (from best to worst) the CDR3 nucleotide sequences are sorted by their frequency from highest to lowest and from the final sorted list the top 1-100 CDR3 nucleotide sequences are selected as candidate set for the next steps. In other embodiments the top 5, 10, 15, 20, 30, 40 or 50 CDR3 sequences are selected. But preferred are 20.

The best scoring clonotypes (up to 20) are stored as
a. template for the synthesis of fluorescent tags
b. template for the synthesis of novel tumour-specific T cells by gene transfer.

The above mentioned tumour sample may be a single sample or a set of samples from the patient. Therefore, a plurality of tumour samples from one patient may be analysed as described above. Clonotypes that occurred in different tumour samples are preferred over clonotypes that occur in the minority of tumour samples.

### Example 2: Target sequence identification

Once the TCR nucleic acid sequences of the T cell clones of interest are identified, further steps are necessary to define the ideal target sequences that can be used for detection and enrichment of said T cell clones. At first, the specific genomic sequence is used to generate an at least partial mature mRNA sequence in order to discard any intronic parts that cannot serve as target for specific recognition by probes in living cells. Said clonal mature mRNA sequences are then compared with the complete transcriptome including the mature TCR mRNA of all other T cells not belonging to the clones of interest in order to identify only target-specific sequences. Particularly, mainly the CDR3 regions of the TCR mRNA are different on a clonotype basis and display difference to other transcripts in the cell as well. The target-clone specific sequences can be further analysed for structures that interfere with probe hybridisation. This can be performed either experimentally by checking the hybridisation efficiency, or by computational analysis using tools such as MFold or UNAFold (http://mfold.rna.albany.edu/). It is preferred that the region with the highest delta G (closer to zero) is chosen for probe design.

### Example 3: Probes for in vivo detection

Having identified the target-specific DNA sequences of the clones of interest, probes for the detection in living T cells can be designed. Different probe formats can be used. However, depending on the length of the target-specific region multipartite probes or single oligonucleotide probes may be chosen. Molecular beacons can be designed to hybridise to target RNA at a temperature compatible with cell cultivation. Software packages such as Beacon DesignerTM developed by PREMIER Biosoft International (www.premierbiosoft.com) are commercially available. Molecular probes can have a pair of mostly terminally conjugated dyes that are quenched due to formation of a stem while not hybridised to a target. Upon target hybridisation, the terminal stem is opened and the dyes are unquenched. However, in a complex environment such as the cytoplasm of living cell, unspecific interaction with proteins may open up the stem resulting in false positive signals. In order to enhance the specificity of a molecular beacon, a second molecular beacon can be designed to hybridise directly adjacent to the first molecular beacon as a bipartite probe. If the termini of both beacons are specifically hybridised within a distance of up to four nucleotides, a highly specific FRET signal between the adjacent dyes can be used to detect the hybridisation event. A multipartite recognition can also be achieved with unstructured probes other than in a molecular beacon format. The so-called SmartFlare is a new probe format that combines the properties of nanogold particles of enhancing cell transfection and quenching of fluorescent dyes which are immobilised in close proximity to the gold surface. Thus a simple probe complementary to a given target sequence bearing a single fluorescent dye is sufficient. The dye of the probe is effectively quenched when hybridised to another nucleic acid which is anchored to a gold nanoparticle. Upon transfection into a living cell, the probe is able to be displaced by specific hybridisation to its complementary target sequence, thus becoming fluorescent by detachment from the nanoparticle. Forced intercalation probes (FIT-probes, WO 2006/072368 A2) are a yet more desirable format. The intercalation of certain dyes between nucleobases of the formed probe-target duplex restricts the torsional flexibility of two heterocyclic ring systems of said dyes. As a result, FIT probes show strong enhancements of fluorescence upon hybridization. A FIT-probe with thiazol orange (TO) has been reported to yield a signal in the presence of complementary DNA or RNA with at least 25-fold enhancement of fluorescence intensity. More recently, it was discovered that dual fluorophore-labelled PNA FIT-probes are extremely responsive and bright hybridization probes for the sensitive detection of complementary DNA or RNA by up to 450-fold enhancements of fluorescence intensity. In contrast to existing DNA-based molecular beacons, this PNA-based probe form does not require a stem sequence to enforce dye-dye communication. Oxazole yellow (YO) containing FIT-probes have been shown to discriminate against single base mismatches by attenuation of fluorescence and may be used if single-nucleotide polymorphisms (SNPs) have to be detected specifically. Furthermore, it has been demonstrated that addition of C-terminal lysine residues enables uptake into living cells without the need for any further transfection reagent. Although FIT-probes have been originally published as PNA-based probes, FIT-probes based on DNA and LNA have been developed as well. DNA FIT probes with dual dye combinations such as TO and YO were found to be very specific in vivo exceeding the brightness of molecular beacons. In addition, so-called mixmers of PNA and DNA have become commercially available. Thus it is possible to optimise specificity, solubility and melting temperature to generate FIT-probes for the efficient fluorescent detection of living T cell clones.

Depending on their base composition and type of nucleotide, different lengths will be optimal for cytoplasmic recognition of target TCR mRNA. It is preferred that the target-specific hybridising part of standard PNA probes are shorter than 20 bases and standard DNA probes less than 35 bases. However, many non-standard modifications exist which can be used to elevate or decrease the specificity and/or melting temperature of nucleic acids. For example, abasic sites and unlocked nucleic acids may decrease melting temperature and increase specificity. LNA has a higher melting temperature than DNA and is protected from nuclease degradation. Even modified bases such as inosine which may pair to three of the four natural bases can be used to fine-tune intracellular recognition.

Due to the vast complexity of nucleic acid structures that may arise in vivo, it is preferred to choose monopartite probes that do not rely on structures for their functionality. Provided with the preferred specific target region previously identified by comparison to other cellular transcript and structural accessibility, the skilled person would know how to design an appropriate probe using respective bioinformatic design tools.

### Example 4: Probe uptake mechanisms

Nucleic acids can be taken up into living cells by a multitude of mechanisms. The process is called transfection, when eukaryotic cells are targeted by a non-viral mechanism. Three general transfection methods are available called chemical-based transfection, non-chemical transfection and particle-based transfection. The chemical-based transfection methods make use of additional chemicals that facilitate cellular uptake. Such additives can be salts, polymers, liposomes and nanoparticles or a mixture thereof.

The efficiency of transfection methods is strongly dependent on the size and form of nucleotides as well as cell-type. Small nucleic acids can be efficiently transfected by pore-forming compounds. Streptolysin-O (SLO) reversible permeabilisation is an efficient method to deliver small nucleic acids such as siRNA or molecular beacons and is compatible with T cells. In addition, T cells have been effectively transfected by gold nanoparticle conjugates with labelled probes such as SmartFlares. Also Lipofectamine® was effectively used for transfection of small oligonucleotides such as siRNA or antisense RNA into T cells.

Especially PNA can be simply elongated by a few lysine residues to achieve cellular uptake without any additional transfection reagents. Preferred non-chemical transfection methods are magnetofection and electroporation. More preferred is cell squeezing which was demonstrated to deliver a range of material, such as carbon nanotubes, proteins, and siRNA, to over 20 cell types, including embryonic stem cells and naïve immune cells. The microfluidic platform of Sqz Biotechnologies Co. allows for the high throughput and efficient transfection of T cells without the need of transfection reagents.

### Example 5: Increase of specific signals:

The level of TCR mRNA transcripts in a cell can be increased in order to provide a higher signal to noise ratio for the specific detection by preferably monopartite probes. The inventors have discovered that a previous overnight treatment of T cells with 10 U/ml IL-2 can increase the transcript level of TCR mRNA. Alternatively, the TCR mRNA level can be increased with cycloheximide. The protein synthesis inhibitor cycloheximide (CHX) induces a 20-fold increase in mature TCR-alpha transcript accumulation without a concomitant increase in TCR-alpha gene transcription suggesting that CHX reverses the nuclear post-transcriptional events which prevent mature TCR-alpha mRNA accumulation. CHX also induces full length TCR-beta transcripts greater than 90-fold while TCR-beta gene transcription increases only 2- to 4-fold (Wilkinson & McLeod EMBO J. 1988 Jan; 7(1): 101-109.). Since the inhibition by CHX was found to be reversible, it is preferred to perform only a brief period of incubation sufficient to raise the mRNA level for detection by probes by a factor of 10.

Another alternative is to activate T cells and incubate activated cells for a period of 24h, thereby doubling the amount of mRNA for specific detection.

### Example 6: Array-based method for sequence-specific isolation of T-cell clonotypes

T cell clonotypes, particularly the tumour-specific clonotypes of the invention may be isolated by the following iterative approach comprising diluting T-cells in clonotype-positive wells and repeating the method until a homogeneous T-cell population comprising the desired clonotype is generated.

The nucleic acid based assay may be performed by either direct probe hybridisation in cells or specific amplification of target sequences for detection. The direct probe hybridisation can be carried out using dead cells (analysis by Microscope, microtiter well scan, or FACS) or live cells (FIT-probes, etc. analysis by Microscope, microtiter well scan, or FACS). The amplification reaction is preferably a (RT-)PCR on array samples.

Suitable samples comprise, without being restricted to, extracellular nucleic acid (cell free), supernatant or array surface may comprise cell-free nucleic acid that can be used for specific and sensitive identification without killing valuable cells. This may allow a more rapid isolation of target cells without the need for cell division, crude lysate derived from an aliquot of the array (well or position), purified nuclear DNA, purified mRNA.

Different array formats that are compatible with the method comprise, without being restricted to.
- microtiter wells (at least 2 wells, preferably more than 6 wells, more preferably between 128 and 384 wells)
- embedded array. The cells are preferably embedded by a matrix that hinders free diffusion of cells and hence preserves the coordinates of an initially deposited clone. The matrix preferably comprises polymers such as agarose, gelatine or polyacrylamide.
- random array. The random array is not dependent on a preformed grid to contain samples.
- Microfluidic. A microfluidic array can be specifically formed by channels and other structures that may allow handling steps comprising initial cell distribution, washing, dilution, expansion and retrieval of cells and/or nucleic acids. A non-liming example of such microfluidic array is shown at http://www.biomemsrc.org/research/cell-tissue-microengineering/living-cell-array.

Depending on the frequency of the target clonotype in a sample, an appropriate limiting dilution may be performed in order to ensure that not more than one clonotype is present in a given diluted aliquot or well.. Even single cells can be directly entrapped in an array with communicating microwells by dielectrophoresis (the process whereby dielectric particles, such as living cells, in a non-uniform electrical field, are prevented from leaving microwells).

Cultivation conditions may be chosen to optimise the proliferation of cells. This may comprise the co-cultivation with feeder cells that prevent the cell death or lack of growth of single cells that were diluted from a sample. In addition, cytokines and nutrients can be included in the media to further enhance cell division. Depending on the desired T-cell type different optimal conditions may be applied. In some cases it may be advantageous to trigger or enhance the production of exosomes by target T-cells as a source of cell-free nucleic acid for testing, wherein the aforementioned production of exosomes may be triggered by activation of said T-cells by antigen presenting cells or contacting with IL-2.

Given that a population of 10⁶ T-cells isolated from a blood sample contains 1 T-cell of interest with a previously identified CDR3, an array-based screening procedure can be employed. Atypical RT-PCR machine can handle 384-well microtiter plates. The 10⁶ T-cells can be equally diluted into 384 wells, amounting to about 3x10³ cells per well, one of which harbours the clonotype of interest. After 4 divisions each cell would be present in 8 copies, whereby the clone of interest is ideally still present in the same 1:3x10³ ratio as before. One half of the supernatant is withdrawn and the DNA (or mRNA) is purified while keeping the coordinates in the aliquot 384 microtiter plate. (For automated DNA or RNA purification methods see here: https://www.promega.de/resources/tools/automated-methods/). The samples are subjected to RT-PCR to detect the coordinates of the target clonotype. Once the coordinates are known, the aliquot of living cells (4 in 10⁴ cells) from the coordinate is diluted into another 384 well plate. Now up to 4 wells may contain the target clonotype with a ratio of about 1:30. After 4 cell divisions, the wells are screened again by PCR and the aliquot of positive wells (4 in 120) may be diluted again into a microtiter plate with appropriate dimensions to yield clonal cultures. All positive wells may be diluted into one 384 plate, even at the potential loss of some target cells. After further 4 cell divisions, the positive clones can be quickly identified in an aliquot by RT-PCR or other probe-based methods. In order to optimise growth conditions appropriate media with cytokines and feeder cells (which can be easily distinguished by surface antigens) can be used.

In the case that more positive clones are present in the original sample of 1 million cells, the procedure can process more of these to have a higher chance of obtaining proliferating clonotypes for expansion.

The cell division rate and the capacity to expand of target clonotypes is limiting for this procedure. It may take 24-48 h for a CD4+ T-cell to divide for the first time in vitro whereas subsequent divisions typically occur much faster. If one T-cell division takes 1 day, then the procedure with 3 arrays will take at least 2 weeks. However, for effective treatment prior expansion of clonotypes is imperative. The above method intrinsically favours the isolation of proliferative T-cells. If the cell-free supernatant contains TCR-beta mRNA, then isolation may proceed faster by non-destructive analysis of the supernatant.

### Example 7: Efficiency of the sequence based prediction of tumour reactive T cell clonotypes

In table 2 the 100 most frequent clonotypes are exemplary depicted (**NN:** clonotype could not be measured in non-tumor tissue).The shown 100 most clonotypes equate to SEQ ID 01 to 100. In Table 3, 4 and 5 the most frequent 5, 10 and 15 clonotypes, respectively, of freshly isolated TILs from NSCLC-tumour samples are shown (column E), characterized by unique CDR3-beta peptides (column A) and their flanking V- and J- segments (columns B and C). IFNgamma secretion assay after co-incubation of expanded CD4- TILs with autologous tumour cells reveals the presence of a significant number of clearly tumour-reactive CD8⁺ clones within the TOP 5, 10 and 15 (column H in Table 3-5, IFNgamma > 0,25).

In table 3 the CDR3 region (peptide) of the beta T cell receptor is shown as found identical in different samples of the same tumour patient (NSCLC) for the top 5 TILs CD8⁺ clonotypes. V-segments and J-segments are denoted according to IMGT nomenclature. The CDR3 frequencies as percent of sequence reads are given for the following samples: **BLOOD:** T cells were extracted from blood (PBMCs).**TILs CD8⁺** : T cells from tumour (TILs) were extracted and sorted with respect to CD8⁺. **non-TUMOUR CD8⁺:** lung tissue samples were taken distal from tumour and T cells extracted and sorted (CD8⁺). **TILs CD4⁻ PD1⁺:** T cells were extracted from tumour, depleted with respect to CD4 and sorted by a PD1 specific antibody, which results in the fraction of activated cytotoxic T cell. **IFNgamma CD4⁻:** T cells originally extracted from tumour were kept in culture for 20 days, co-cultured with tumour cells and measured for secretion of IFNgamma by a commercial assay, which shows the activation of T cells as a direct measure of tumour reactivity. **TILs CD8⁺ / non-TUMOUR CD8⁺:** Ratio of frequencies found in TILs and non-tumour samples (CD8⁺). For ratios >5 (>20) there is a clear prevalence of highly tumour reactive clonotypes as shown simultaneously by the IFNgamma and PD1+ frequencies.

Table 4 shows the same as in Table 3, but for the top 10 TILs CD8⁺ clonotypes. Again, for TILs CD8⁺ / non-TUMOUR CD8⁺ ratios >5 (>20) there is a clear prevalence of highly tumour reactive clonotypes as shown simultaneously by the IFNgamma and PD1+ frequencies.

Table 5 shows the same as in Table 3, but for the top 15 TILs CD8⁺ clonotypes. Again, for TILs CD8⁺ / non-TUMOUR CD8⁺ ratios >5 (>20) there is a clear prevalence of highly tumour reactive clonotypes as shown simultaneously by the IFNgamma and PD1+ frequencies.

These high frequency, tumour-reactive clones can be predicted and identified applying the ratio of frequencies between tumour and non-tumour CD8⁺ T-cells (T/nT ratio, column I).

In table 3, within the Top 5, the T/nT ratio of > 20 identifies clone 2, the ratio of > 5 the clones 1, 2 and 4. Thus, all tumour-reactive clones within the Top 5 are identified using the T/nT ratio.

In table 4, within the TOP 10, the ratio of > 20 identifies the clones 2 and 7, the ratio of > 5 the clones 1, 2, 4, and 7 as tumour-reactive.

In table 5, within the TOP 15, the ratio of > 20 identifies the clones 2 and 7, the ratio of > 5 the clones 1, 2, 4, 11 and 12, comprising all tumour-reactive clones within the 15 most frequent CD8⁺ TILs.

In table 6 the comparison of 3 methods of identifying tumour specific T cells is shown for IFNgamma frequencies >0.25 : **a)** only tumour tissue is used, i.e. all statistics refer to TILs alone. **b)** TIL (CD8+) frequencies are compared to T cells (CD8+) from non-tumour tissue and only TILs with a tumour/non-tumour ratio of >20 are used. **c)** TIL (CD8+)frequencies are compared to T cells (CD8+) from non-tumour tissue and only TILs with a tumour/non-tumour ratio of >5 are used. It is obvious that the best results in terms of number of tumour reactive T cells and strength of measured IFNγ signal are reached by the tissue comparisons, preferably with a ratio >5 .

For a selection of TOP 15 clonotypes, the prediction of tumour-reactivity is shown to be quite accurate in figure 1: The rule ratio T/nT > 5 separates the T cell clonotypes efficiently into highly tumour-reactive and minor tumour-reactive ones. For a ratio T/nT > 20 the prediction of tumour-reactivity is 100% correct, with the price to miss a number of truly tumour-reactive clonotypes.

### Example 8: TCR-sequence-specific isolation of tumour-reactive clonotypes

The identification of tumour-reactive clonotypes characterized by specific sequences (CDR3beta, Vbeta segment) opens the way for sequence specific strategies for enrichment of tumour-reactive clones.

6 weeks after resection of the tumour (NSCLC), blood was taken from the patient and PBMCs prepared. Part of the PBMCs were sequenced for TCRbeta. An aliquot of the PBMC preparation was incubated with a Vbeta-30 antibody specific for clone 2 of the patient.

Result are given in Table 7 showing the enrichment of desired T cell clones by sequence specific sorting with respective Vbeta-segment specific antibodies. **CDR3 peptide:** The CDR3 region (peptide) of the beta T cell receptor. **V-segments** and **J-segments** are denoted according to IMGT nomenclature. **TILs CD8⁺:** T cells from tumour (TILs) were extracted and sorted with respect to CD8⁺. **IFNgamma CD4⁻:** T cells originally extracted from tumour were kept in culture for 20 days, co-cultured with tumour cells and measured for secretion of IFNgamma by a commercial assay, which shows the activation of T cells stimulated by the respective antigens. **TILs CD4⁻ PD1⁺:** T cells were extracted from tumour, depleted with respect to CD4 and sorted by a PD1 specific antibody, which results in the fraction of activated cytotoxic T cell. **TILs CD8⁺ / non_TUMOUR CD8⁺**: Ratio of frequencies found in TILs and non-tumour samples (CD8⁺). **Vbeta-AB:** The respective Vbeta-segment specific antibody used for capturing of dedicated clonotypes. **Freq. in Vbeta AB selection:** Frequency of respective clonotype after using bead separation with a Vbeta-specific antibody. **Freq. in PBMC:** Frequency of respective clonotype in peripheral blood. **Enrichment factor:** The ratio of clonotype frequencies after separation by Vbeta-antibody versus frequency in peripheral blood. For the second clonotype there was no detectable frequency in peripheral blood, so that the enrichment factor could only be guessed by employing the lower threshold of 0,001% as the highest possible value.

Clone 2 was measured in the PBMCs of the patient with a frequency of 0,097% (column J). Using the Vbeta-30 antibody and beads separation the frequency was increased to 5,52% (column I). This is an enrichment factor of 57,0, setting the stage for full isolation of the clone with standard procedures from peripheral blood of the patient.

### Methods and Materials

The following experiments were approved by the Berlin chamber of physicians ethics committee (Nr. Eth-62-15).

### Initiation and Expansion of T-Lymphocyte Microcultures from Tumour and Lung Tissue Fragments

Each tumour specimen was dissected free of surrounding normal tissue and necrotic areas. Approx. 1 g cubes from tumour and normal lung tissue were cut into small chunks measuring about 2-3 mm in each dimension. Sliced tumour (and also non-tumour) biopsies were subjected to a commercial mechanical/ enzymatic tissue dissociation system (GentleMACS, Miltenyi Biotec, Bergisch-Gladbach, Germany), using the Tumour Dissociation Kit (Miltenyi Biotech) and following the manufacturer's instructions.

After GentleMACS disaggregation, cell suspensions were passed through 70-µm strainers. Aliquots of tumour cells were taken and cryopreserved in 10% DMSO (Sigma-Aldrich) and 90% FCS (Life Technologies) for later use. The remaining cell suspension was subjected to density gradient centrifugation using a 40%/80% step gradient of Percoll® (GE Healthcare Europe GmbH) in PBS/RPMI 1640. T-lymphocytes were harvested from the interphase and washed in complete medium (RPMI 1640, Lonza). Subsequently, T-lymphocyte were placed in a 24-well tissue culture plate with 2 mL of recovery medium (RM) at a concentration of 0.5x10⁶ cells/ml. RM consisted of RPMI 1640 supplemented with 25 mM HEPES pH 7.2 and L-glutamine (Lonza), 100 IU/mL penicillin, 100 µg/mL streptomycin, and 50 µM β-mercaptoethanol (ThermoFisher Scientific, Waltham, Massachusetts, USA), supplemented with 10% autologous human serum. Plates were placed in a humidified 37°C incubator with 5% CO₂ and cultured overnight.

The next day, cells were harvested and pooled from the wells and separated by the magnetic beads-based MidiMACS system, using CD4 and CD8 MicroBeads and LS columns (Miltenyi Biotech), according to the manufacturer's protocol. The flow-through of the CD4MicroBeads experiments, i.e. the CD4-depleted cell fractions were further used for tracking CD8 TIL clnotypes in PD1 and INFgamma experiments. For separation of PD1+ clonotypes PD-1 Microbeads (Miltenyi Biotech, positive selection) were used. All cell fractions were cultured in complete medium (CM) at a density of 0.5-1x10⁶ cells/ml. CM consisted of RPMI 1640 supplemented with 25 mM HEPES pH 7.2 and L-glutamine (Lonza), 100 IU/mL penicillin, 100 µg/mL streptomycin, 2.5 mg/L amphotericin B (Sigma-Aldrich, St Louis, USA), and 50 µM β-mercaptoethanol (ThermoFisher Scientic), supplemented with 10% fetal calf serum (FCS), plus 3000 IU/mL of recombinant human IL-2 (Miltenyi Biotec) and Dynabeads Human T-Activator CD3/CD28 (Life Technologies) at a bead:T-cell ratio of 1 : 1. The plates were placed in a humidified 37°C incubator with 5% CO₂ and cultured until day 22. Every second or third day, half of the medium was removed and replaced with fresh medium supplemented with fresh IL-2. Whenever necessary, cells were split in doubled wells by the addition of fresh medium supplemented with IL-2 to maintain a cell density of ≈ 10⁶ cells/ml. Within the first week, the cell cultures were harvested for DNA extraction and NGS library preparation, residual TILs were further expanded. Between day 14 and 18 Dynabeads were removed, IL-2 concentration in the medium was reduced to 1500 IU/ml and 10% FCS was replaced by 6% autologous human serum.

### Interferon-gamma Secretion Assay - Cell Enrichment and Detection

For the tumour co-culture assay on day 22, the IL-2 was omitted from the medium. The co-culture was established with a 1 : 1 ratio of expanded TILs and autologous tumour cells (10⁵ TILs and 10⁵ autologous tumour cells per well). Tumour cells were derived from the initial tumour digest that was cryopreserved in 10% DMSO (Sigma-Aldrich) and 90% FCS (Life Technologies) and were washed in RPMI 1640 before addition. The co-culture was incubated in a humidified incubator for 20 h at 37°C before the cells were harvested and analysed for interferon gamma (IFNγ) production in an IFNγ Secretion Assay and Detection Kit (Miltenyi Biotec) according to the manufacturer's instructions. Beads bound cells were eluted and pelleted for genomic DNA isolation and NGS library preparation.

### V-beta Antibody-based Cell Enrichment

For isolation of T cells from blood 3 ml of freshly drawn blood were incubated with 5 volumes of erythrocyte lysis buffer (EL buffer, Qiagen) for 15 minutes at 4°C. Mononuclear cells were pelleted in a refrigerated centrifuge at 400g. Cells were washed several times with EL buffer and PBS and finally labeled with anti-Vbeta 30 antibody PE-conjugate (Beckman Coulter, Brea, California, USA). Cells were indirectly magnetically labeled with anti-PE MicroBeads (Miltenyi Biotec) and separated on MS columns using the MiniMACS magnetic separation system following the manufacturer's instructions (Miltenyi Biotec). Beads bound cells were eluted and pelleted for genomic DNA isolation and NGS library preparation.

### Genomic DNA Isolation

Genomic DNA (gDNA) was extracted from tissue materials using the NucleoSpin® Tissue Kit from Macherey-Nagel (Düren, Germany). Blood gDNA was isolated from 2-3 ml fresh blood with either QIAamp® DNA Blood Mini Kit (Qiagen, Hilden, Germany) or AllPrep® DNA/RNA/miRNA Universal Kit (Qiagen) following the manufacturer's protocols.

### Calculation of clonotype (sequence cluster) frequencies from NGS data

CDR3 regions of the TCRβ-chain were sequenced with NGS (Illumina MiSEQ) technology following a proprietary 2-step PCR amplification method (as disclosed in WO 2014/096394 A1) which is using TCRβ primers binding specifically to the V- and J-segments adjacent to the CDR3 region. Genomic DNA was used as starting material for the NGS process.

Per sample a large (>10⁵) number of paired reads (nucleotide sequences) is commonly produced by NGS. The read-pairs are overlapping by typically 40 to 80 bases and are merged read-pair by read-pair to contiguous sequences. These sequences are then assembled into clusters of virtually identical nucleotide sequences, the number of reads per cluster determines the frequency of that cluster, where frequency of a cluster is measured in percentage of reads of this sample falling into this cluster.

Clustering is very conservative and works in two rounds: In a first step all reads with 100% nucleotide sequence identity are counted as 1 cluster with the cluster sequence being identical to the read sequence. In the second step clusters are compared among each other and those with
- not more than 1 bp mismatch and
- where one cluster (cluster A) has at least 20x more reads than the other cluster (cluster B)
are merged and regarded as identical to cluster A. The nucleotide sequence clusters are regarded as equivalent to clonotypes.

The nucleotide sequence clusters are translated to amino acid sequences (peptides) and tabulated. Each cluster is regarded as one clonotype with a frequency as defined above. The frequency is a direct measure of the frequency of the respective T cell in the sample.

### Comparison of TCR sequence profiles between samples

CDR3 amino acid sequences of clonotypes were compared between samples by an identity test procedure, where only sequences without mismatches are accepted as one and the same CDR3 amino acid sequence. The result of a multi-sample comparison is a table with one TCRβ CDR3 amino acid sequence shared by one or more samples per row, each sample is represented by one column containing the respective CDR3 frequencies in that sample. Ratios between distinct samples (sharing the same CDR3 amino acid sequence) are calculated by ratio of the respective frequencies.

**Table 2:**

| **A** | **B** | **C** | **D** | **E** | **F** | **G** |
|---|---|---|---|---|---|---|
| **CDR3 peptide** | **Vsegm** | **Jsegm** | **BLOOD** | **TILS CD8⁺** | **non-TUMOR CD8⁺** | **TILs CD8⁺ non_TUMOR CD8⁺** |
| CASSVDRGAEAFF | V19*01/*02/*03 | J1-1*01 | 0,000 | 3,660 | 0,407 | 8,993 |
| CAWNKQVDGYTF | V30*01/**03/*05 | J1-2*01 | 0,026 | 2,394 | 0,055 | 43,527 |
| CASSFGVMNTEAFF | V5-5*01/*02/*03 | J1-1*01 | 0,000 | 2,330 | 1,461 | 1,595 |
| CASSPDGETQYF | V4-2*01/*02 | J2-5*01 | 0,000 | 2,256 | 0,201 | 11,224 |
| CASSLGQAYEQYF | V7-8*01/*02/*03 | J2-7*01 | 0,608 | 1,786 | 1,016 | 1,758 |
| CASSPVALGMNTEAFF | V7-3*01/*05 | J1-1*01 | 0,035 | 1,417 | 3,386 | 0,418 |
| CAISDWTGSNYGYTF | V10-3*01/*02/*09/*04 | J1-2*01 | 0,000 | 1,162 | 0,058 | 20,034 |
| CASSGRGDLLEQYF | V5-6*01 | J2-7*01 | 0,326 | 1,121 | 0,596 | 1,881 |
| CASSETGAAETQYF | V18*01 | J2-5*01 | 0,000 | 1,095 | 4,883 | 0,224 |
| CASSRLAGGTDTQYF | V7-3*01/*05 | J2-3*01 | 0,564 | 0,950 | 2,477 | 0,384 |
| CASSSGLVYEQYF | V19*01/*02/*03 | J2-7*01 | 0,000 | 0,898 | 0,128 | 7,016 |
| CASSTGTGGLGELFF | V28*01 | J2-2*01 | 0,000 | 0,875 | 0,102 | 8,578 |
| CASSEAPPLYYEQYF | V6-1*01/V6--5*01/-6*01/-6*02/-6*03/-6*04/-6*05 | J2-7*01 | 0,051 | 0,855 | 0,211 | 4,052 |
| CASSNDRAGLNEQFF | V6-1*01/V6--5*01/-6*01/-6*02/-6*03/-6*04/-6*05 | J2-1*01 | 0,352 | 0,846 | 0,739 | 1,145 |
| CATSDGRLEQFF | V24-1*01 | J2-1*01 | 0,127 | 0,822 | 0,113 | 7,274 |
| CASSLGYRYGTEAFF | V5-4*01/*02/*03/*04 | J1-1*01 | 0,752 | 0,810 | 3,512 | 0,231 |
| CASSQDNGGYGYTF | V4-1*01/*02 | J1-2*01 | 0,000 | 0,803 | 0,148 | 5,426 |
| CASSQGDSFYGYTF | Y4-1*01/*02 | J1-2*01 | 0,232 | 0,800 | 0,195 | 4,103 |
| CASSADLGDRVNGYTF | V5-1*01/*02 | J1-2*01 | 0,000 | 0,782 | 0,807 | 0,969 |
| CASSLDRGGYEQYF | V4-1*01/*02 | J2-7*01 | 0,000 | 0,754 | 0,140 | 5,386 |
| CARPPAGIPDTQYF | V28*01 | J2-3*01 | 0,000 | 0,731 | 0,000 | NN |
| CASSDQGHSNQPQHF | V4-1*01/*02 | J1-5*01 | 0,160 | 0,713 | 0,130 | 5,485 |
| CASSRPSFRVSEQFF | V4-1*01/*02 | J2-1*01 | 0,464 | 0,704 | 1,214 | 0,580 |
| CASSLLLAGASYEQYF | V5-5*01/*02/*03 | J2-7*01 | 0,343 | 0,665 | 0,392 | 1,696 |
| CASSSFQGGNEQFF | V28*01 | J2-1*01 | 0,874 | 0,614 | 3,060 | 0,201 |
| CASSLVRGNEQFF | V27*01 | J2-1*01 | 0,068 | 0,609 | 0,189 | 3,222 |
| CASSLERSERPYEQYF | V7-9*01-*07 | J2-7*01 | 0,801 | 0,596 | 4,943 | 0,121 |
| CASTPRGNTGELFF | V6-1*01/V6--5*01/-6*01/-6*02/-6*03/-6*04/-6*05 | J2-2*01 | 0,145 | 0,571 | 0,280 | 2,039 |
| CASSNPGRGTREQYF | V5-6*01 | J2-7*01 | 0,020 | 0,564 | 0,098 | 5,755 |
| CASSLRINYEQYF | V5-5*01/*02/*03 | J2-7*01 | 0,000 | 0,557 | 0,307 | 1,814 |
| CASSRPEATNEKLFF | V4-1*011*02 | J1-4*01 | 0,000 | 0,556 | 0,012 | 46,333 |
| CASSWGTDTEAFF | V27*01 | J1-1*01 | 0,041 | 0,472 | 0,098 | 4,816 |
| CAWAKGTEAFF | V30*01/**03/*05 | J1-1*01 | 0,000 | 0,471 | 0,015 | 31,400 |
| CASSQVTGITEAFF | V14*01/*02 | J1-1*01 | 0,302 | 0,457 | 0,668 | 0,684 |
| CASSPGGRPYEQYF | V5-4*01/*02/*03/*04 | J2-7*01 | 0,000 | 0,417 | 0,010 | 41,700 |
| CASSPGQGEGYEQYF | V4-1*01/*02 | J2-7*01 | 0,070 | 0,387 | 0,104 | 3,721 |
| CASSQVGSSVAGGRSEA | V4-1*01/*02 | J1-1*01 | 0,000 | 0,351 | 0,288 | 1,219 |
| CASSSTGTGGSSWNEQF | V6-1*01/V6-5*01/-6*01/-6*02/-6*03/-6*04/-6*05 | J2-1*01 | 0,000 | 0,350 | 0,018 | 19,444 |
| CATGTGSYEQYF | V19*01/*02/*03 | J2-7*01 | 0,000 | 0,284 | 0,000 | NN |
| CASSLWEASYGYTF | V5-6*01 | J1-2*01 | 0,012 | 0,282 | 0,174 | 1,621 |
| CASSQTGTGSYEQYF | V4-1*01/*02 | J2-7*01 | 0,000 | 0,280 | 0,130 | 2,154 |
| CASSIAQGVYEQYF | V27*01 | J2-7*01 | 0,000 | 0,278 | 0,866 | 0,321 |
| CASSQRRLNTEAFF | V16*01/**02/*03 | J1-1*01 | 0,000 | 0,273 | 0,000 | NN |
| CASSLGTAKETQYF | V7-9*01-*07 | J2-5*01 | 0,214 | 0,262 | 1,014 | 0,258 |
| CASSFEAPAYEQYF | V5-8*01/*02 | J2-7*01 | 0.000 | 0,252 | 0,258 | 0,977 |
| CASSLAGGLVEQYF | V19*01/*02/*03 | J2-7*01 | 0,267 | 0,249 | 0,188 | 1,324 |
| CATTQAGTENTEAFF | V19*01/*02/*03 | J1-1*01 | 0,000 | 0,246 | 0,055 | 4,473 |
| CASSPGQGEGYEQYF | V4-1*01/*02 | J2-7*01 | 0,030 | 0,242 | 0,022 | 11,000 |
| CASSQEGEGETQYF | V4-1*01/*02 | J2-5*01 | 0,026 | 0,237 | 0,019 | 12,474 |
| CASSVGPGLNMQVTDTQ | V7-6*01/*02 | J2-3*01 | 0,000 | 0,236 | 0,027 | 8,741 |
| CASSYRDSSSYEQYF | V9*01/*02/*03 | J2-7*01 | 0,000 | 0,229 | 0,000 | NN |
| CASSYLAEPPGNEQFF | V6-2*01/**02/**03/-3*01 | J2-1*01 | 0,078 | 0,229 | 0,199 | 1,151 |
| CASSSYSETANYGYTF | V5-1*03/*02 | J1-2*01 | 0,014 | 0,223 | 0,097 | 2,299 |
| CASSQERSTGELFF | V4-2*01/*02 | J2-2*01 | 0,000 | 0,223 | 0,132 | 1,689 |
| CASSYWGGTNTEAFF | V6-1*01/V6-5*01/-6*01/-6*02/-6*03/-6*04/-6*05 | J1-1*01 | 0,000 | 0,219 | 0,189 | 1,159 |
| CASSIDRGSEAFF | V19*01/*02/*03 | J1-1*01 | 0,000 | 0,217 | 0,144 | 1,507 |
| CASSQVLSGGFYEQYF | V4-1*01/*02 | J2-7*01 | 0,000 | 0,216 | 0,154 | 1,403 |
| CAWSKEYGYTF | V30*01/**03/*05 | J1-2*01 | 0,000 | 0,213 | 0,000 | NN |
| CAWTWGGGNEQYF | V30*01/**03/*05 | J2-7*01 | 0,194 | 0,213 | 0,084 | 2,536 |
| CATSDLHRTPDLNTEAF | V24-1*01 | J1-1*01 | 0,038 | 0,207 | 0,111 | 1,865 |
| CASSSQGDGTDTQYF | V7-9*01-*07 | J2-3*01 | 0,000 | 0,202 | 0,135 | 1,496 |
| CASSPGPNYEQYF | V7-6*01/*02 | J2-7*01 | 0,021 | 0,201 | 0,012 | 16,750 |
| CASSLEEYGYTF | V7-2*01/*02/*03/*04 | J1-2*01 | 0,605 | 0,199 | 1,816 | 0,110 |
| CASSQDRSVAYEQYF | V4-3*01/*02/*03/*04 | J2-7*01 | 0,000 | 0,198 | 0,000 | NN |
| CASSLRGKTSTYEQYF | V7-8*01/*02/*03 | J2-7*01 | 0,017 | 0,191 | 0,194 | 0,985 |
| CASSLSSKNEQFF | V27*01 | J2-1*01 | 0,000 | 0,188 | 0,085 | 2,212 |
| CAVNQAGWGGTQYF | V27*01 | J2-3*01 | 0,108 | 0,180 | 0,060 | 3,000 |
| CAWSFPGASGG*ETQYF | V30*01/**03/*05 | J2-5*01 | 0,000 | 0,180 | 0,132 | 1,364 |
| CASSQRAAPYGYTF | V4-1*01/*02 | J1-2*01 | 0,000 | 0,177 | 0,039 | 4,538 |
| CASSSGHGYNEQFF | V3-1*01/*02 | J2-1*01 | 0,000 | 0,169 | 0,129 | 1,310 |
| CASSLLLSGGAADTQYF | V27*01 | J2-3*01 | 0,011 | 0,166 | 0,560 | 0,296 |
| CASSRGPNYEQYF | V7-6*01/*02 | J2-7*01 | 0,043 | 0,158 | 0,172 | 0,919 |
| CASSIDSNNEQFF | V19*01/*02/*03 | J2-1*01 | 0,077 | 0,155 | 0,163 | 0,951 |
| CATSDLIDFDRVDGYTF | V24-1*01 | J1-2*01 | 0,000 | 0,153 | 0,000 | NN |
| CASSPLTGMQFF | V7-6*01/*02 | J2-1*01 | 0,000 | 0,147 | 0,098 | 1,500 |
| CASIWRLGMNTEAFF | V19*01/*02/*03 | J1-1*01 | 0,000 | 0,145 | 0,260 | 0,558 |
| CASSSTVAGEQYF | V27*01 | J2-7*01 | 0,444 | 0,145 | 1,494 | 0,097 |
| CASSPRTGNTGELFF | V4-2*01/*02 | J2-2*01 | 0,065 | 0,143 | 0,164 | 0,872 |
| CASTRSVGAGTEAFF | V27*01 | J1-1*01 | 0,000 | 0,140 | 0,085 | 1,647 |
| CASSPGTDGGSLGSPLH | V27*01 | J1-6*01 | 0,000 | 0,137 | 0,015 | 9,133 |
| CASSWDSSYEQYF | V6-2*01/**02/**03/-3*01 | J2-7*01 | 0,000 | 0,134 | 0,029 | 4,621 |
| CASSPLGGEKLFF | V6-1*01/V6--5*01/-6*01/-6*02/-6*03/-6*04/-6*05 | J1-4*01 | 0,000 | 0,134 | 0,271 | 0,494 |
| CASSQAGIHGYTF | V14*01/*02 | J1-2*01 | 0,000 | 0,130 | 0,079 | 1,646 |
| CASSIAGGPGETQYF | V19*01/*02/*03 | J2-5*01 | 0,000 | 0,126 | 0,088 | 1,432 |
| CASSQVPDRDGYTF | V4-3*01/*02/*03/*04 | J1-2*01 | 0,000 | 0,123 | 0,194 | 0,634 |
| CASSQGAALGYEQYF | V4-1*01/*02 | J2-7*01 | 0,000 | 0,122 | 0,000 | NN |
| CASSEYLEVQETQYF | V25-1*01 | J2-5*01 | 0,027 | 0,120 | 0,090 | 1,333 |
| CASSLEANNEQFF | V5-6*01 | J2-1*01 | 0,000 | 0,120 | 0,101 | 1,188 |
| CAISESKDRPSSYNEQF | V10-3*01/*02/*03/*04 | J2-1*01 | 0,000 | 0,119 | 0,129 | 0,922 |
| CASSPGAGLYEQYF | V5-4*01/*02/*03/*04 | J2-7*01 | 0,000 | 0,119 | 0,149 | 0,799 |
| CASSQKWGNIQYF | V14*01/*02 | J2-4*01 | 0,017 | 0,118 | 0,046 | 2,565 |
| CATGLAGGQEQYF | V24-1*01 | J2-7*01 | 0,099 | 0,118 | 0,070 | 1,686 |
| CASSLTDYGYTF | V7-2*01/*02/*03/*04 | J1-2*01 | 0,306 | 0,118 | 1,023 | 0,115 |
| CASSLTDYGYTF | V7-2*01/*02/*03/*04 | J1-2*01 | 0,083 | 0,117 | 0,176 | 0,665 |
| CASTPGSYRETQYF | V5-1*01/*02 | J2-5*01 | 0,055 | 0,116 | 0,491 | 0,236 |
| CASGTDFPSYEQYF | V19*01/*02/*03 | J2-7*01 | 0,000 | 0,115 | 0,017 | 6,765 |
| CAIPSSSGANVLTF | V10-3*01/*02/*03/*04 | J2-6*01 | 0,000 | 0,112 | 0,000 | NN |
| CASSLVGGPHEQYF | V7-9*01-*07 | J2-7*01 | 0,000 | 0,111 | 0,000 | NN |
| CASSSAGTGHNEQFF | V6-1*01/V6--5*01/-6*01/-6*02/-6*03/-6*04/-6*05 | J2-1*01 | 0,000 | 0,111 | 0,054 | 2,056 |
| CASSQKDRYGYTF | V4-2*01/*02 | J1-2*01 | 0,000 | 0,109 | 0,010 | 10,900 |

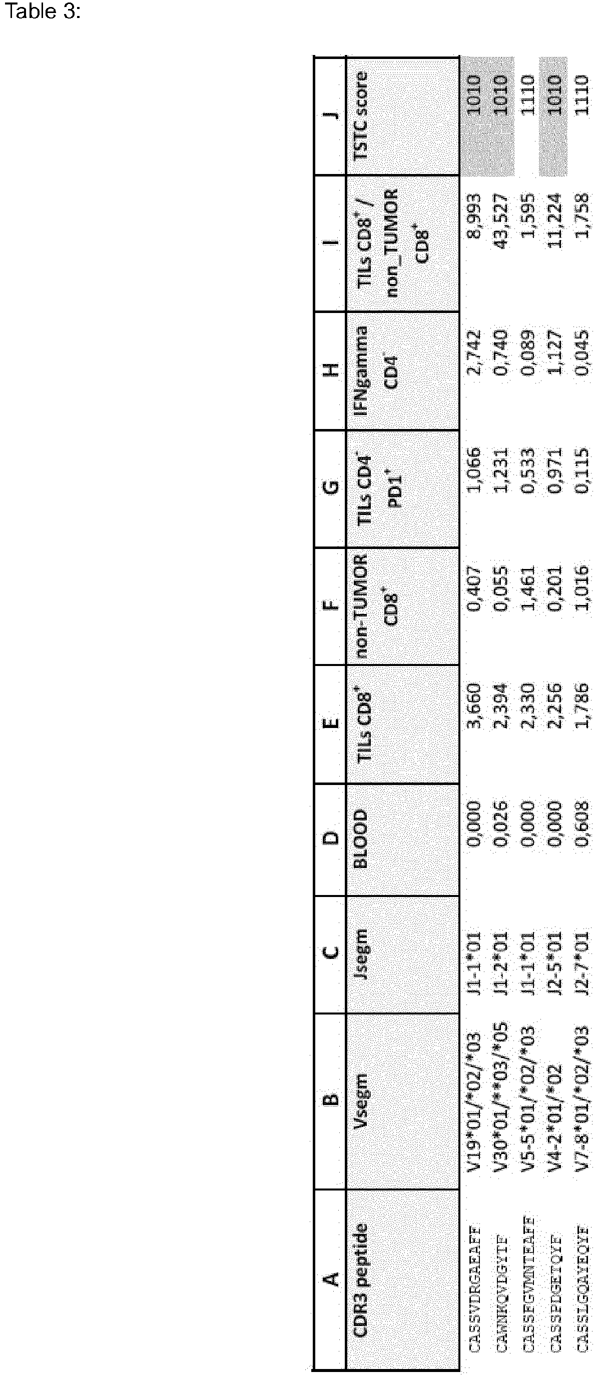

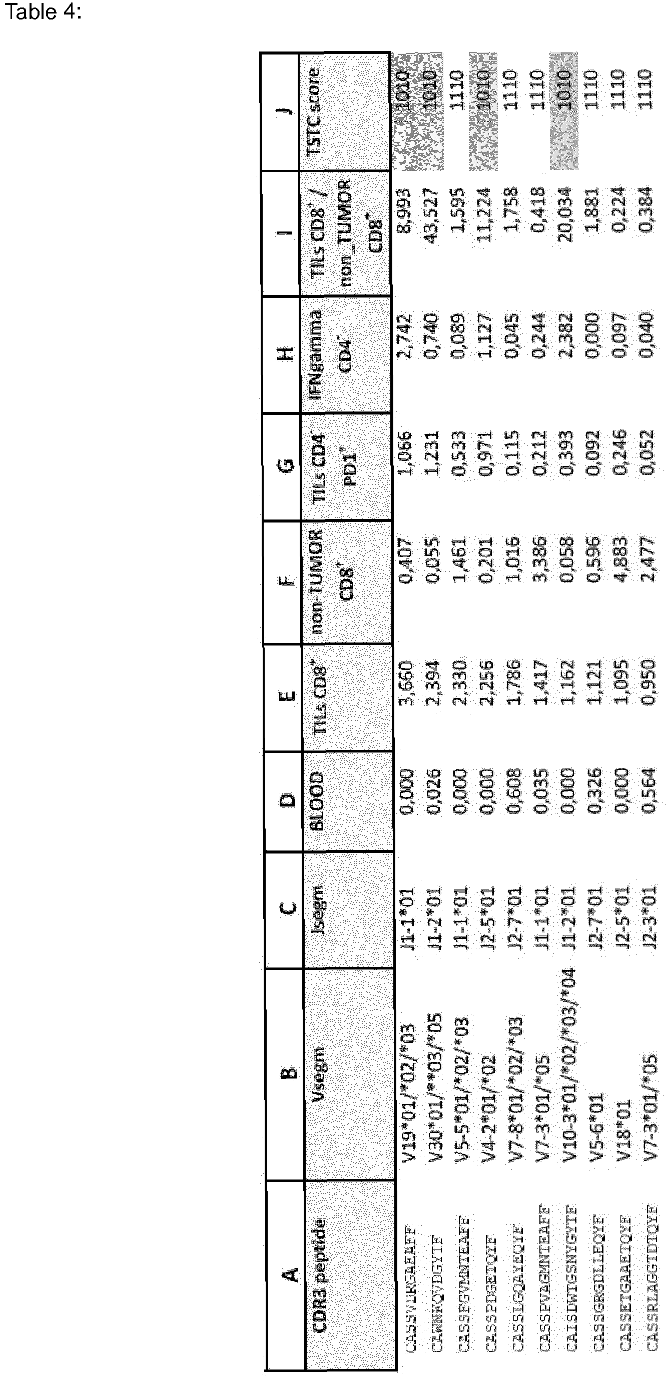

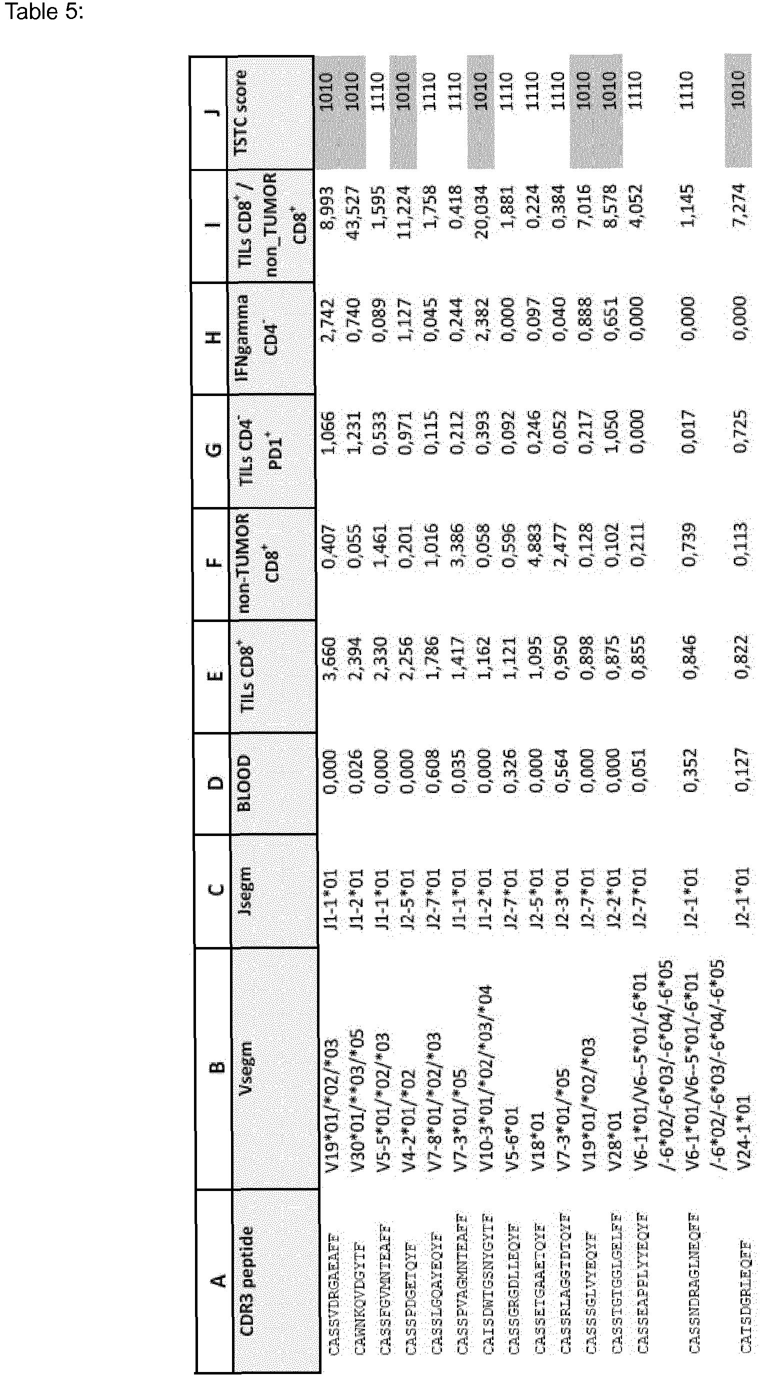

**Table 6:**

| | **median IFNgamma** | **tumor reactive clones** | **non-reactive clones** | **percentage tumor reactive clones** |
|---|---|---|---|---|
| | **top 5 TILs** | | | |
| a. no non-tumor tissue used | 0,74 | 3 | 2 | 60% |
| b. ratio tumor/non-tumor > 20 | 0,74 | 1 | 0 | 100% |
| c. ratio tumor/non-tumor > 5 | 1,13 | 3 | 0 | 100% |

| | **top 10 TILs** | | | |
|---|---|---|---|---|
| a. no non-tumor tissue used | 0,17 | 4 | 6 | 40% |
| b. ratio tumor/non-tumor > 20 | 1,56 | 2 | 0 | 100% |
| c. ratio tumor/non-tumor > 5 | 1,76 | 4 | 0 | 100% |

| | **top 15 TILs** | | | |
|---|---|---|---|---|
| a. no non-tumor tissue used | 0,10 | 6 | 9 | 40% |
| b. ratio tumor/non-tumor > 20 | 1,56 | 2 | 0 | 100% |
| c. ratio tumor/non-tumor > 5 | 0,89 | 7 | 1 | 88% |

**Table 7:**

| **CDR3 peptide** | **Vsegm** | **Jsegm** | **TILs CD8+** | **IFNgamma CD4⁻ PD1⁺** | **TILs CD4⁻ PD1⁺** | **TILS**_**CD8**/ **non**_**TUMOR** | **Vbeta**-**AB** | **Freq. In Vbeta AB** | **Freq. In PBMC** | **enrichment factor** |
|---|---|---|---|---|---|---|---|---|---|---|
| CAWNKQVDGYTF | V30*01/**03/*05 | J1-2*01 | 2,394 | 0,740 | 1,231 | 43,527 | V30-AB | 5,525 | 0,097 | 57,019 |
| CAWAKGTEAFF | V30*01/**03/*05 | J1-1*01 | 0,471 | 0,703 | 0,254 | 31,400 | V30-AB | 1,294 | no value | > 1000 |

## Claims

1. A method for providing a tumour specific T cell preparation, comprising the steps of:
a. selecting tumour-specific T cell clones by:
- providing a tumour sample obtained from a patient;
- isolating a nucleic acid preparation from said tumour sample in a nucleic acid isolation step;
- obtaining a plurality of T cell receptor nucleic acid sequences from said nucleic acid preparation or a plurality of T cell receptor amino acid sequences encoded by said plurality of T cell receptor nucleic acid sequences;
- selecting a tumour-specific T cell receptor nucleic acid sequence from said plurality of T cell receptor nucleic acid sequences or a tumour-specific T cell receptor amino acid sequence from said plurality of T cell receptor amino acid sequences in a sequence selection step;
b. sorting tumour-specific T cell clones by:
- providing a lymphocyte preparation obtained from said patient;
- isolating cells that comprise said selected tumour-specific T cell receptor nucleic acid sequence or said selected tumour-specific T cell receptor amino acid sequence from said lymphocyte preparation in an isolation step.

2. The method according to claim 1, wherein said isolation step comprises the steps of:
- contacting said lymphocyte preparation with a specifically reactive ligand being able to bind an amino acid sequence comprised within the V region of the T cell receptor that corresponds to said selected tumour-specific T cell receptor nucleic acid sequence or to said selected T cell receptor amino acid sequence, wherein said ligand is attached to a detectable label, and wherein particularly said ligand binds to said amino acid sequence with a dissociation constant of 10⁻⁷, 10⁻⁸ or 10⁻⁹ mol/l or less, and
- isolating T cells carrying said detectable label from said lymphocytes preparation.

3. The method according to claim 1, wherein said isolation step comprises the steps of;
- contacting said lymphocyte preparation with a nucleic acid probe specifically binding to said selected tumour-specific T cell receptor nucleic acid sequence, wherein said nucleic acid probe is attached to a detectable label;
- isolating T cells carrying said detectable label from said lymphocyte preparation, or
said isolation step comprises:
- a separating step, wherein said lymphocyte preparation is separated into a plurality of fractions,
- an expanding step, wherein cells comprised within said plurality of fractions are expanded under conditions of cell culture, and
- a selecting step, wherein at least one fraction of said plurality of fraction that comprises said selected tumour-specific T cell receptor nucleic acid sequence or said selected tumour-specific T cell receptor amino acid sequence is selected, and said isolation step optionally further comprises repeating said separating step, said expanding step and said selecting step with said selected at least one fraction of said plurality.

4. The method according to claim 3, wherein said selecting step comprises,
- contacting said plurality of fractions with a nucleic acid probe specifically binding to said selected tumour-specific T cell receptor nucleic acid sequence, wherein said nucleic acid probe is attached to a detectable label and identifying fractions comprising said selected tumour-specific T cell receptor sequences,or
- obtaining T cell receptor nucleic acid sequences from said plurality of fraction and identifying fraction comprising said selected tumour-specific T cell receptor nucleic acid sequence.

5. The method according to any one of claims 1 to 4, wherein said sequence selection step comprises the steps of
- aligning said plurality of T cell receptor nucleic acid sequences or said plurality of T cell receptor amino acid sequences;
- grouping T cell receptor nucleic acid sequences comprised in said plurality of T cell receptor nucleic acid sequences or T cell receptor amino acid sequences comprised in said plurality of T cell receptor amino acid sequence into a plurality of tumour sample clonotypes, wherein nucleic acid sequences or amino acid sequence comprised within a particular clonotype exhibit a virtually identical or an identical sequence;
- determining the number of T cell receptor nucleic acid sequences associated with each clonotype or determining the number of T cell receptor amino acid sequences associated with each clonotype, thereby yielding a clonotype frequency for each of said clonotypes;
- selecting a tumour-specific clonotype from said plurality of tumour sample clonotypes, wherein said tumour-specific clonotype is one of the 100 most frequent clonotypes of said plurality of tumour sample clonotypes and/or is another clonotype of said plurality of tumour sample clonotypes that comprises a T cell receptor amino acid sequence being identical or virtually identical to a T cell receptor amino acid encoded by a T cell receptor nucleic acid sequence of said plurality of T cell receptor nucleic sequences comprised within said one tumour-specific clonotype of the 100 most frequent clonotypes of said plurality of tumour sample clonotypes, and
- selecting a T cell receptor nucleic acid sequence of said plurality of T cell receptor nucleic acid sequences comprised within said selected tumour-specific clonotype as said tumour-specific receptor nucleic acid sequence or selecting a T cell receptor amino acid sequenced of said plurality of said T cell receptor amino acid sequences comprised within said selected tumour-specific clonotype as said tumour-specific amino acid sequence.

6. The method according to claim 5, further comprising
- providing a non-tumour sample obtained from said patient;
- isolating a nucleic acid preparation from said non-tumour sample in a nucleic acid isolation step;
- obtaining a plurality of T cell receptor nucleic acid sequences from said nucleic acid preparation or a plurality of T cell receptor amino acid sequences encoded by said plurality of T cell receptor nucleic acid sequences, yielding a plurality of non-tumour-specific T cell receptor nucleic acid sequences or a plurality of non-tumour-specific T cell receptor amino acid sequences;
- aligning said plurality of non-tumour-specific T cell receptor nucleic acid sequences or said plurality of non-tumour-specific T cell receptor amino acid sequences;
- grouping T cell receptor nucleic acid sequences comprised in said plurality of non-tumour-specific T cell receptor nucleic acid sequences or said plurality of non-tumour-specific T cell receptor amino acid sequences into a plurality of non-tumour-specific clonotypes, wherein T cell receptor nucleic acid sequences or T cell receptor amino acid sequences comprised within a particular clonotype exhibit a virtually identical or an identical sequence;
- selecting a tumour specific clonotype from said plurality of tumour sample clonotypes, wherein
• said tumour specific clonotype is one of the 100 most frequent clonotypes of said plurality of tumour sample or is another clonotype of said plurality of tumour sample clonotypes that comprises a T cell amino acid sequence being identical or virtually identical to a T cell receptor amino acid encoded by a T cell receptor nucleic acid sequence of said plurality of T cell receptor nucleic acid sequences comprised within said one tumour-specific clonotype of the 100 most frequent clonotypes of said plurality of tumour sample clonotypes, and
• said tumour-specific clonotype of the 100 most frequent clonotypes of said plurality of tumour sample clonotypes is absent in said non-tumour sample or can be assigned to a non-tumour-specific clonotype that exhibits a frequency of not more than 20 %, 15 %, 10 % or 5 % of the frequency of said tumour-specific clonotype.

7. The method according to claim 5 or 6, further comprising:
- providing a blood sample obtained from said patient;
- isolating a nucleic acid preparation from said blood sample in a nucleic acid isolation step;
- obtaining a plurality of T cell receptor nucleic acid sequences from said nucleic acid preparation or a plurality of T cell receptor amino acid sequences encoded by said plurality of T cell receptor nucleic acid sequences;
- aligning said plurality of T cell receptor nucleic acid sequences or said plurality of T cell receptor amino acid sequences;
- grouping T cell receptor nucleic acid sequences comprised in said plurality of T cell receptor nucleic acid sequences or T cell receptor amino acids sequences into a plurality of blood sample clonotypes, wherein T cell receptor nucleic acid sequences or T cell receptor amino acids sequences comprised within a particular clonotype exhibit a virtually identical or an identical sequence;
- selecting a tumour specific clonotype from said plurality of tumour sample clonotypes, wherein
• said tumour specific clonotype is one of the 100 most frequent clonotypes of said plurality of tumour sample or is another clonotype of said plurality of tumour sample clonotypes that comprises a T cell amino acid sequence being identical or virtually identical to a T cell receptor amino acid encoded by a T cell receptor nucleic acid sequence of said plurality of T cell receptor nucleic acid sequences comprised within said one tumour-specific clonotype of the 100 most frequent clonotypes of said plurality of tumour sample clonotypes, and
• said tumour-specific clonotype of the 100 most frequent clonotypes of said plurality of tumour sample can be assigned to a blood sample clonotype that shows a frequency of less than the frequency of said tumour-specific clonotype.

8. The method according to any one of claims 5 to 7, further comprising:
- providing a cell-free sample obtained from said patient;
- isolating a nucleic acid preparation from said cell-free sample in a nucleic acid isolation step;
- obtaining a plurality of T cell receptor nucleic acid sequences from said nucleic acid preparation or a plurality of T cell receptor amino acid sequences encoded by said plurality of T cell receptor nucleic acid sequences;
- aligning said plurality of T cell receptor nucleic acid sequences or said plurality of T cell receptor amino acid sequences;
- grouping T cell receptor nucleic acid sequences comprised in said plurality of T cell receptor nucleic acid sequences or T cell receptor amino acid sequences comprises in said plurality of T cell amino acid sequences into a plurality of cell-free sample clonotypes, wherein T cell receptor nucleic acid sequences or T cell receptor amino acid sequences comprised within a particular clonotype exhibit a virtually identical or an identical sequence;
- selecting a tumour specific clonotype from said plurality of tumour sample clonotypes, wherein
• said tumour specific clonotype is one of the 100 most frequent clonotypes of said plurality of tumour sample clonotypes or is another clonotype of said plurality of tumour sample clonotypes that comprises a T cell amino acid sequence being identical or virtually identical to a T cell receptor amino acid sequence encoded by a T cell receptor nucleic acid sequence of said plurality of T cell receptor nucleic acid sequences comprised within said one tumour-specific clonotype of the 100 most frequent clonotypes of said plurality of tumour sample clonotypes, and
• said tumour-specific clonotype of the 100 most frequent clonotypes of said plurality of tumour sample can be assigned to a cell-free sample clonotype.

9. The method according to any one of claims 5 to 8, wherein said tumour-specific clonotype of the 100 most frequent clonotypes of said plurality of tumour sample clonotypes can be assigned to another clonotype of said plurality of tumour sample clonotypes that comprises a T cell amino acid sequence being identical or virtually identical to a T cell receptor amino acid encoded by a T cell receptor nucleic acid sequence of said plurality of T cell receptor nucleic acid sequences comprised within said one tumour-specific clonotype of the 100 most frequent tumour-specific clonotypes or to a T cell receptor amino acid sequence of said plurality of T cell receptor amino acid sequences comprised within said one tumour-specific clonotype of the 100 most frequent tumour-specific clonotypes.

10. The method according to any one of claims 5 to 9, wherein the most frequent clonotype of said tumour sample clonotypes or another clonotype of said plurality of tumour sample clonotypes that comprises a T cell amino acid sequence being virtually identical or identical to a T cell receptor amino acid encoded by a T cell receptor sequence of said plurality of T cell receptor nucleic acid sequences comprised within said most frequent tumour-specific clonotype is selected, wherein particularly said most frequent clonotype is absent in said non-tumour sample or can be assigned to a non-tumour clonotype that shows a frequency of not more than 20 %, 15 %, 10 % or 5 % of the frequency of said tumour-specific clonotype, and/or can be assigned to a blood sample clonotype that shows a frequency less than the frequency of the respective said tumour sample clonotypes, and/or can be assigned to a cell-free clonotype and/or can be assigned to another clonotype of said plurality of tumour sample clonotypes that comprises a T cell amino acid sequence being identical or virtually identical to a T cell receptor amino acid encoded by a T cell receptor nucleic acid sequence of said plurality of T cell receptor nucleic acid sequences comprised within said most frequent tumour-specific clonotype.

11. The method according to any one of claims 5 to 10, comprising
- selecting 5, 10, 15 or 20 tumour-specific clonotypes from said tumour sample, wherein
• said selected tumour-specific clonotypes are 5, 10, 15 or 20 of the 100 most frequent clonotypes, the 5 most frequent clonotypes, the 10 most frequent clonotypes, the 15 most frequent clonotypes, or the 20 most frequent clonotypes of said plurality of tumour sample clonotypes and/or are another clonotypes of said plurality of tumour sample clonotypes that comprise a T cell amino acid sequence being virtually identical or identical to a T cell receptor amino acid encoded by a T cell receptor nucleic acid sequence of said plurality of T cell receptor nucleic acid sequences comprised within any one of said selected 5, 10, 15 or 20 tumour-specific clonotypes of said plurality of tumour sample clonotypes, and optionally
• said selected 5, 10, 15 or 20 tumour-specific clonotypes are absent in said non-tumour sample or can be assigned to a non-tumour-specific clonotype that exhibits a frequency of not more than 20 % 15 %, 10 % or 5 % of the frequency of said selected 5, 10, 15 or 20 tumour-specific clonotypes of said plurality of tumour sample clonotypes, and/or
• said selected 5, 10, 15 or 20 tumour-specific clonotypes can be assigned to a blood sample clonotype that shows a frequency of less than the frequency of said selected 5, 10, 15 or 20 tumour-specific clonotypes of said plurality of tumour sample clonotypes, and/or
• said selected 5, 10, 15 or 20 tumour-specific clonotypes can be assigned to a cell-free sample clonotype, and/or
• said selected 5, 10, 15 or 20 tumour-specific clonotypes can be assigned to another clonotype of said plurality of tumour sample clonotypes that comprises a T cell amino acid sequence being virtually identical or identical to a T cell receptor amino acid encoded by any one of said T cell receptor nucleic acid sequences of said plurality of T cell receptor sequences comprised within said selected 5, 10, 15 or 10 tumour-specific clonotypes of said plurality of tumour sample clonotypes.

12. The method according to any one of claims 5 to 11, wherein
- any one of said one of the 100 most frequent clonotypes, said selected 5, 10, 15 or 20 tumour-specific clonotypes of said plurality of tumour sample clonotypes is assigned to a non-tumour-specific clonotype, if a T cell receptor amino acid sequence encoded by a T cell nucleic acid receptor sequence of said plurality of T cell receptor nucleic acid sequences comprised within said tumour-specific clonotype or a T cell amino acid sequence of said plurality of amino acid sequences comprised with said tumour-specific clonotype is identical to a T cell receptor amino acid sequence encoded by a T cell receptor nucleic acid sequence comprised within said non-tumour sample clonotype, or if a T cell amino acid sequence of said plurality of T cell receptor amino acid sequences comprised with said tumour-specific clonotype is identical to a T cell receptor amino acid sequence comprised within said non-tumour sample clonotype, and/or,
- any one of said one of the 100 most frequent clonotypes, said selected 5, 10, 15 or 20 tumour-specific clonotypes of said plurality of tumour sample clonotypes is assigned to a blood sample clonotype, if a T cell receptor amino acid sequence encoded by a T cell receptor sequence comprised within said tumour-specific clonotype is identical to a T cell receptor amino acid sequence encoded by a T cell receptor nucleic acid sequence comprised within said blood sample clonotype, or if a T cell amino acid sequence comprised with said tumour-specific clonotype is identical to a T cell receptor amino acid sequence comprised within said blood sample clonotype, and/or,
- any one of said one of the 100 most frequent clonotypes, said selected 5, 10 or 20 tumour-specific clonotypes of said plurality of tumour sample clonotypes is assigned to a cell-free sample clonotype, if a T cell receptor amino acid sequence encoded by a T cell receptor nucleic acid sequence of said plurality of T cell receptor nucleic acid sequences comprised within said tumour-specific clonotype is identical to a T cell receptor amino acid sequence encoded by a T cell receptor nucleic acid sequence comprised with said cell-free sample clonotype, or if a T cell amino acid sequence of said plurality of T cell amino acid sequences comprised with said tumour-specific clonotype is identical to a T cell receptor amino acid sequence comprised with said cell-free sample clonotype.

13. The method according to any one of the previous claims, wherein said nucleic acid isolation step comprises the steps of
a. isolating T cells from said tumour sample and isolating nucleic acid from said isolated T cells, and/or
b. conducting a nucleic acid amplification reaction that specifically amplifies T cell receptor nucleic acid sequences.

14. The method according to any one of the preceding claims, wherein said tumour-specific T cell receptor nucleic acid sequence encodes the CDR3 region of a chain of the human T cell receptor or said tumour-specific T cell receptor amino acid sequence is or comprised within the CDR3 region of a chain of the human T cell receptor.

## Patentansprüche

1. Ein Verfahren zur Bereitstellung einer tumorspezifischen T-Zellpräparation, umfassend die Schritte:
a. Selektieren von tumorspezifischen T-Zellklonen durch:
- Bereitstellen einer Tumorprobe erhalten von einem Patienten;
- Isolieren einer Nukleinsäurepräparation von der besagten Tumorprobe in einem Nukleinsäureisolationsschritt;
- Erhalten einer Vielzahl von T-Zellrezeptor-Nukleinsäuresequenzen von der besagten Nukleinsäurepräparation oder einer Vielzahl von T-Zellrezeptor-Aminosäuresequenzen kodiert durch die besagte Vielzahl von T-Zellrezeptor-Nukleinsäuresequenzen;
- Selektieren einer tumorspezifischen T-Zellrezeptor-Nukleinsäuresequenz von der besagten Vielzahl von T-Zellrezeptor-Nukleinsäuresequenzen oder einer tumorspezifischen T-Zellrezeptor-Aminosäuresequenz von der besagten Vielzahl von T-Zellrezeptor-Aminosäuresequenzen;
b. Sortieren von tumorspezifischen T-Zellklonen durch;
- Bereitstellen einer Lymphozytenpräparation erhalten vom besagten Patienten;
- Isolieren von Zellen, welche die besagte tumorspezifischen T-Zellrezeptor-Nukleinsäuresequenz oder die besagte tumorspezifischen T-Zellrezeptor-Aminosäuresequenz umfasst, aus der besagten Lymphozytenpräparation in einem Isolationsschritt.

2. Das Verfahren nach Anspruch 1, wobei der besagte Isolationsschritt die Schritte umfasst:
- In Kontakt bringen der besagten Lymphozytenpräparation mit einem spezifisch reaktiven Liganden, welcher zur Bindung an eine Aminosäuresequenz innerhalb der V-Region des T-Zellrezeptors fähig ist, wobei die V-Region mit der besagten selektierten tumorspezifischen T-Zellrezeptor-Aminosäuresequenz entspricht, und wobei der besagte Ligand mit einem detektierbaren Marker verbunden ist, und wobei insbesondere der besagte Ligand an die besagte Aminosäuresequenz mit einer Dissoziationskontante von 10⁻⁷, 10⁻⁸ oder 10⁻⁹ mol/l oder weniger bindet; und
- Isolieren von T-Zellen, welche den besagten Marker tragen, von der besagten Lymphozytenpräparation.

3. Das Verfahren nach Anspruch 1, wobei der besagte Isolationsschritt die Schritte umfasst:
- In Kontakt bringen der besagten Lymphozytenpräparation mit einer Nukleinsäuresonde, welche spezifisch an die besagte tumorspezifische T-Zellrezeptor-Nukleinsäuresequenz bindet, wobei die besagte Nukleinsäuresonde mit einem detektierbaren Marker verbunden ist;
- Isolieren von T-Zellen, welche den besagten Marker tragen, von der besagten Lymphozytenpräparation, oder
der besagte Isolationsschritt umfasst:
- einen Separierungsschritt, wobei die besagte Lymphozytenpräparation in eine Vielzahl von Fraktionen separiert wird,
- einen Expansionsschritt, wobei Zellen, welche von der Vielzahl von Fraktionen umfasst sind, unter Zellkulturbedingungen expandiert werden, und
- einen Selektierungsschritt, wobei mindestens eine Fraktion der besagten Vielzahl von Fraktionen, welche die besagte selektierte tumorspezifische T-Zellrezeptor-Nukleinsäuresequenz oder die besagte selektierte tumorspezifische T-Zellrezeptor-Aminosäuresequenz umfasst, selektiert wird, und der besagte Isolationsschritt optional ein Wiederholen des besagten Separierungsschritts, des besagten Expansionsschritts und des besagten Selektierungsschritts mit der besagten mindestens einen Fraktion der besagten Vielzahl umfasst.

4. Das Verfahren nach Anspruch 3, wobei der besagte Selektionsschritt umfasst:
- In Kontakt bringen der besagten Vielzahl von Fraktionen mit einer Nukleinsäuresonde, welche spezifisch an die besagte tumorspezifische T-Zellrezeptor-Nukleinsäuresequenz bindet, wobei die besagte Nukleinsäuresonde mit einem detektierbaren Marker verbunden ist, und Identifizieren von Fraktionen, welche die besagte selektierte tumorspezifische T-Zellrezeptor-Sequenz umfassen; oder
- Erhalten von T-Zellrezeptor-Nukleinsäuresequenzen von der besagten Vielzahl von Fraktionen und Identifizieren von Fraktionen, welche die besagte selektierte tumorspezifische T-Zellrezeptor-Nukleinsäuresequenz umfassen.

5. Das Verfahren nach einem der Ansprüche 1 bis 4, wobei der besagte Selektionsschritt die Schritte umfasst:
- Alignen der besagten Vielzahl von T-Zellrezeptor-Nukleinsäuresequenzen oder der besagten Vielzahl von T-Zellrezeptor-Aminosäuresequenzen;
- Gruppieren der T-Zellrezeptor-Nukleinsäuresequenzen, welche von der besagten Vielzahl von T-Zellrezeptor-Nukleinsäuresequenzen umfasst werden, oder der T-Zellrezeptor-Aminosäuresequenzen, welche von der besagten Vielzahl von T-Zellrezeptor-Aminosäuresequenzen umfasst werden, in eine Vielzahl von Klonotypen, wobei Nukleinsäuresequenzen oder Aminosäuresequenzen, welche von einem bestimmten Klonotypen umfasst werden, eine nahezu identische oder identische Sequenz aufweisen;
- Bestimmen der Anzahl von T-Zellrezeptor-Nukleinsäuresequenzen, welche mit jedem Klonotypen assoziiert sind, oder Bestimmen der Anzahl von T-Zellrezeptor-Aminosäuresequenzen, welche mit jedem Klonotypen assoziiert sind, dadurch erhaltend eine Klonotyphäufigkeit für jeden der besagten Klonotypen;
- Selektieren eines tumorspezifischen Klonotyps aus der besagten Vielzahl von Tumorprobenklonotypen, wobei der besagte tumorspezifische Klonotyp einer der 100 häufigsten Klonotypen der besagten Vielzahl von Klonotypen ist und/oder ein anderer Klonotyp ist, der eine T-Zellrezeptor-Aminosäuresequenz umfasst, welche identisch oder nahezu identisch zu einer T-Zellrezeptor-Aminosäuresequenz ist, welche von einer T-Zellrezeptor-Nukleinsäuresequenz der besagten Vielzahl von T-Zellrezeptor-Nukleinsäuresequenzen kodiert wird, welche vom besagten einen tumorspezifischen Klonotyp der 100 häufigsten Klonotypen der besagten Vielzahl von Tumorprobenklonotypen umfasst wird; und
- Selektieren einer T-Zellrezeptor-Nukleinsäuresequenz der besagten Vielzahl von T-Zellrezeptor-Nukleinsäuresequenzen, welcher vom besagten selektierten tumorspezifischen Klonotypen umfasst wird, als tumorspezifische T-Zellrezeptor-Nukleinsäuresequenz, oder Selektieren einer T-Zellrezeptor-Aminosäuresequenz der besagten Vielzahl von T-Zellrezeptor-Aminosäuresequenzen, welcher vom besagten selektierten tumorspezifischen Klonotypen umfasst wird, als tumorspezifische T-Zellrezeptor-Aminosäuresequenz.

6. Das Verfahren nach Anspruch 5, weiter umfassend:
- Bereitstellen einer Nichttumorprobe erhalten von dem besagten Patienten;
- Isolieren einer Nukleinsäurepräparation von der besagten Nichttumorprobe in einem Nukleinsäureisolationsschritt;
- Erhalten einer Vielzahl von T-Zellrezeptor-Nukleinsäuresequenzen von der besagten Nukleinsäurepräparation oder einer Vielzahl von T-Zellrezeptor-Aminosäuresequenzen, welche durch die besagte Vielzahl von T-Zellrezeptor-Nukleinsäuresequenzen kodiert sind, erhaltend eine Vielzahl von nichttumorspezifischen T-Zellrezeptor-Nukleinsäuresequenzen oder eine Vielzahl von nichttumorspezifischen T-Zellrezeptor-Aminosäuresequenzen;
- Alignen der besagten Vielzahl von T-Zellrezeptor-Nukleinsäuresequenzen oder der besagten Vielzahl von T-Zellrezeptor-Aminosäuresequenzen;
- Gruppieren der T-Zellrezeptor-Nukleinsäuresequenzen, welche von der besagten Vielzahl von T-Zellrezeptor-Nukleinsäuresequenzen umfasst werden, oder der T-Zellrezeptor-Aminosäuresequenzen, welche von der besagten Vielzahl von T-Zellrezeptor-Aminosäuresequenzen umfasst werden, in eine Vielzahl von Klonotypen, wobei Nukleinsäuresequenzen oder Aminosäuresequenzen, welche von einem bestimmten Klonotypen umfasst werden, eine nahezu identische oder identische Sequenz aufweisen;
- Selektieren eines tumorspezifischen Klonotyps aus der Vielzahl von Tumorprobenklonotypen, wobei:
• der besagte tumorspezifische Klonotyp einer der 100 häufigsten Klonotypen der besagten Vielzahl von Klonotypen ist und/oder ein anderer Klonotyp ist, der eine T-Zellrezeptor-Aminosäuresequenz umfasst, welche identisch oder nahezu identisch zur einer T-Zellrezeptor-Aminosäuresequenz ist, welche von einer T-Zellrezeptor-Nukleinsäuresequenz der besagten Vielzahl von T-Zellrezeptor-Nukleinsäuresequenzen kodiert wird, welche vom besagten einen tumorspezifischen Klonotyp der 100 häufigsten Klonotypen der besagten Vielzahl von Tumorprobenklonotypen umfasst wird, und
• der besagte Klonotyp der 100 häufigsten Klonotypen oder besagten Vielzahl von Tumorprobenklonotypen abwesend in der Nichttumorprobe ist oder einem nichttumorspezifischen Klonotypen zugeordnet werden kann, der einer Häufigkeit von nicht mehr als 20 %, 15 %, 10 % oder 5 % der Häufigkeit des besagten tumorspezifischen Klonotyps aufweist.

7. Das Verfahren nach Anspruch 5 oder 6, weiter umfassend:
- Bereitstellen einer Blutprobe erhalten von dem besagten Patienten;
- Isolieren einer Nukleinsäurepräparation von der besagten Nichttumorprobe in einem Nukleinsäureisolationsschritt;
- Erhalten einer Vielzahl von T-Zellrezeptor-Nukleinsäuresequenzen von der besagten Nukleinsäurepräparation oder einer Vielzahl von T-Zellrezeptor-Aminosäuresequenzen, welche durch die besagte Vielzahl von T-Zellrezeptor-Nukleinsäuresequenzen kodiert sind, erhaltend eine Vielzahl von nichttumorspezifischen T-Zellrezeptor-Nukleinsäuresequenzen oder eine Vielzahl von nichttumorspezifischen T-Zellrezeptor-Aminosäuresequenzen;
- Alignen der besagten Vielzahl von T-Zellrezeptor-Nukleinsäuresequenzen oder der besagten Vielzahl von T-Zellrezeptor-Aminosäuresequenzen;
- Gruppieren der T-Zellrezeptor-Nukleinsäuresequenzen, welche von der besagten Vielzahl von T-Zellrezeptor-Nukleinsäuresequenzen umfasst werden, oder der T-Zellrezeptor-Aminosäuresequenzen, welche von der besagten Vielzahl von T-Zellrezeptor-Aminosäuresequenzen umfasst werden, in eine Vielzahl von Klonotypen, wobei Nukleinsäuresequenzen oder Aminosäuresequenzen, welche von einem bestimmten Klonotypen umfasst werden, eine nahezu identische oder identische Sequenz aufweisen;
- Selektieren eines tumorspezifischen Klonotyps aus der Vielzahl von Tumorprobenklonotypen, wobei
• der besagte tumorspezifische Klonotyp einer der 100 häufigsten Klonotypen der besagten Vielzahl von Klonotypen ist und/oder ein anderer Klonotyp der besagten Vielzahl von Tumorprobenklonotypen ist, der eine T-Zellrezeptor-Aminosäuresequenz umfasst, welche identisch oder nahezu identisch zur einer T-Zellrezeptor-Aminosäuresequenz ist, welche von einer T-Zellrezeptor-Nukleinsäuresequenz der besagten Vielzahl von T-Zellrezeptor-Nukleinsäuresequenzen kodiert wird, welche vom besagten einen tumorspezifischen Klonotyp der 100 häufigsten Klonotypen der besagten Vielzahl von Tumorprobenklonotypen umfasst wird, und
• der besagte Klonotyp der 100 häufigsten Klonotypen oder besagten Vielzahl von Tumorprobenklonotypen einem Blutprobenklonotypen zugeordnet werden kann, der eine Häufigkeit kleiner als die Häufigkeit des besagten tumorspezifischen Klonotyps aufweist.

8. Das Verfahren nach einem der Ansprüche 5 bis 7, weiter umfassend:
- Bereitstellen einer zellfreien Probe erhalten von dem besagten Patienten;
- Isolieren einer Nukleinsäurepräparation von der besagten Nichttumorprobe in einem Nukleinsäureisolationsschritt;
- Erhalten einer Vielzahl von T-Zellrezeptor-Nukleinsäuresequenzen von der besagten Nukleinsäurepräparation oder einer Vielzahl von T-Zellrezeptor-Aminosäuresequenzen, welche durch die besagte Vielzahl von T-Zellrezeptor-Nukleinsäuresequenzen kodiert sind, erhaltend eine Vielzahl von nichttumorspezifischen T-Zellrezeptor-Nukleinsäuresequenzen oder eine Vielzahl von nichttumorspezifischen T-Zellrezeptor-Aminosäuresequenzen;
- Alignen der besagten Vielzahl von T-Zellrezeptor-Nukleinsäuresequenzen oder der besagten Vielzahl von T-Zellrezeptor-Aminosäuresequenzen;
- Gruppieren der T-Zellrezeptor-Nukleinsäuresequenzen, welche von der besagten Vielzahl von T-Zellrezeptor-Nukleinsäuresequenzen umfasst werden, oder der T-Zellrezeptor-Aminosäuresequenzen, welche von der besagten Vielzahl von T-Zellrezeptor-Aminosäuresequenzen umfasst werden, in eine Vielzahl von Klonotypen, wobei Nukleinsäuresequenzen oder Aminosäuresequenzen, welche von einem bestimmten Klonotypen umfasst werden, eine nahezu identische oder identische Sequenz aufweisen;
- Selektieren eines tumorspezifischen Klonotyps aus der Vielzahl von Tumorprobenklonotypen, wobei:
• der besagte tumorspezifische Klonotyp einer der 100 häufigsten Klonotypen der besagten Vielzahl von Klonotypen ist und/oder ein anderer Klonotyp der besagten Vielzahl von Tumorprobenklonotypen ist, der eine T-Zellrezeptor-Aminosäuresequenz umfasst, welche identisch oder nahezu identisch zur einer T-Zellrezeptor-Aminosäuresequenz ist, welche von einer T-Zellrezeptor-Nukleinsäuresequenz der besagten Vielzahl von T-Zellrezeptor-Nukleinsäuresequenzen kodiert wird, welche vom besagten einen tumorspezifischen Klonotyp der 100 häufigsten Klonotypen der besagten Vielzahl von Tumorprobenklonotypen umfasst wird, und
• der besagte Klonotyp der 100 häufigsten Klonotypen oder besagten Vielzahl von Tumorprobenklonotypen einem Klonotypen der zellfreien Probe zugeordnet werden kann.

9. Das Verfahren nach einem der Ansprüche 5 bis 8, wobei der besagte tumorspezifische Klonotyp der 100 häufigsten Klonotypen der besagten Vielzahl der Tumorprobenklonotypen einem anderen Klonotypen der besagten Vielzahl der Tumorprobenklonotypen zugeordnet werden kann, welcher eine T-Zellrezeptor-Aminosäuresequenz umfasst, welche identisch oder nahezu identisch zur einer T-Zellrezeptor-Aminosäuresequenz, welche von einer T-Zellrezeptor-Nukleinsäuresequenz der besagten Vielzahl von T-Zellrezeptor-Nukleinsäuresequenzen kodiert wird, welche vom besagten einen tumorspezifischen Klonotyp der 100 häufigsten Klonotypen der besagten Vielzahl von Tumorprobenklonotypen umfasst wird, oder zu einer T-Zellrezeptor-Aminosäuresequenz der besagten Vielzahl von T-Zellrezeptor-Aminosäuresequenzen, welche vom besagten einen tumorspezifischen Klonotypen der 100 häufigsten tumorspezifischen Klonotypen umfasst wird.

10. Das Verfahren nach einem der Ansprüche 5 bis 9, wobei der häufigste Klonotyp der besagten Vielzahl von Tumorprobenklonotypen oder ein anderer Klonotyp der besagten Vielzahl von Tumorprobenklonotypen, der eine T-Zellrezeptor-Aminosäuresequenz umfasst, welche identisch oder nahezu identisch zur einer T-Zellrezeptor-Aminosäuresequenz ist, welche von einer T-Zellrezeptor-Nukleinsäuresequenz der besagten Vielzahl von T-Zellrezeptor-Nukleinsäuresequenzen kodiert wird, welche vom besagten einen tumorspezifischen Klonotyp der 100 häufigsten Klonotypen der besagten Vielzahl von Tumorprobenklonotypen umfasst wird, selektiert wird, wobei insbesondere der besagte häufigste Klonotyp abwesend in der Nichttumorprobe ist oder einem nichttumorspezifischen Klonotypen zugeordnet werden kann, der einer Häufigkeit von nicht mehr als 20 %, 15 %, 10 % oder 5 % der Häufigkeit des besagten tumorspezifischen Klonotyps aufweist, und/oder einem Blutprobenklonotypen zugeordnet werden kann, der eine Häufigkeit kleiner als die Häufigkeit des besagten tumorspezifischen Klonotyps aufweist; und/oder einem Klonotypen der zellfreien Probe zugeordnet werden kann; und/oder einem anderen Klonotypen der besagten Vielzahl der Tumorprobenklonotypen zugeordnet werden kann, welcher eine T-Zellrezeptor-Aminosäuresequenz umfasst, welche identisch oder nahezu identisch zur einer T-Zellrezeptor-Aminosäuresequenz, welche von einer T-Zellrezeptor-Nukleinsäuresequenz der besagten Vielzahl von T-Zellrezeptor-Nukleinsäuresequenzen kodiert wird, welche vom besagten einen tumorspezifischen Klonotyp der 100 häufigsten Klonotypen der besagten Vielzahl von Tumorprobenklonotypen umfasst wird.

11. Das Verfahren nach einem der Ansprüche 5 bis 10, umfassend:
- Selektieren von 5, 10. 15 oder 20 tumorspezifischen Klonotypen von der besagten Tumorprobe, wobei
• die besagten selektierten tumorspezifischen 5, 10, 15 oder 20 der 100 häufigsten Klonotypen die 5 häufigsten Klonotypen, die 10 häufigsten Klonotypen, die 15 häufigsten Klonotypen oder die 20 häufigsten Klonotypen der besagten Vielzahl von Tumorprobenklonotypen und/oder andere Klonotypen sind, welche eine T-Zellrezeptor-Aminosäuresequenz umfasst, welche identisch oder nahezu identisch zur einer T-Zellrezeptor-Aminosäuresequenz ist, welche von einer T-Zellrezeptor-Nukleinsäuresequenz der besagten Vielzahl von T-Zellrezeptor-Nukleinsäuresequenzen kodiert wird, welche von irgend einem der besagten selektierten 5, 10, 15 oder 20 tumorspezifischen Klonotypen der besagten Vielzahl von Tumorprobenklonotypen umfasst wird; und optional
• die besagten selektierten 5, 10, 15 oder 20 tumorspezifischen Klonotypen abwesend in der Nichttumorprobe sind oder einem nichttumorspezifischen Klonotypen zugeordnet werden können, der einer Häufigkeit von nicht mehr als 20 %, 15 %, 10 % oder 5 % der Häufigkeit des besagten 5, 10, 15 oder 20 tumorspezifischen Klonotypen aufweist; und/oder
• die besagten selektierten 5, 10, 15 oder 20 tumorspezifischen Klonotypen einem Klonotypen der zellfreien Probe zugeordnet werden können; und/oder
• die besagten selektierten 5, 10, 15 oder 20 tumorspezifischen Klonotypen einem anderen Klonotypen zugeordnet werden können, welcher eine T-Zellrezeptor-Aminosäuresequenz umfasst, welche identisch oder nahezu identisch zur einer T-Zellrezeptor-Aminosäuresequenz ist, welche von einer T-Zellrezeptor-Nukleinsäuresequenz der besagten Vielzahl von T-Zellrezeptor-Nukleinsäuresequenzen kodiert wird, welche den besagten selektierten 5, 10, 15 oder 20 tumorspezifischen Klonotypen der besagten Vielzahl von Tumorprobenklonotypen umfasst werden.

12. Das Verfahren nach einen der Ansprüche 5 bis 11, wobei
- irgendeiner des besagten einen der 100 häufigsten Klonotypen, oder der besagten selektierten 5, 10, 15 oder 20 tumorspezifischen Klonotypen einem Nichttumorklonotyp zugeordnet wird, wenn ein T-Zellrezeptor-Aminosäuresequenz, welche von einer T-Zellrezeptor-Nukleinsäuresequenz der besagten Vielzahl von T-Zellrezeptor-Nukleinsäuresequenzen kodiert wird, welche vom besagten tumorspezifischen Klonotypen umfasst wird, oder eine T-Zellrezeptor-Aminosäuresequenz, welche vom besagten tumorspezifischen Klonotypen umfasst wird, identisch zu einer T-Zellrezeptor-Aminosäuresequenz ist, welche von einer T-Zellrezeptor-Nukleinsäuresequenz ist, welche vom besagten Nichttumorklonotypen umfasst wird, oder wenn eine T-Zellrezeptor-Aminosäuresequenz der besagten Vielzahl von T-Zellrezeptor-Aminosäuresequenzen, welche vom besagten tumorspezifischen Klonotypen umfasst wird, identisch einer T-Zellrezeptor-Aminosäuresequenz ist, welche vom besagten Nichttumorklonotypen umfasst wird; und/oder
- irgendeiner des besagten einen der 100 häufigsten Klonotypen, oder der besagten selektierten 5, 10, 15 oder 20 tumorspezifischen Klonotypen einem Blutprobenklonotypen zugeordnet wird, wenn ein T-Zellrezeptor-Aminosäuresequenz, welche von einer T-Zellrezeptor-Nukleinsäuresequenz der besagten Vielzahl von T-Zellrezeptor-Nukleinsäuresequenzen kodiert wird, welche vom besagten tumorspezifischen Klonotypen umfasst wird, oder eine T-Zellrezeptor-Aminosäuresequenz, welche vom besagten tumorspezifischen Klonotypen umfasst wird, identisch zu einer T-Zellrezeptor-Aminosäuresequenz ist, welche von einer T-Zellrezeptor-Nukleinsäuresequenz ist, welche vom besagten Blutprobenklonotypen umfasst wird, oder wenn eine T-Zellrezeptor-Aminosäuresequenz der besagten Vielzahl von T-Zellrezeptor-Aminosäuresequenzen, welche vom besagten tumorspezifischen Klonotypen umfasst wird, identisch einer T-Zellrezeptor-Aminosäuresequenz ist, welche vom besagten Blutprobenklonotypen umfasst wird; und/oder
- irgendeiner des besagten einen der 100 häufigsten Klonotypen, oder der besagten selektierten 5, 10, 15 oder 20 tumorspezifischen Klonotypen einem Klonotypen der zellfreien Probe zugeordnet wird, wenn ein T-Zellrezeptor-Aminosäuresequenz, welche von einer T-Zellrezeptor-Nukleinsäuresequenz der besagten Vielzahl von T-Zellrezeptor-Nukleinsäuresequenzen kodiert wird, welche vom besagten tumorspezifischen Klonotypen umfasst wird, oder eine T-Zellrezeptor-Aminosäuresequenz, welche vom besagten tumorspezifischen Klonotypen umfasst wird, identisch zu einer T-Zellrezeptor-Aminosäuresequenz ist, welche von einer T-Zellrezeptor-Nukleinsäuresequenz ist, welche vom besagten Klonotypen der zellfreien Probe umfasst wird, oder wenn eine T-Zellrezeptor-Aminosäuresequenz der besagten Vielzahl von T-Zellrezeptor-Aminosäuresequenzen, welche vom besagten tumorspezifischen Klonotypen umfasst wird, identisch einer T-Zellrezeptor-Aminosäuresequenz ist, welche vom besagten Klonotypen der zellfreien Probe umfasst wird.

13. Das Verfahren nach einem der vorherigen Ansprüche, wobei der besagte Nukleinsäureisolationsschritt die Schritte umfasst:
a. Isolieren von T-Zell von der besagten Tumorprobe und Isolieren von Nukleinsäure von den isolierten T-Zellen, und/oder
b. Durchführen einer Nukleinsäureamplifikationsreaktion, welche spezifisch T-Zellrezeptor-Nukleinsäuresequenzen amplifiziert.

14. Das Verfahren nach einem der vorherigen Ansprüche, wobei die besagte tumorspezifische T-Zellrezeptor-Nukleinsäuresequenz die CDR3-Region einer Kette des humanen T-Zellrezeptors kodiert, und/oder die besagte tumorspezifische T-Zellrezeptor-Aminosäuresequenz die CDR3-Region einer Kette des humanen T-Zellrezeptors ist oder von der CDR3-Region einer Kette des humanen T-Zellrezeptors umfasst wird.

## Revendications

1. Procédé de fourniture d'une préparation de lymphocytes T spécifiques à la tumeur, comprenant les étapes consistant à :
a. sélectionner des clones de lymphocytes T spécifiques à la tumeur par :
- fourniture d'un échantillon de tumeur obtenu auprès d'un patient ;
- isolement d'une préparation d'acide nucléique à partir dudit échantillon de tumeur dans une étape d'isolement d'acide nucléique ;
- obtention d'une pluralité de séquences d'acides nucléiques récepteurs de lymphocytes T à partir de ladite préparation d'acide nucléique ou d'une pluralité de séquences d'acides aminés récepteurs de lymphocytes T codés par ladite pluralité de séquences d'acides nucléiques récepteurs de lymphocytes T ;
- sélection d'une séquence d'acide nucléique récepteurs de lymphocytes T spécifique à la tumeur à partir de ladite pluralité de séquences d'acides nucléiques récepteurs de lymphocytes T ou d'une séquence d'acide aminé récepteur de lymphocytes T spécifique à la tumeur à partir d'une pluralité de séquences d'acides aminés récepteurs de lymphocytes T dans une étape de sélection de séquence ;
b. trier des clones de lymphocytes T spécifiques à la tumeur par :
- fourniture d'une préparation de lymphocytes obtenus auprès dudit patient ;
- isoler des cellules qui comprennent ladite séquence d'acide nucléique récepteur de lymphocytes T spécifiques à la tumeur sélectionnée ou ladite séquence d'acide aminé récepteurs de lymphocytes T spécifiques à la tumeur sélectionnée à partir de ladite préparation de lymphocytes dans une étape d'isolement.

2. Procédé selon la revendication 1, dans lequel ladite étape d'isolement comprend les étapes consistant à :
- mettre en contact ladite préparation de lymphocytes avec un ligand spécifiquement réactif qui est à même de se lier à une séquence d'acide aminé comprise dans la région V du récepteur de lymphocytes T qui correspond à ladite séquence d'acide nucléique de récepteur de lymphocytes spécifiques à la tumeur sélectionnée ou à ladite séquence d'acide aminé récepteur de lymphocytes T sélectionnée, dans lequel ledit ligand est fixé à une étiquette détectable et dans lequel en particulier ledit ligand se lie à ladite séquence d'acide aminé avec une constante de dissociation de 10⁻⁷, 10⁻⁸ ou 10⁻⁹ mole/L ou moins et
- isoler les lymphocytes T portant ladite étiquette détectable de ladite préparation de lymphocytes.

3. Procédé selon la revendication 1, dans lequel ladite étape d'isolement comprend les étapes consistant à :
- mettre en contact ladite préparation de lymphocytes avec une sonde d'acide nucléique se liant spécifiquement à ladite séquence d'acide nucléique récepteur de lymphocyte T spécifiques à la tumeur sélectionnée, dans lequel ladite sonde d'acide nucléique est fixée à une étiquette détectable ;
- isoler les lymphocytes T portant ladite étiquette détectable de ladite préparation de lymphocytes ou
ladite étape d'isolement comprend :
- une étape de séparation, dans laquelle ladite préparation de lymphocytes est séparée en une pluralité de fractions,
- une étape d'expansion, dans laquelle les cellules comprises dans ladite pluralité de fractions sont expansées dans des conditions de culture cellulaire et
- une étape de sélection, dans laquelle au moins une fraction de ladite pluralité de fractions qui comprend ladite séquence d'acide nucléique récepteur de lymphocytes T spécifiques à la tumeur sélectionnée ou ladite séquence d'acide aminé récepteur de lymphocytes spécifiques à la tumeur sélectionnée est sélectionnée, et ladite étape d'isolement comprend éventuellement en outre la répétition de ladite étape de séparation, de ladite étape d'expansion et de ladite étape de sélection avec ladite au moins une fraction sélectionnée de ladite pluralité.

4. Procédé selon la revendication 3, dans lequel ladite étape de sélection comprend :
- la mise en contact de ladite pluralité de fractions avec une sonde d'acide nucléique se liant spécifiquement à ladite séquence d'acide nucléique récepteur de lymphocytes T spécifiques à la tumeur sélectionnée, dans laquelle ladite sonde d'acide nucléique est fixée à une étiquette détectable et l'identification de fractions comprenant lesdites séquences de récepteurs de lymphocytes spécifiques à la tumeur sélectionnées ou
- l'obtention de séquences d'acides nucléiques récepteurs de lymphocytes T à partir de ladite pluralité de fractions et l'identification de fractions comprenant ladite séquence d'acide nucléique récepteur de lymphocytes T spécifiques à la tumeur sélectionnée.

5. Procédé selon l'une quelconque des revendications 1 à 4, dans lequel ladite étape de sélection de séquences comprend les étapes consistant à :
- aligner ladite pluralité de séquences d'acides nucléiques récepteurs de lymphocytes T ou ladite pluralité de séquences d'acides aminés récepteurs de lymphocytes T ;
- grouper les séquences d'acides nucléiques récepteurs de lymphocytes T comprises dans ladite pluralité de séquences d'acides nucléiques récepteurs de lymphocytes T ou de séquences d'acides aminés récepteurs de lymphocytes T comprises dans ladite pluralité de séquences d'acides aminés récepteurs de lymphocytes T en une pluralité de clonotypes d'échantillon de tumeur, dans lequel les séquences d'acides nucléiques ou les séquences d'acides aminés comprises dans un clonotype particulier présentent une séquence virtuellement identique ou une séquence identique ;
- déterminer le nombre de séquences d'acides nucléiques récepteurs de lymphocytes T associées à chaque clonotype et déterminer le nombre de séquences d'acides aminés récepteurs de lymphocytes T associées à chaque clonotype, en donnant de la sorte une fréquence de clonotype pour chacun desdits clonotypes ;
- sélectionner un clonotype spécifique à la tumeur parmi ladite pluralité de clonotypes d'échantillon de tumeur, dans lequel ledit clonotype spécifique à la tumeur est l'un des 100 clonotypes les plus fréquents de ladite pluralité de clonotypes d'échantillon de tumeur et/ou est un autre clonotype de ladite pluralité de clonotypes d'échantillon de tumeur qui comprend une séquence d'acides aminés récepteurs de lymphocytes T qui est identique ou virtuellement identique à un acide aminé récepteur de lymphocytes T codé par une séquence d'acide nucléique récepteur de lymphocytes T de ladite pluralité de séquences nucléiques récepteurs de lymphocytes T comprises dans ledit un clonotype spécifique à la tumeur parmi les 100 clonotypes les plus fréquents de ladite pluralité de clonotypes d'échantillon de tumeur, et
- la sélection d'une séquence d'acide nucléique récepteur de lymphocytes T de ladite pluralité de séquences d'acides nucléiques récepteurs de lymphocytes T comprise dans ledit clonotype spécifique à la tumeur sélectionné comme dite séquence d'acide nucléique récepteur spécifique à la tumeur ou la sélection d'une séquence d'acide aminé récepteur de lymphocytes T de ladite pluralité de séquences d'acides aminés récepteurs de lymphocytes T comprise dans le clonotype spécifique à la tumeur sélectionné comme dite séquence d'acide aminé spécifique à la tumeur.

6. Procédé selon la revendication 5, comprenant en outre :
- la fourniture d'un échantillon non tumoral obtenu auprès dudit patient ;
- l'isolement d'une préparation d'acide nucléique à partir dudit échantillon non tumoral dans une étape d'isolement d'acide nucléique ;
- l'obtention d'une pluralité de séquences d'acides nucléiques récepteurs de lymphocytes T dans ladite préparation d'acide nucléique ou d'une pluralité de séquences d'acides aminés récepteurs de lymphocytes T codées par ladite pluralité de séquences d'acides nucléiques récepteurs de lymphocytes T, en donnant une pluralité de séquences d'acides nucléiques récepteurs de lymphocytes T non spécifiques à la tumeur ou une pluralité de séquences d'acides aminés récepteurs de lymphocytes T non spécifiques à la tumeur ;
- l'alignement de ladite pluralité de séquences d'acides nucléiques récepteurs de lymphocytes T non spécifiques à la tumeur ou de ladite pluralité de séquences d'acides aminés récepteurs de lymphocytes T non spécifiques à la tumeur ;
- le groupement de séquences d'acides nucléiques récepteurs de lymphocytes T comprises dans ladite pluralité de séquences d'acides nucléiques récepteurs de lymphocytes T non spécifiques à la tumeur ou ladite pluralité de séquences d'acides aminés récepteurs de lymphocytes T non spécifiques à la tumeur dans une pluralité de clonotypes non spécifiques à la tumeur, dans lequel les séquences d'acides nucléiques récepteurs de lymphocytes T ou les séquences d'acides aminés récepteurs de lymphocytes T comprises dans un clonotype particulier présentent une séquence virtuellement identique ou une séquence identique ;
- la sélection d'un clonotype spécifique à la tumeur parmi ladite pluralité de clonotypes d'échantillon de tumeur, dans lequel :
• ledit clonotype spécifique à la tumeur est l'un des 100 clonotypes les plus fréquents de ladite pluralité d'échantillons de tumeur ou est un autre clonotype de ladite pluralité de clonotypes d'échantillon de tumeur qui comprend une séquence d'acides aminés de lymphocytes T qui est identique ou virtuellement identique à un acide aminé récepteur de lymphocytes T codé par une séquence d'acide nucléique récepteur de lymphocytes T de ladite pluralité de séquences d'acides nucléiques récepteurs de lymphocytes T comprise dans ledit un clonotype spécifique à la tumeur parmi les 100 clonotypes les plus fréquents de ladite pluralité de clonotypes d'échantillon de tumeur, et
• ledit clonotype spécifique à la tumeur parmi les 100 clonotypes les plus fréquents de ladite pluralité de clonotypes d'échantillon de tumeur est absent dans ledit échantillon non tumoral ou peut être affecté à un clonotype non spécifique à la tumeur qui présente une fréquence qui n'est pas supérieure à 20 %, 15 %, 10 % ou 5 % de la fréquence dudit clonotype spécifique à la tumeur.

7. Procédé selon la revendication 5 ou 6, comprenant en outre :
- la fourniture d'un échantillon de sang obtenu auprès dudit patient ;
- l'isolement d'une préparation d'acide nucléique à partir dudit échantillon de sang dans une étape d'isolement d'acide nucléique ;
- l'obtention d'une pluralité de séquences d'acides nucléiques récepteurs de lymphocytes T à partir de ladite préparation d'acide nucléique ou d'une pluralité de séquences d'acides aminés récepteurs de lymphocytes T codés par ladite pluralité de séquences d'acides nucléiques récepteurs de lymphocytes T ;
- l'alignement de séquences d'acides nucléiques récepteurs de lymphocytes T comprises dans ladite pluralité de séquences d'acides nucléiques récepteurs de lymphocytes T ou de séquences d'acides aminés récepteurs de lymphocytes T en une pluralité de clonotypes d'échantillon de sang, dans lequel les séquences d'acides nucléiques récepteurs de lymphocytes T ou les séquences d'acides aminés récepteurs de lymphocytes T comprises dans un clonotype particulier présentent une séquence virtuellement identique ou une séquence identique ;
- la sélection d'un clonotype spécifique à la tumeur parmi ladite pluralité de clonotypes d'échantillon de tumeur, dans lequel :
• ledit clonotype spécifique à la tumeur est l'un des 100 clonotypes les plus fréquents de ladite pluralité d'échantillons de tumeur ou est un autre clonotype de ladite pluralité de clonotypes d'échantillon de tumeur qui comprend une séquence d'acides aminés de lymphocytes qui est identique ou virtuellement identique à un acide aminé récepteur de lymphocytes T codé par une séquence d'acide nucléique récepteur de lymphocytes T de ladite pluralité de séquences d'acides nucléiques récepteurs de lymphocyte T comprise dans ledit un clonotype spécifique à la tumeur parmi les 100 clonotypes les plus fréquents de ladite pluralité de clonotypes d'échantillon de tumeur, et
• ledit clonotype spécifique à la tumeur parmi les 100 clonotypes les plus fréquents de ladite pluralité d'échantillons de tumeur peut être affecté à un clonotype d'échantillon de sang qui présente une fréquence inférieure à la fréquence dudit clonotype spécifique à la tumeur.

8. Procédé selon l'une quelconque des revendications 5 à 7, comprenant en outre :
- la fourniture d'un échantillon sans cellule obtenu auprès dudit patient ;
- l'isolement d'une préparation d'acide nucléique à partir dudit échantillon exempt de cellules dans une étape d'isolement d'acide nucléique ;
- l'obtention d'une pluralité de séquences d'acides nucléiques récepteurs de lymphocytes T à partir de ladite préparation d'acide nucléique ou d'une pluralité de séquences d'acides aminés récepteurs de lymphocytes T codée par ladite pluralité de séquences d'acides nucléiques récepteurs de lymphocytes T ;
- l'alignement de ladite pluralité de séquences d'acides nucléiques récepteurs de lymphocytes T ou de ladite pluralité de séquences d'acides aminés récepteurs de lymphocytes T ;
- le groupement de séquences d'acides nucléiques récepteurs de lymphocytes T comprises dans ladite pluralité de séquences d'acides nucléiques récepteurs de lymphocytes T ou de séquences d'acides aminés récepteurs de lymphocytes T comprise dans ladite pluralité de séquences d'acides nucléiques de lymphocytes T en une pluralité de clonotypes d'échantillon exempt de cellules, dans lequel les séquences d'acides nucléiques récepteurs de lymphocytes T ou les séquences d'acides aminés récepteurs de lymphocytes T comprises dans un clonotype particulier présentent une séquence virtuellement identique ou une séquence identique ;
- la sélection d'un clonotype spécifique à la tumeur parmi ladite pluralité de clonotypes d'échantillon de tumeur, dans lequel :
• ledit clonotype spécifique à la tumeur est l'un des 100 clonotypes les plus fréquents de ladite pluralité de clonotypes d'échantillon de tumeur ou est un autre clonotype de ladite pluralité de clonotypes d'échantillon de tumeur qui comprend une séquence d'acide aminé de lymphocytes T qui est identique ou virtuellement identique à une séquence d'acide aminé récepteur de lymphocytes T codée par une séquence d'acide nucléique récepteur de lymphocytes T de ladite pluralité de séquences d'acides nucléiques récepteurs de lymphocytes T comprise dans ledit clonotype spécifique à la tumeur des 100 clonotypes les plus fréquents de ladite pluralité de clonotypes d'échantillon de tumeur et
• ledit clonotype spécifique à la tumeur des 100 clonotypes les plus fréquents de ladite pluralité d'échantillons de tumeur peut être affecté à un clonotype d'échantillon exempt de cellules.

9. Procédé selon l'une quelconque des revendications 5 à 8, dans lequel ledit clonotype spécifique à la tumeur des 100 clonotypes les plus fréquents de ladite pluralité de clonotypes d'échantillon de tumeur peut être affecté à un autre clonotype de ladite pluralité de clonotypes d'échantillon de tumeur qui comprend une séquence d'acide aminé de lymphocyte T qui est identique ou virtuellement identique à un acide aminé récepteur de lymphocyte T codé par une séquence d'acide nucléique récepteur de lymphocytes T de ladite pluralité de séquences d'acide nucléiques récepteurs de lymphocytes T comprise dans ledit un clonotype spécifique à la tumeur des 100 clonotypes spécifiques à la tumeur les plus fréquents ou à une séquence d'acide aminé récepteur de lymphocytes T de ladite pluralité de séquences d'acides aminés récepteurs de lymphocytes T comprise dans ledit un clonotype spécifique à la tumeur des 100 clonotypes spécifiques à la tumeur les plus fréquents.

10. Procédé selon l'une quelconque des revendications 5 à 9, dans lequel on sélectionne le clonotype le plus fréquent desdits clonotypes d'échantillon de tumeur ou un autre clonotype de ladite pluralité de clonotypes d'échantillon de tumeur qui comprend une séquence d'acides aminés de lymphocytes T qui est virtuellement identique ou identique à un acide aminé récepteur de lymphocytes T codé par une séquence de récepteur de lymphocytes T de ladite pluralité de séquences d'acides nucléiques récepteurs de lymphocytes T comprise dans ledit clonotype spécifique à la tumeur le plus fréquent, dans lequel, en particulier, ledit clonotype le plus fréquent est absent dans ledit échantillon non tumoral ou peut être affecté à un clonotype non tumoral qui montre une fréquence de pas plus de 20 %, de 15 %, de 10 % ou de 5 % de la fréquence dudit clonotype spécifique à la tumeur, et/ou peut être affecté à un clonotype d'échantillon de sang qui présente une fréquence inférieure à la fréquence desdits clonotypes d'échantillon de tumeur respectifs, et/ou peut être affecté à un clonotype exempt de cellules et/ou peut être affecté à un autre clonotype de ladite pluralité de clonotypes d'échantillon de tumeur qui comprend une séquence d'acide aminé de lymphocytes T qui est identique ou virtuellement identique à un acide aminé récepteur de lymphocytes T par une séquence d'acide nucléique récepteur de lymphocytes T de ladite pluralité de séquences d'acides nucléiques récepteurs de lymphocytes T comprise dans ledit clonotype spécifique à la tumeur le plus fréquent.

11. Procédé selon l'une quelconque des revendications 5 à 10, comprenant :
- la sélection de 5, 10, 15 ou 20 clonotypes spécifiques à la tumeur issus dudit échantillon de tumeur, dans lequel :
• lesdits clonotypes spécifiques à la tumeur sélectionnés sont 5, 10, 15 ou 20 des 100 clonotypes les plus fréquents, les 5 clonotypes les plus fréquents, les 10 clonotypes les plus fréquents, les 15 clonotypes les plus fréquents ou les 20 clonotypes les plus fréquents de ladite pluralité de clonotypes d'échantillon de tumeur et/ou sont d'autres clonotypes de ladite pluralité de clonotypes d'échantillon de tumeur qui comprennent une séquence d'acide aminé de lymphocytes T qui est virtuellement identique ou identique à un acide aminé récepteur de lymphocytes T par une séquence d'acide nucléique récepteur de lymphocytes T de ladite pluralité de séquences d'acide nucléique récepteurs de lymphocytes T comprise dans l'un quelconque des 5, 10, 15 ou 20 clonotypes spécifiques à la tumeur sélectionnés de ladite pluralité de clonotypes d'échantillon de tumeur et éventuellement
• lesdits 5, 10, 15 ou 20 clonotypes spécifiques à la tumeur sélectionnés sont absents dans ledit échantillon non tumoral ou peuvent être affectés à un clonotype non spécifique à la tumeur qui présente une fréquence de pas plus de 20 %, de 15 %, de 10 % ou de 5 % de la fréquence desdits 5, 10, 15 ou 20 clonotypes spécifiques à la tumeur sélectionnés de ladite pluralité de clonotypes d'échantillon de tumeur, et/ou
• lesdits 5, 10, 15 ou 20 clonotypes spécifiques à la tumeur sélectionnés peuvent être affectés à un clonotype d'échantillon de sang qui présente une fréquence inférieure à la fréquence desdits 5, 10, 15 ou 20 clonotypes spécifiques à la tumeur de ladite pluralité de clonotypes d'échantillon de tumeur, et/ou
• lesdits 5, 10, 15 ou 20 clonotypes spécifiques à la tumeur sélectionnés peuvent être affectés à un clonotype d'échantillon exempt de cellules, et/ou
• lesdits 5, 10, 15 ou 20 clonotypes spécifiques à la tumeur sélectionnés peuvent être affectés à un autre clonotype de ladite pluralité de clonotypes d'échantillon de tumeur qui comprend une séquence d'acide aminé de lymphocytes T qui est virtuellement identique ou identique à un acide aminé récepteur de lymphocytes T par l'une quelconque des séquences d'acides nucléiques récepteurs de lymphocytes T de ladite pluralité de séquences de récepteurs de lymphocytes T comprises dans lesdits 5, 10, 15 ou 10 clonotypes spécifiques à la tumeur sélectionnés de ladite pluralité de clonotypes d'échantillon de tumeur.

12. Procédé selon l'une quelconque des revendications 5 à 11, dans lequel :
- l'un quelconque dudit un des 100 clonotypes les plus fréquents, des 5, 10, 15 ou 20 clonotypes spécifiques à la tumeur sélectionnés de ladite pluralité de clonotypes d'échantillon de tumeur est affecté à un clonotype non spécifique à la tumeur, si une séquence d'acide aminé récepteur de lymphocytes T codée par une séquence de récepteur d'acide nucléique de lymphocytes T de ladite pluralité de séquences d'acides nucléiques récepteurs de lymphocytes T comprise dans ledit clonotype spécifique à la tumeur ou une séquence d'acide aminé de lymphocytes T de ladite pluralité de séquences d'acides aminés comprises avec ledit clonotype spécifique à la tumeur est identique à une séquence d'acide aminé récepteur de lymphocytes T codée par une séquence d'acide nucléique récepteur de lymphocytes T comprise dans ledit clonotype d'échantillon non tumoral ou si une séquence d'acide aminé de lymphocytes T de ladite pluralité de séquences d'acides aminés récepteurs de lymphocytes T comprise avec ledit clonotype spécifique à la tumeur est identique à une séquence d'acide aminé récepteur de lymphocytes T comprise dans ledit clonotype d'échantillon non tumoral, et/ou
- l'un quelconque desdits un des 100 clonotypes les plus fréquents, lesdits 5, 10, 15 ou 20 clonotypes spécifiques à la tumeur de ladite pluralité de clonotypes d'échantillon de tumeur est affecté à un clonotype d'échantillon de sang, si une séquence d'acide aminé récepteur de lymphocytes T codée par une séquence de récepteur de lymphocytes T comprise dans ledit clonotype spécifique à la tumeur est identique à une séquence d'acide aminé récepteur de lymphocytes T codée par une séquence d'acide nucléique récepteur de lymphocytes T comprise dans ledit clonotype d'échantillon de sang ou si une séquence d'acide aminé de lymphocytes T comprise avec ledit clonotype spécifique à la tumeur est identique à une séquence d'acide aminé récepteur de lymphocytes T comprise dans ledit clonotype d'échantillon de sang, et/ou
- l'un quelconque desdits un des 100 clonotypes les plus fréquents, desdits 5, 10 ou 20 clonotypes spécifiques à la tumeur sélectionnés de ladite pluralité de clonotypes d'échantillon de tumeur est affecté à un clonotype d'échantillon exempt de cellules, si une séquence d'acide aminé récepteur de lymphocytes T codée par une séquence d'acide nucléique récepteur de lymphocytes T de ladite pluralité de séquences d'acides nucléiques récepteurs de lymphocytes T comprise dans ledit clonotype spécifique à la tumeur est identique à un séquence d'acide aminé récepteur de lymphocytes T codée par une séquence d'acide nucléique récepteur de lymphocytes T comprise avec ledit clonotype d'échantillon exempt de cellules, ou si une séquence d'acide aminé de lymphocytes T de ladite pluralité de séquences d'acides aminés de lymphocytes T comprise dans ledit clonotype spécifique à la tumeur est identique à une séquence d'acide aminé récepteur de lymphocytes T comprise avec ledit clonotype d'échantillon exempt de cellules.

13. Procédé selon l'une quelconque des revendications précédentes, dans lequel ladite étape d'isolement d'acide nucléique comprend les étapes consistant à :
a. isoler des lymphocytes T dudit échantillon de tumeur et isoler l'acide nucléique desdits lymphocytes T isolés et/ou
b. effectuer une réaction d'amplification d'acide nucléique qui amplifie spécifiquement les séquences d'acide nucléique récepteur de lymphocytes T.

14. Procédé selon l'une quelconque des revendications précédentes, dans lequel ladite séquence d'acide nucléique récepteur de lymphocytes T spécifiques à la tumeur code la région CDR3 d'une chaîne du récepteur de lymphocytes T humains ou de ladite séquence d'acide aminé récepteur de lymphocytes T spécifique à la tumeur est comprise dans la région CDR3 d'une chaîne du récepteur de lymphocytes T humains.


### SEQUENCE LISTING

<110> HS Diagnomics GmbH
<120> Method for providing tumour-specific T cells
<130> hsd103wo
<160> 101
<170> PatentIn version 3.5
<210> 1
   <211> 13
   <212> PRT
   <213> Homo sapiens
<400> 1
   Cys Ala Ser Ser Val Asp Arg Gly Ala Glu Ala Phe Phe
   1               5                   10
<210> 2
   <211> 12
   <212> PRT
   <213> Homo sapiens
<400> 2
   Cys Ala Trp Asn Lys Gln Val Asp Gly Tyr Thr Phe
   1               5                   10
<210> 3
   <211> 14
   <212> PRT
   <213> Homo sapiens
<400> 3
   Cys Ala Ser Ser Phe Gly Val Met Asn Thr Glu Ala Phe Phe
   1               5                   10
<210> 4
   <211> 12
   <212> PRT
   <213> Homo sapiens
<400> 4
   Cys Ala Ser Ser Pro Asp Gly Glu Thr Gln Tyr Phe
   1               5                   10
<210> 5
   <211> 13
   <212> PRT
   <213> Homo sapiens
<400> 5
   Cys Ala Ser Ser Leu Gly Gln Ala Tyr Glu Gln Tyr Phe
   1 5 10
<210> 6
   <211> 15
   <212> PRT
   <213> Homo sapiens
<400> 6
   Cys Ala Ser Ser Pro Val Ala Gly Met Asn Thr Glu Ala Phe Phe
   1 5 10 15
<210> 7
   <211> 15
   <212> PRT
   <213> Homo sapiens
<400> 7
   Cys Ala Ile Ser Asp Trp Thr Gly Ser Asn Tyr Gly Tyr Thr Phe
   1 5 10 15
<210> 8
   <211> 14
   <212> PRT
   <213> Homo sapiens
<400> 8
   Cys Ala Ser Ser Gly Arg Gly Asp Leu Leu Glu Gln Tyr Phe
   1 5 10
<210> 9
   <211> 14
   <212> PRT
   <213> Homo sapiens
<400> 9
   Cys Ala Ser Ser Glu Thr Gly Ala Ala Glu Thr Gln Tyr Phe
   1 5 10
<210> 10
   <211> 15
   <212> PRT
   <213> Homo sapiens
<400> 10
   Cys Ala Ser Ser Arg Leu Ala Gly Gly Thr Asp Thr Gln Tyr Phe
   1 5 10 15
<210> 11
   <211> 13
   <212> PRT
   <213> Homo sapiens
<400> 11
   Cys Ala Ser Ser Ser Gly Leu Val Tyr Glu Gln Tyr Phe
   1 5 10
<210> 12
   <211> 15
   <212> PRT
   <213> Homo sapiens
<400> 12
   Cys Ala Ser Ser Thr Gly Thr Gly Gly Leu Gly Glu Leu Phe Phe
   1 5 10 15
<210> 13
   <211> 15
   <212> PRT
   <213> Homo sapiens
<400> 13
   Cys Ala Ser Ser Glu Ala Pro Pro Leu Tyr Tyr Glu Gln Tyr Phe
   1 5 10 15
<210> 14
   <211> 15
   <212> PRT
   <213> Homo sapiens
<400> 14
   Cys Ala Ser Ser Asn Asp Arg Ala Gly Leu Asn Glu Gln Phe Phe
   1 5 10 15
<210> 15
   <211> 12
   <212> PRT
   <213> Homo sapiens
<400> 15
   Cys Ala Thr Ser Asp Gly Arg Leu Glu Gln Phe Phe
   1 5 10
<210> 16
   <211> 15
   <212> PRT
   <213> Homo sapiens
<400> 16
   Cys Ala Ser Ser Leu Gly Tyr Arg Tyr Gly Thr Glu Ala Phe Phe
   1 5 10 15
<210> 17
   <211> 14
   <212> PRT
   <213> Homo sapiens
<400> 17
   Cys Ala Ser Ser Gln Asp Asn Gly Gly Tyr Gly Tyr Thr Phe
   1               5                   10
<210> 18
   <211> 14
   <212> PRT
   <213> Homo sapiens
<400> 18
   Cys Ala Ser Ser Gln Gly Asp Ser Phe Tyr Gly Tyr Thr Phe
   1               5                   10
<210> 19
   <211> 16
   <212> PRT
   <213> Homo sapiens
<400> 19
   Cys Ala Ser Ser Ala Asp Leu Gly Asp Arg Val Asn Gly Tyr Thr Phe
   1               5                   10                  15
<210> 20
   <211> 14
   <212> PRT
   <213> Homo sapiens
<400> 20
   Cys Ala Ser Ser Leu Asp Arg Gly Gly Tyr Glu Gln Tyr Phe
   1               5                   10
<210> 21
   <211> 14
   <212> PRT
   <213> Homo sapiens
<400> 21
   Cys Ala Arg Pro Pro Ala Gly Ile Pro Asp Thr Gln Tyr Phe
   1               5                   10
<210> 22
   <211> 15
   <212> PRT
   <213> Homo sapiens
<400> 22
   Cys Ala Ser Ser Asp Gln Gly His Ser Asn Gln Pro Gln His Phe
   1               5                   10                  15
<210> 23
   <211> 15
   <212> PRT
   <213> Homo sapiens
<400> 23
   Cys Ala Ser Ser Arg Pro Ser Phe Arg Val Ser Glu Gln Phe Phe
   1 5 10 15
<210> 24
   <211> 16
   <212> PRT
   <213> Homo sapiens
<400> 24
   Cys Ala Ser Ser Leu Leu Leu Ala Gly Ala Ser Tyr Glu Gln Tyr Phe
   1 5 10 15
<210> 25
   <211> 14
   <212> PRT
   <213> Homo sapiens
<400> 25
   Cys Ala Ser Ser Ser Phe Gln Gly Gly Asn Glu Gln Phe Phe
   1 5 10
<210> 26
   <211> 13
   <212> PRT
   <213> Homo sapiens
<400> 26
   Cys Ala Ser Ser Leu Val Arg Gly Asn Glu Gln Phe Phe
   1 5 10
<210> 27
   <211> 16
   <212> PRT
   <213> Homo sapiens
<400> 27
   Cys Ala Ser Ser Leu Glu Arg Ser Glu Arg Pro Tyr Glu Gln Tyr Phe
   1 5 10 15
<210> 28
   <211> 14
   <212> PRT
   <213> Homo sapiens
<400> 28
   Cys Ala Ser Thr Pro Arg Gly Asn Thr Gly Glu Leu Phe Phe
   1 5 10
<210> 29
   <211> 14
   <212> PRT
   <213> Homo sapiens
<400> 29
   Cys Ala Ser Asn Pro Gly Arg Gly Thr Arg Glu Gln Tyr Phe
   1 5 10
<210> 30
   <211> 13
   <212> PRT
   <213> Homo sapiens
<400> 30
   Cys Ala Ser Ser Leu Arg Ile Asn Tyr Glu Gln Tyr Phe
   1 5 10
<210> 31
   <211> 15
   <212> PRT
   <213> Homo sapiens
<400> 31
   Cys Ala Ser Ser Arg Pro Glu Ala Thr Asn Glu Lys Leu Phe Phe
   1 5 10 15
<210> 32
   <211> 13
   <212> PRT
   <213> Homo sapiens
<400> 32
   Cys Ala Ser Ser Trp Gly Thr Asp Thr Glu Ala Phe Phe
   1 5 10
<210> 33
   <211> 11
   <212> PRT
   <213> Homo sapiens
<400> 33
   Cys Ala Trp Ala Lys Gly Thr Glu Ala Phe Phe
   1 5 10
<210> 34
   <211> 14
   <212> PRT
   <213> Homo sapiens
<400> 34
   Cys Ala Ser Ser Gln Val Thr Gly Ile Thr Glu Ala Phe Phe
   1 5 10
<210> 35
   <211> 14
   <212> PRT
   <213> Homo sapiens
<400> 35
   Cys Ala Ser Ser Pro Gly Gly Arg Pro Tyr Glu Gln Tyr Phe
   1 5 10
<210> 36
   <211> 15
   <212> PRT
   <213> Homo sapiens
<400> 36
   Cys Ala Ser Ser Pro Gly Gln Gly Glu Gly Tyr Glu Gln Tyr Phe
   1 5 10 15
<210> 37
   <211> 19
   <212> PRT
   <213> Homo sapiens
<400> 37
   Cys Ala Ser Ser Gln Val Gly Ser Ser Val Ala Gly Gly Arg Ser Glu
   1 5 10 15
   Ala Phe Phe
<210> 38
   <211> 18
   <212> PRT
   <213> Homo sapiens
<400> 38
   Cys Ala Ser Ser Ser Thr Gly Thr Gly Gly Ser Ser Trp Asn Glu Gln
   1 5 10 15
   Phe Phe
<210> 39
   <211> 12
   <212> PRT
   <213> Homo sapiens
<400> 39
   Cys Ala Thr Gly Thr Gly Ser Tyr Glu Gln Tyr Phe
   1 5 10
<210> 40
   <211> 14
   <212> PRT
   <213> Homo sapiens
<400> 40
   Cys Ala Ser Ser Leu Trp Glu Ala Ser Tyr Gly Tyr Thr Phe
   1 5 10
<210> 41
   <211> 15
   <212> PRT
   <213> Homo sapiens
<400> 41
   Cys Ala Ser Ser Gln Thr Gly Thr Gly Ser Tyr Glu Gln Tyr Phe
   1 5 10 15
<210> 42
   <211> 14
   <212> PRT
   <213> Homo sapiens
<400> 42
   Cys Ala Ser Ser Ile Ala Gln Gly Val Tyr Glu Gln Tyr Phe
   1 5 10
<210> 43
   <211> 14
   <212> PRT
   <213> Homo sapiens
<400> 43
   Cys Ala Ser Ser Gln Arg Arg Leu Asn Thr Glu Ala Phe Phe
   1 5 10
<210> 44
   <211> 14
   <212> PRT
   <213> Homo sapiens
<400> 44
   Cys Ala Ser Ser Leu Gly Thr Ala Lys Glu Thr Gln Tyr Phe
   1 5 10
<210> 45
   <211> 14
   <212> PRT
   <213> Homo sapiens
<400> 45
   Cys Ala Ser Ser Phe Glu Ala Pro Ala Tyr Glu Gln Tyr Phe
   1 5 10
<210> 46
   <211> 14
   <212> PRT
   <213> Homo sapiens
<400> 46
   Cys Ala Ser Ser Leu Ala Gly Gly Leu Val Glu Gln Tyr Phe
   1 5 10
<210> 47
   <211> 15
   <212> PRT
   <213> Homo sapiens
<400> 47
   Cys Ala Thr Thr Gln Ala Gly Thr Glu Asn Thr Glu Ala Phe Phe
   1 5 10 15
<210> 48
   <211> 15
   <212> PRT
   <213> Homo sapiens
<400> 48
   Cys Ala Ser Ser Pro Gly Gln Gly Glu Gly Tyr Glu Gln Tyr Phe
   1 5 10 15
<210> 49
   <211> 14
   <212> PRT
   <213> Homo sapiens
<400> 49
   Cys Ala Ser Ser Gln Glu Gly Glu Gly Glu Thr Gln Tyr Phe
   1 5 10
<210> 50
   <211> 19
   <212> PRT
   <213> Homo sapiens
<400> 50
   Cys Ala Ser Ser Val Gly Pro Gly Leu Asn Met Gln Val Thr Asp Thr
   1 5 10 15
   Gln Tyr Phe
<210> 51
   <211> 15
   <212> PRT
   <213> Homo sapiens
<400> 51
   Cys Ala Ser Ser Tyr Arg Asp Ser Ser Ser Tyr Glu Gln Tyr Phe
   1 5 10 15
<210> 52
   <211> 16
   <212> PRT
   <213> Homo sapiens
<400> 52
   Cys Ala Ser Ser Tyr Leu Ala Glu Pro Pro Gly Asn Glu Gln Phe Phe
   1 5 10 15
<210> 53
   <211> 16
   <212> PRT
   <213> Homo sapiens
<400> 53
   Cys Ala Ser Ser Ser Tyr Ser Glu Thr Ala Asn Tyr Gly Tyr Thr Phe
   1 5 10 15
<210> 54
   <211> 14
   <212> PRT
   <213> Homo sapiens
<400> 54
   Cys Ala Ser Ser Gln Glu Arg Ser Thr Gly Glu Leu Phe Phe
   1 5 10
<210> 55
   <211> 15
   <212> PRT
   <213> Homo sapiens
<400> 55
   Cys Ala Ser Ser Tyr Trp Gly Gly Thr Asn Thr Glu Ala Phe Phe
   1 5 10 15
<210> 56
   <211> 13
   <212> PRT
   <213> Homo sapiens
<400> 56
   Cys Ala Ser Ser Ile Asp Arg Gly Ser Glu Ala Phe Phe
   1 5 10
<210> 57
   <211> 16
   <212> PRT
   <213> Homo sapiens
<400> 57
   Cys Ala Ser Ser Gln Val Leu Ser Gly Gly Phe Tyr Glu Gln Tyr Phe
   1 5 10 15
<210> 58
   <211> 11
   <212> PRT
   <213> Homo sapiens
<400> 58
   Cys Ala Trp Ser Lys Glu Tyr Gly Tyr Thr Phe
   1 5 10
<210> 59
   <211> 13
   <212> PRT
   <213> Homo sapiens
<400> 59
   Cys Ala Trp Thr Trp Gly Gly Gly Asn Glu Gln Tyr Phe
   1 5 10
<210> 60
   <211> 18
   <212> PRT
   <213> Homo sapiens
<400> 60
   Cys Ala Thr Ser Asp Leu His Arg Thr Pro Asp Leu Asn Thr Glu Ala
   1 5 10 15
   Phe Phe
<210> 61
   <211> 15
   <212> PRT
   <213> Homo sapiens
<400> 61
   Cys Ala Ser Ser Ser Gln Gly Asp Gly Thr Asp Thr Gln Tyr Phe
   1               5                   10                  15
<210> 62
   <211> 13
   <212> PRT
   <213> Homo sapiens
<400> 62
   Cys Ala Ser Ser Pro Gly Pro Asn Tyr Glu Gln Tyr Phe
   1               5                   10
<210> 63
   <211> 12
   <212> PRT
   <213> Homo sapiens
<400> 63
   Cys Ala Ser Ser Leu Glu Glu Tyr Gly Tyr Thr Phe
   1               5                   10
<210> 64
   <211> 15
   <212> PRT
   <213> Homo sapiens
<400> 64
   Cys Ala Ser Ser Gln Asp Arg Ser Val Ala Tyr Glu Gln Tyr Phe
   1               5                   10                  15
<210> 65
   <211> 16
   <212> PRT
   <213> Homo sapiens
<400> 65
   Cys Ala Ser Ser Leu Arg Gly Lys Thr Ser Thr Tyr Glu Gln Tyr Phe
   1               5                   10                  15
<210> 66
   <211> 13
   <212> PRT
   <213> Homo sapiens
<400> 66
   Cys Ala Ser Ser Leu Ser Ser Lys Asn Glu Gln Phe Phe
   1               5                   10
<210> 67
   <211> 14
   <212> PRT
   <213> Homo sapiens
<400> 67
   Cys Ala Val Asn Gln Ala Gly Trp Gly Gly Thr Gln Tyr Phe
   1               5                   10
<210> 68
   <211> 16
   <212> PRT
   <213> Homo sapiens
<400> 68
   Cys Ala Trp Ser Phe Pro Gly Ala Ser Gly Gly Glu Thr Gln Tyr Phe
   1               5                   10                  15
<210> 69
   <211> 14
   <212> PRT
   <213> Homo sapiens
<400> 69
   Cys Ala Ser Ser Gln Arg Ala Ala Pro Tyr Gly Tyr Thr Phe
   1               5                   10
<210> 70
   <211> 14
   <212> PRT
   <213> Homo sapiens
<400> 70
   Cys Ala Ser Ser Ser Gly His Gly Tyr Asn Glu Gln Phe Phe
   1               5                   10
<210> 71
   <211> 17
   <212> PRT
   <213> Homo sapiens
<400> 71
   Cys Ala Ser Ser Leu Leu Leu Ser Gly Gly Ala Ala Asp Thr Gln Tyr
   1               5                   10                  15
   Phe
<210> 72
   <211> 13
   <212> PRT
   <213> Homo sapiens
<400> 72
   Cys Ala Ser Ser Arg Gly Pro Asn Tyr Glu Gln Tyr Phe
   1               5                   10
<210> 73
   <211> 13
   <212> PRT
   <213> Homo sapiens
<400> 73
   Cys Ala Ser Ser Ile Asp Ser Asn Asn Glu Gln Phe Phe
   1 5 10
<210> 74
   <211> 17
   <212> PRT
   <213> Homo sapiens
<400> 74
   Cys Ala Thr Ser Asp Leu Ile Asp Phe Asp Arg Val Asp Gly Tyr Thr
   1 5 10 15
   Phe
<210> 75
   <211> 12
   <212> PRT
   <213> Homo sapiens
<400> 75
   Cys Ala Ser Ser Pro Leu Thr Gly Met Gln Phe Phe
   1 5 10
<210> 76
   <211> 15
   <212> PRT
   <213> Homo sapiens
<400> 76
   Cys Ala Ser Ile Trp Arg Leu Gly Met Asn Thr Glu Ala Phe Phe
   1 5 10 15
<210> 77
   <211> 13
   <212> PRT
   <213> Homo sapiens
<400> 77
   Cys Ala Ser Ser Ser Thr Val Ala Gly Glu Gln Tyr Phe
   1 5 10
<210> 78
   <211> 15
   <212> PRT
   <213> Homo sapiens
<400> 78
   Cys Ala Ser Ser Pro Arg Thr Gly Asn Thr Gly Glu Leu Phe Phe
   1 5 10 15
<210> 79
   <211> 15
   <212> PRT
   <213> Homo sapiens
<400> 79
   Cys Ala Ser Thr Arg Ser Val Gly Ala Gly Thr Glu Ala Phe Phe
   1 5 10 15
<210> 80
   <211> 18
   <212> PRT
   <213> Homo sapiens
<400> 80
   Cys Ala Ser Ser Pro Gly Thr Asp Gly Gly Ser Leu Gly Ser Pro Leu
   1 5 10 15
   His Phe
<210> 81
   <211> 13
   <212> PRT
   <213> Homo sapiens
<400> 81
   Cys Ala Ser Ser Trp Asp Ser Ser Tyr Glu Gln Tyr Phe
   1 5 10
<210> 82
   <211> 13
   <212> PRT
   <213> Homo sapiens
<400> 82
   Cys Ala Ser Ser Pro Leu Gly Gly Glu Lys Leu Phe Phe
   1 5 10
<210> 83
   <211> 13
   <212> PRT
   <213> Homo sapiens
<400> 83
   Cys Ala Ser Ser Gln Ala Gly Ile His Gly Tyr Thr Phe
   1 5 10
<210> 84
   <211> 15
   <212> PRT
   <213> Homo sapiens
<400> 84
   Cys Ala Ser Ser Ile Ala Gly Gly Pro Gly Glu Thr Gln Tyr Phe
   1 5 10 15
<210> 85
   <211> 14
   <212> PRT
   <213> Homo sapiens
<400> 85
   Cys Ala Ser Ser Gln Val Pro Asp Arg Asp Gly Tyr Thr Phe
   1 5 10
<210> 86
   <211> 15
   <212> PRT
   <213> Homo sapiens
<400> 86
   Cys Ala Ser Ser Gln Gly Ala Ala Leu Gly Tyr Glu Gln Tyr Phe
   1 5 10 15
<210> 87
   <211> 15
   <212> PRT
   <213> Homo sapiens
<400> 87
   Cys Ala Ser Ser Glu Tyr Leu Glu Val Gln Glu Thr Gln Tyr Phe
   1 5 10 15
<210> 88
   <211> 13
   <212> PRT
   <213> Homo sapiens
<400> 88
   Cys Ala Ser Ser Leu Glu Ala Asn Asn Glu Gln Phe Phe
   1 5 10
<210> 89
   <211> 18
   <212> PRT
   <213> Homo sapiens
<400> 89
   Cys Ala Ile Ser Glu Ser Lys Asp Arg Pro Ser Ser Tyr Asn Glu Gln
   1 5 10 15
   Phe Phe
<210> 90
   <211> 14
   <212> PRT
   <213> Homo sapiens
<400> 90
   Cys Ala Ser Ser Pro Gly Ala Gly Leu Tyr Glu Gln Tyr Phe
   1 5 10
<210> 91
   <211> 13
   <212> PRT
   <213> Homo sapiens
<400> 91
   Cys Ala Ser Ser Gln Lys Trp Gly Asn Ile Gln Tyr Phe
   1 5 10
<210> 92
   <211> 13
   <212> PRT
   <213> Homo sapiens
<400> 92
   Cys Ala Thr Gly Leu Ala Gly Gly Gln Glu Gln Tyr Phe
   1 5 10
<210> 93
   <211> 12
   <212> PRT
   <213> Homo sapiens
<400> 93
   Cys Ala Ser Ser Leu Thr Asp Tyr Gly Tyr Thr Phe
   1 5 10
<210> 94
   <211> 12
   <212> PRT
   <213> Homo sapiens
<400> 94
   Cys Ala Ser Ser Leu Thr Asp Tyr Gly Tyr Thr Phe
   1 5 10
<210> 95
   <211> 14
   <212> PRT
   <213> Homo sapiens
<400> 95
   Cys Ala Ser Thr Pro Gly Ser Tyr Arg Glu Thr Gln Tyr Phe
   1 5 10
<210> 96
   <211> 14
   <212> PRT
   <213> Homo sapiens
<400> 96
   Cys Ala Ser Gly Thr Asp Phe Pro Ser Tyr Glu Gln Tyr Phe
   1 5 10
<210> 97
   <211> 14
   <212> PRT
   <213> Homo sapiens
<400> 97
   Cys Ala Ile Pro Ser Ser Ser Gly Ala Asn Val Leu Thr Phe
   1 5 10
<210> 98
   <211> 14
   <212> PRT
   <213> Homo sapiens
<400> 98
   Cys Ala Ser Ser Leu Val Gly Gly Pro His Glu Gln Tyr Phe
   1 5 10
<210> 99
   <211> 15
   <212> PRT
   <213> Homo sapiens
<400> 99
   Cys Ala Ser Ser Ser Ala Gly Thr Gly His Asn Glu Gln Phe Phe
   1 5 10 15
<210> 100
   <211> 13
   <212> PRT
   <213> Homo sapiens
<400> 100
   Cys Ala Ser Ser Gln Lys Asp Arg Tyr Gly Tyr Thr Phe
   1 5 10
<210> 101
   <211> 890
   <212> DNA
   <213> Homo sapiens
<400> 101
   gattcaggga tgcccgagga tcgattctca gctaagatgc ctaatgcatc attctccact      60
   ctgaagatcc agccctcaga acccagggac tcagctgtgt acttctgtgc cagcagtttc     120
   tcgacctgtt cggctaacta tggctacacc ttcggttcgg ggaccaggtt aaccgttgta     180
   gaggacctga acaaggtgtt cccacccgag gtcgctgtgt ttgagccatc agaagcagag     240
   atctcccaca cccaaaaggc cacactggtg tgcctggcca caggcttctt ccccgaccac     300
   gtggagctga gctggtgggt gaatgggaag gaggtgcaca gtggggtcag cacagacccg     360
   cagcccctca aggagcagcc cgccctcaat gactccagat actgcctgag cagccgcctg     420
   agggtctcgg ccaccttctg gcagaacccc cgcaaccact tccgctgtca agtccagttc     480
   tacgggctct cggagaatga cgagtggacc caggataggg ccaaacccgt cacccagatc     540
   gtcagcgccg aggcctgggg tagagcagac tgtggcttta cctcggtgtc ctaccagcaa     600
   ggggtcctgt ctgccaccat cctctatgag atcctgctag ggaaggccac cctgtatgct     660
   gtgctggtca gtgcccttgt gttgatggcc atggtcaaga gaaaggattt ctgaaggcag     720
   ccctggaagt ggagttagga gcttactaac ccgtcatggt tcaatacaca ttcttctttt     780
   gccagcgctt ctgaagagct gctctcacct ctctgcatcc caatagatat ccccctatgt     840
   gcatgcacac ctgcacactc acggctgaaa tctccctaac ccagggggac                890